(19) Europäisches Patentamt
European Patent Office
Office européen des brevets

(11) EP 3 904 357 A1

(12) **EUROPEAN PATENT APPLICATION**
published in accordance with Art. 153(4) EPC

(43) Date of publication:
**03.11.2021 Bulletin 2021/44**

(21) Application number: **19886563.6**

(22) Date of filing: **20.11.2019**

(51) Int Cl.:
***C07D 495/04*** *(2006.01)* ***A61K 31/497*** *(2006.01)*
***A61P 1/16*** *(2006.01)*

(86) International application number:
**PCT/CN2019/119631**

(87) International publication number:
**WO 2020/103851 (28.05.2020 Gazette 2020/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.11.2018 CN 201811385712**

(71) Applicant: **The National Institutes of Pharmaceutical R&D Co., Ltd**
**Shahe**
**Changping**
**Beijing 102206 (CN)**

(72) Inventors:
- **YIN, Huijun**
**Beijing 102206 (CN)**
- **YAN, Xu**
**Beijing 102206 (CN)**
- **WEN, Junge**
**Beijing 102206 (CN)**
- **TIAN, Weixue**
**Beijing 102206 (CN)**
- **MOU, Qiyong**
**Beijing 102206 (CN)**
- **FU, Ning**
**Beijing 102206 (CN)**
- **JIANG, Jingqian**
**Beijing 102206 (CN)**
- **WU, Yao**
**Beijing 102206 (CN)**
- **WU, Guosheng**
**Beijing 102206 (CN)**
- **FENG, Xueying**
**Beijing 102206 (CN)**
- **QIAN, Su**
**Beijing 102206 (CN)**

(74) Representative: **EP&C**
**P.O. Box 3241**
**2280 GE Rijswijk (NL)**

# (54) SPIRO COMPOUND AND MEDICAL USES THEREOF

(57) The present invention relates to a spiro compound and medical uses thereof. Specifically, the present invention relates to a spiro compound represented by the general formula (I), a preparation method thereof, a pharmaceutical composition containing the same, and is used as an acetyl-Coenzyme A carboxylase (ACC) inhibitor and is used for treating diseases associated with ACC activity. Each substituent in the general formula (I) is as defined in description.

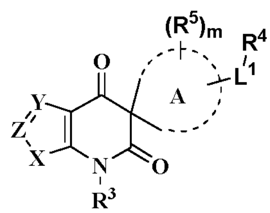

(I)

EP 3 904 357 A1

## Description

## FIELD OF THE INVENTION

The present invention relates to a spiro compound, the preparation method thereof, and the pharmaceutical composition comprising the same, as well as the use thereof as an acetyl-Coenzyme A carboxylase (ACC) inhibitor, in particular in the preparation of drugs for treating or preventing the diseases associated with ACC activity.

## BACKGROUND OF THE INVENTION

Acetyl-Coenzyme A (CoA) carboxylase (ACC) is an enzyme related to fat synthesis and metabolism. In human and other animals, ACC is present in the forms of two tissue-specific isoenzymes: ACC1 of 265 KDa is mainly distributed in adipogenic tissues (liver, fat); ACC2 of 280 KDa is mainly distributed in oxidizing tissues (liver, heart, skeletal muscle) (Molecules, 2013, 18, 1704-1719).

In the liver, ACC1 forms malonyl CoA in the cytosol for fatty acid synthesis and extension, leading to triglyceride formation and VLDL production. In the heart and skeletal muscle, the malonyl CoA formed by ACC2 mainly acts on CPT-1 as a regulator of fatty acid oxidation. Malonyl-CoA formed by ACC2 on the surface of mitochondria mainly regulates the oxidation of mitochondrial fatty acids (Elsevier Science & Technology, 2010, 45(10): 95-108).

ACC forms malonyl CoA via carboxylation of acetyl CoA with ATP. The reaction is carried out in two half reactions, namely the biotin carboxylase (BC) reaction and the carboxyl transferase (CT) reaction, which is the first step in fatty acid biosynthesis and the rate-limiting step in this pathway. In human and other animals, the activity of ACC is strictly regulated by various diets, hormones and other physiological responses. These effects are carried out through feedforward allosteric activation of citric acid, feedback inhibition of long-chain fatty acids, reversible phosphorylation and inactivation, and by changing the expression level of ACC protein (Elsevier Science & Technology, 2010, 45(10): 95-108).

Studies have shown that ACC1/2 is closely related to the onset and development of a variety of fat-related diseases. For example, ACC2 knockout mice can avoid liver fat accumulation, insulin resistance and dyslipidemia induced by high-fat diet, and increase energy expenditure. Liver-specific ACC1 knockout can reduce liver malonyl CoA synthesis. In human, exercise will reduce the expression of ACC2 and increase fatty acid oxidation, while obese and type II diabetes patients have increased activity of ACC2 and increased level of malonyl-CoA. Part of the efficacy of the major hypoglycemic drug metformin is derived from AMPK agonism, and AMPK can inhibit the activity of ACC2 by phosphorylation. It is worth noting that in recent years, the role of ACC1/2 in acne and fat supply of tumor cell has also been focused on. In summary, ACC1/2 is an ideal target for the treatment of fat metabolic diseases, especially fatty liver diseases (J. Med. Chem. 2015, 58(2): 525-36).

In recent years, the morbidity and mortality of non-alcoholic steatohepatitis (NASH) have been increasing year by year, and has become similar to those of cardiovascular disease, malignant tumors and liver cirrhosis. The fat content in normal liver is usually less than 5% of the total liver. When obesity, hypertriglyceridemia, type 2 diabetes and metabolic syndrome exist, they will lead to elevated blood lipids, liver fat deposition, and liver steatosis. Once liver steatosis occurs, insulin resistance, disorders of carbohydrate and lipid metabolism are prone to occur, the energy consumption of the body is reduced, and the accumulation increases. When adipogenesis increases, free fatty acids increase, which affects the mitochondrial function of the liver and leads to two results: one is liver cell apoptosis, and one is oxidative stress. Apoptosis of hepatocytes triggers cell inflammation, thus causing the activation of Kupffer cells in the liver. The activated Kupffer cells release cytokines, which in turn stimulates the activation of hepatic stellate cells. The activation of stellate cells leads to increased collagen production. In this way, when various factors such as obesity, hypertriglyceridemia, type 2 diabetes, and metabolic syndrome exist, the liver undergoes three major processes from increased adipogenesis to inflammation and to liver fibrosis, and eventually NASH.

The NASH-specific drugs, having currently entered Phase III clinical trials, are mainly divided into three categories: insulin resistance and fat degradation, anti-oxidant and anti-inflammatory, and anti-fibrotic drugs. Drugs that inhibit fat synthesis, especially the bile acid pathway, include FXR ligands, such as obeticholic acid (OCA), which can block the activation of farnesoid X receptors and thereby regulate lipid metabolism. Drugs that promote fat degradation include PPAR agonists, such as Elafibranor, which can stimulate PPAR$\alpha$ and $\beta$ to promote fat degradation. Drugs that inhibit cell apoptosis, such as ASK-1 (apoptosis signal-regulating kinase 1) inhibitor (Selonsertib), can inhibit cell apoptosis during the apoptotic stage. Anti-liver fibrosis agents inhibit the release of inflammatory factors by Kupffer cells, such as the chemokine CCR2/5 antagonist Cenicriviroc, which inhibits the release of cytokines by Kupffer cells.

ACC acts on one of the several biologically relevant pathways related to the progression of NASH disease. ACC is the first step to catalyze the regeneration of fat in the liver. Excessive synthesis and accumulation of fatty acids can cause liver steatosis, and subsequently cause inflammation and liver fibrosis. ACC1/2 inhibition not only inhibits fatty acid synthesis, but also promotes fatty acid metabolism ($\beta$-oxidation) to achieve the effect of lowering lipid levels

in specific tissues or the whole body.

GS-0976 of Gilead is the first non-selective ACC allosteric inhibitor that targets the liver. It prevents the dimerization of ACC subunits by acting on the BC domain of ACC and inhibits ACC enzyme activity. The results of preclinical studies and early clinical studies have shown that the drug has a good effect on NASH, and also has potential effects on obesity and diabetes. It can reduce liver steatosis, improve insulin sensitivity, and regulate dyslipidemia. It is reported that GS-0976 has a significant effect on the two allosteric enzymes ACC1 and ACC2 *in vitro.* At the cellular level, it inhibits fatty acid synthesis and promotes fatty acid oxidative degradation. Its *in vivo* animal experiment shows a significant effect of quickly reducing the content of malonyl CoA and TG. In the 28-day administration safety test in rats, no drug-related toxicity is found when the dose is 60 $mg.kg^{-1}.d^{-1}$ (Proc. Natl. Acad. Sci. 2016, 113(13): E1796-805).

In April 2017, Gilead announced the results of the initial clinical study of GS-0976: 10 patients received 20 mg dose of GS-0976 orally once a day for 12 weeks in the clinical trial; the results preliminarily showed that the therapeutic regimen was associated with statistically significant improvements in liver fat content and non-invasive fibrosis markers according to the effective mechanism of inhibiting the denovo synthesis of fat in liver cells. On October 24, 2017, Gilead announced the success of the Phase II clinical study of GS-0976. This study evaluated the efficacy and safety of two doses of GS-0976 in patients with non-alcoholic steatohepatitis (NASH). In patients receiving GS-0976, after 12 weeks, the amount of denovo fat synthesis decreased from the baseline level by a median of 29%. In the $12^{th}$ week, the liver fat content of patients receiving GS-0976 relatively decreased by 43%: measured by magnetic resonance imaging-proton density fat fraction (MRI-PDFF), decreasing from 15.7% to 9.0% ($p$ = 0.006). At the same time, in the $12^{th}$ week, the median cirrhosis, a non-invasive marker of fibrosis, was reduced from 3.4 kPa to 3.1 kPa ($p$ = 0.049) as evaluated by magnetic resonance elastography (MRE). In addition, in patients with reduced liver fat, the liver biochemical indicators, serum markers of fibrosis and cell apoptosis have also been improved, which supports the biological activity characteristics of GS-0976. GS-0976 was well tolerated, and nausea, abdominal pain and diarrhea were the most common adverse events.

At present, there is no specific drug for NASH on the market. The success of ACC inhibitor Phase II clinical study gives us hope. Although the improvement in fibrosis was limited after 12 weeks of GS-0976 treatment, the reason may be that GS-0976 does not directly bind to targets related to liver fibrosis, or may be because slowing and reversing fibrosis is a relatively slow process. The use of a single drug to treat NASH, especially to reverse fibrosis, has limited effect. However, reducing or eliminating fat deposition is to remove the root cause of liver inflammation. Removing the root cause of inflammation and controlling existing inflammation is a good way to avoid liver cell death/apoptosis. Therefore, continually researching and developing new ACC inhibitors with better selectivity and efficacy and exploring new combined therapiesare of great significance for the treatment of related diseases such as NASH.

## SUMMARY OF THE INVENTION

After painstaking research, the inventors have designed and synthesized a series of spiro compounds, which show inhibitory activity of acetyl-CoA carboxylase (ACC), and can be developed as drugs for the prevention or treatment of diseases associated with ACC activity.

Thus, an object of the present invention is to provide a compound of formula (I):

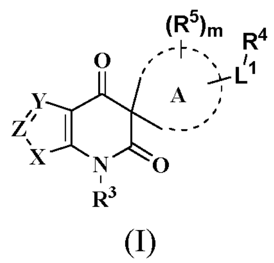

(I)

or a mesomer, racemate, enantiomer, diastereomer, or mixture thereof, or a pharmaceutically acceptable salt thereof, wherein,

X is selected from -O-, -S- and -NR-;

Y is selected from N and $CR^1$;

Z is selected from N and $CR^2$;

ring A is cycloalkyl or heterocyclyl;

$L^1$ is each independently selected from a single bond and a $C_1$-$C_3$ alkylene, wherein said alkylene is optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;

$R^4$ is selected from the group consisting of hydrogen, halogen, cyano, $-R^a$, $-OR^a$, - $S(O)_nR^a$, - $-N(R^a)C(O)R^b$, $-C(O)NR^aR^b$, $-C(O)N(R^a)S(O)_nR^b$, $-N(R^a)C(O)NR^aR^b$, $-N(R^a)C(O)OR^b$, $-OC(O)NR^aR^b$, $-N(R^a)S(O)_nR^b$, $-S(O)_nNR^aR^b$, $-C(O)R^a$, $-C(O)OR^a$, - $OC(O)R^a$, $-P(O)(OR^a)(OR^b)$ and $-B(OH)_2$;

$R^5$ is each independently selected from the group consisting of hydrogen, halogen, amino, nitro, cyano, hydroxyl, thiol, carboxyl, ester, oxo, alkyl, alkoxyl, cycloalkyl, heterocyclyl, aryl and heteroaryl, wherein said alkyl, alkoxyl, cycloalkyl, heterocyclyl, aryl and heteroaryl are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;

R is selected from the group consisting of hydrogen, halogen, oxo, alkyl, alkoxyl, cycloalkyl, heterocyclyl, aryl and heteroaryl, wherein said alkyl, alkoxyl, cycloalkyl, heterocyclyl, aryl and heteroaryl are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl; $R^1$ is selected from the group consisting of hydrogen, halogen, alkyl, cycloalkyl and heterocyclyl, wherein said alkyl, cycloalkyl and heterocyclyl are optionally further substituted by one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;

$R^2$ is selected from the group consisting of hydrogen, halogen, alkyl, alkoxyl, cycloalkyl, heterocyclyl, aryl and heteroaryl, wherein said alkyl, alkoxyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;

$R^3$ is selected from alkyl, said alkyl is further substituted with one or more Q groups;

each Q is independently selected from the group consisting of halogen, alkyl, cycloalkyl, heterocyclyl, aryl, heteroaryl, $OR^a$ and $SR^a$; wherein said alkyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxy, haloalkyl, haloalkoxyl, cycloalkyl, heterocyclyl, aryl and heteroaryl;

$R^a$ and $R^b$ are each independently selected from the group consisting of hydrogen, halogen, hydroxyl, nitro, cyano, oxo, carboxyl, ester, alkyl, cycloalkyl, heterocyclyl, aryl and heteroaryl, wherein said alkyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl; or

$R^a$ and $R^b$ together with the nitrogen atom attached to them form a N-containing heterocyclic group, wherein said N-containing heterocyclic group is optionally further substituted with one or more groups selected from the group consisting of halogen, amino, nitro, cyano, oxo, hydroxyl, thiol, carboxyl, ester, alkyl, alkoxyl, cycloalkyl, heterocyclyl, aryl and heteroaryl;

m is an integer from 0 to 5;

n is an integer from 0 to 2.

In a preferred embodiment of the invention, in the compound of formula (I) according to the invention, X is -S-.

In another preferred embodiment of the invention, the compound of formula (I) according to the invention is a compound of formula (II),

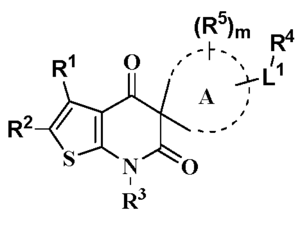

(II)

wherein, $R^1$, $R^2$, $R^3$, ring A, $L^1$, $R^4$ and m are as defined in formula (I).

In another preferred embodiment of the invention, in the compound of formula (I) according to the invention,

$R^3$ is selected from alkyl, preferably $C_1$-$C_6$ alkyl, said alkyl is further substituted by one or more Q groups;

each Q is independently selected from the group consisiting of aryl, heteroaryl, $OR^a$ and $SR^a$; wherein said aryl and heteroaryl are optionally further substituted with one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;

$R^a$ is selected from the group consisting of alkyl, cycloalkyl and heterocyclyl, wherein said alkyl, cycloalkyl and heterocyclic are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl.

In another preferred embodiment of the invention, the compound of formula (I) according to the invention is a compound of formula (III),

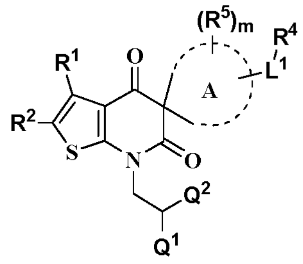

(III)

wherein,

$Q_1$ is selected from aryl and heteroaryl, preferably $C_5$-$C_{10}$ aryl or 5- to 10-membered heteroaryl, said aryl and heteroaryl are optionally further substituted with one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;
$Q_2$ is selected from $OR^a$ and $SR^a$, preferably $OR^a$;
$R^a$ is selected from cycloalkyl and heterocyclyl, preferably $C_3$-$C_7$ cycloalkyl or 5- to 7-membered heterocyclyl; wherein said cycloalkyl and heterocyclyl are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
wherein, $R^1$, $R^2$, ring A, $L^1$, $R^4$, $R^5$ and m are as defined in formula (I).

In another preferred embodiment of the invention, the compound of formula (I) according to the invention is a compound of formula (IV),

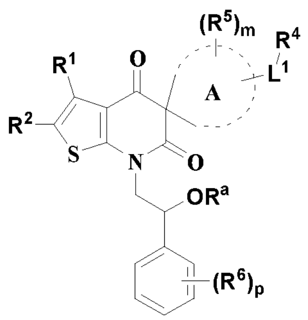

(IV)

wherein,

$R^6$ is selected from the group consisting of hydrogen, halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;
$R^a$ is selected from cycloalkyl and heterocyclyl, preferably $C_3$-$C_7$ cycloalkyl or 5- to 7-membered heterocyclyl; wherein said cycloalkyl and heterocyclyl are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
p is an integer from 1 to 4;
wherein, $R^1$, $R^2$, ring A, $L^1$, $R^4$, $R^5$ and m are as defined in formula (I).

In another preferred embodiment of the invention, in the compound of formula (I) according to the invention,

ring A is a $C_3$-$C_7$ cycloalkyl or a 5- to 7-membered heterocyclyl;
$L^1$ is selected from a single bond and an alkylene, preferably a single bond;
$R^4$ is selected from the group consisting of hydrogen, halogen, cyano, $-R^a$, $-OR^a$, $-S(O)_nR^a$, $-NR^aR^b$, $-N(R^a)C(O)R^b$, $-C(O)NR^aR^b$, $-C(O)N(R^a)S(O)_nR^b$, $-N(R^a)C(O)NR^aR^b$, $-N(R^a)C(O)OR^b$, $-OC(O)NR^aR^b$, $-N(R^a)S(O)_nR^b$, $-S(O)_nNR^aR^b$, $-C(O)R^a$, $-C(O)OR^a$, $-OC(O)R^a$, $-P(O)(OR^a)(OR^b)$ and $-B(OH)_2$;
$R^5$ is each independently selected from the group consisting of hydrogen, halogen, amino, hydroxyl, thiol, carboxyl, ester, oxo and alkyl, wherein said alkyl is optionally further substituted with halogen;
$R^a$ and $R^b$ are each independently selected from the group consisting of hydrogen, halogen, hydroxyl, nitro, cyano, oxo, carboxyl, ester, alkyl, cycloalkyl, heterocyclyl, aryl and heteroaryl, wherein said alkyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl; or
$R^a$ and $R^b$ together with the nitrogen atom attached to them form a N-containing heterocyclic group, wherein said N-containing heterocyclic group is optionally further substituted with one or more groups selected from the group consisting of halogen, amino, nitro, cyano, oxo, hydroxyl, thiol, carboxyl, ester, alkyl, alkoxyl, cycloalkyl, heterocyclyl, aryl and heteroaryl;
m is 0, 1 or 2;
n is an integer from 0 to 2; preferably 1 or 2.

In another preferred embodiment of the invention, in the compound of formula (I) according to the invention,

ring A is selected from the group consisting of cyclopropyl, cyclobutyl, cyclopentyl, cyclohexyl, pyrrolidinyl, piperidinyl and piperazinyl;
$L^1$ is selected from a single bond;
$R^4$ is selected from the group consisting of hydrogen, $-R^a$, $-C(O)R^a$, $-C(O)OR^a$, $-C(O)NR^aR^b$, $-S(O)_nR^a$ and $-P(O)(OR^a)(OR^b)$;
$R^5$ is each independently selected from the group consisting of hydrogen, halogen, amino, hydroxyl, thiol, carboxyl, ester, oxo and alkyl, wherein said alkyl is optionally further substituted with halogen;
$R^a$ and $R^b$ are each independently selected from the group consisting of hydrogen, hydroxyl, carboxyl, ester and alkyl, wherein said alkyl is optionally further substituted with one or more groups selected from the group consisting of hydroxyl, thiol, carboxyl and ester;
m is 0, 1 or 2;
n is 1 or 2.

In another preferred embodiment of the invention, in the compound of formula (I) according to the invention, $R^1$ is selected from the group consisting of hydrogen, halogen and alkyl, wherein said alkyl is optionally further substituted with halogen.

In another preferred embodiment of the present invention, in the compound formula (I) according to the invention, $R^2$ is selected from aryl and heteroaryl, preferably $C_5$-$C_{10}$ aryl or 5- to 10-membered heteroaryl, more preferably oxazolyl, imidazolyl, pyrazolyl, thiazolyl, said aryl and heteroaryl are optionally further substituted by one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl.

Typical compounds of the present invention include, but are not limited to:

| Example No. | Structure and name |
|---|---|
| 1 | 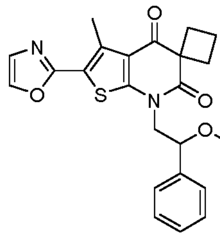 |
| | 3'-methyl-2'-(oxazol-2-yl)-7'-(2-phenyl-2-((tetrahydro-2*H*-pyran-4-yl)oxy)ethyl)-4'*H*-spiro [cyclobutane-1,5'-thieno[2,3-b]pyridine]-4',6'(7'*H*)-dione |
| 2 | 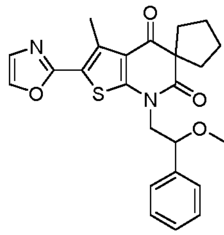 |
| | 3'-methyl-2'-(oxazol-2-yl)-7'-(2-phenyl-2-((tetrahydro-2*H*-pyran-4-yl)oxy)ethyl)-4'*H*-spiro [cyclopentane-1,5'-thieno[2,3-b]pyridine]-4',6'(7'*H*)-dione |
| 3 | 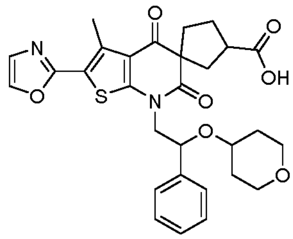 |
| | 3'-methyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylic acid |
| 4 | 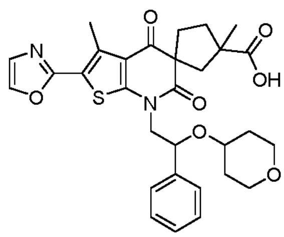 |
| | 3,3'-dimethyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylic acid |
| 5 | 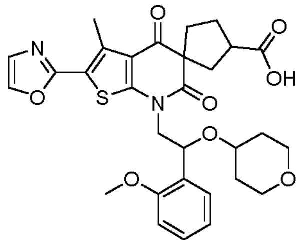 |
| | 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclopentane-1,5'-thieno[2,3-b]pyiidine]-3-carboxylic acid |

(continued)

| Example No. | Structure and name |
|---|---|
| 6 | 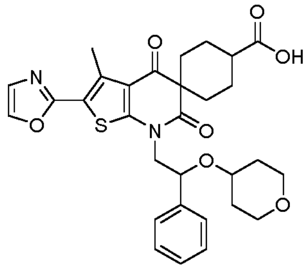 |
| | 3'-methyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclohexane-1,5'-thieno[2,3-b]pyridine]-4-carboxylic acid |
| 7 | 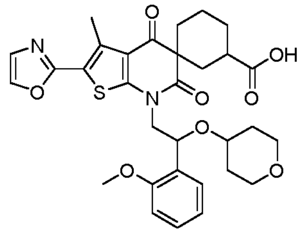 |
| | 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclohexane-1,5'-thieno[2,3-b]pyridine]-3-carboxylic acid |
| 8 | 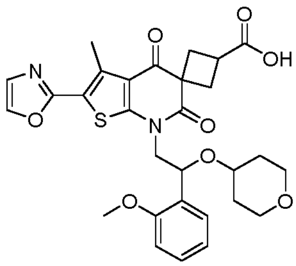 |
| | 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxylic acid |
| 9 | 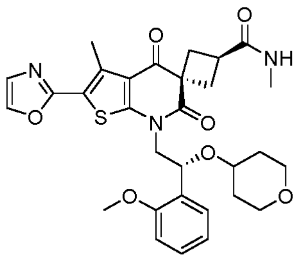 (1s,3S)-7'-((R)-2-(2-methoxyphenyl)-2-((tetrahydro-2*H*-pyran-4-yl)oxy)ethyl)-N,3'-dimethyl-2'-(oxazol-2-yl)-4',6'-dioxo-6',7'-dihydro-4'*H*-spiro[cyclohexane-1,5'-thieno[2,3-b]pyridine]-3-carboxyamide |

(continued)

| Example No. | Structure and name |
|---|---|
| 10 | 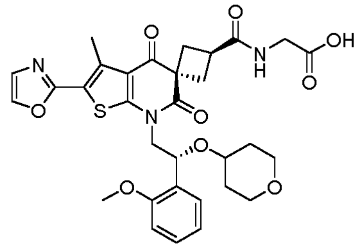 |
| | ((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)glycine |
| 10-1 | 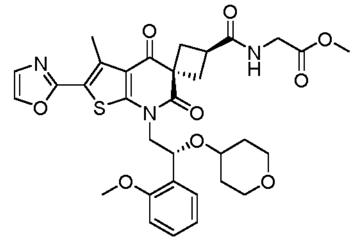 |
| | Methyl ((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl) glycinate |
| 11 | 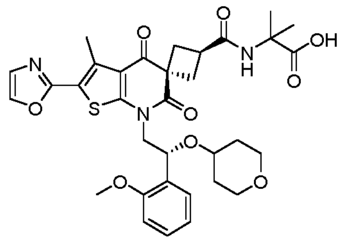 |
| | 2-((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[cyclobutane-1,5'-thieno[2,3-b]pyridine]-3-carboxyamido)-2-methylpropionic acid |
| 12 | 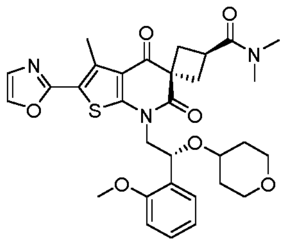 |
| | (1s,3S)-7'-((R)-2-(2-methoxyphenyl)-2-((tetrahydro-2*H*-pyran-4-yl)oxy)ethyl)-N,N,3'-trimethyl-2'-(oxazol-2-yl)-4',6'-dioxo-6',7'-dihydro-4'*H*-spiro[cyclohexane-1,5'-thieno[2,3-b]pyridine]-3-carboxamide |

(continued)

| Example No. | Structure and name |
|---|---|
| 13 | 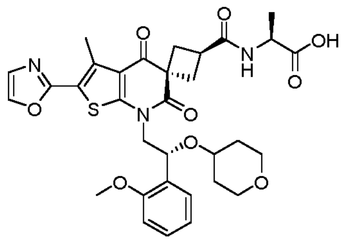 |
| | ((1R,3S)-7'-((R)-2-(2-methoxyphenyl)-2-((tetrahydro-2*H*-pyran-4-yl)oxy)ethyl)-3'-dimethyl-2'-(oxazol-2-yl)-4',6'-dioxo-6',7'-dihydro-4'*H*-spiro[cyclohexane-1,5'-thieno[2,3-b]pyridine]-3-carbonyl)-L-alanine |
| 14 | 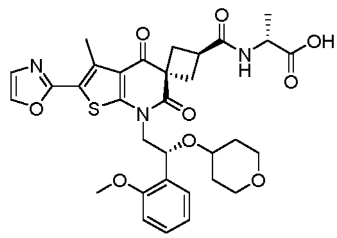 |
| | ((1S,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)-D-alanine |
| 14-1 | 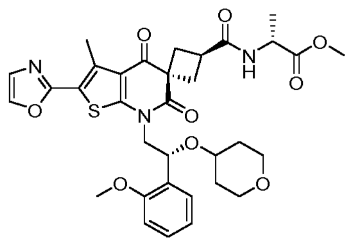 |
| | Methyl ((1S,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)-D-alaninate |
| 15 | 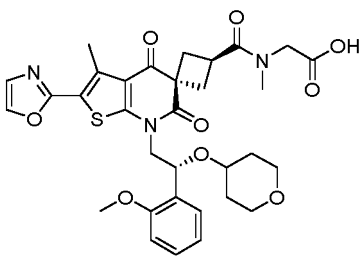 |
| | *N*-((1s,3S)-1'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyiidine]-3-carbonyl)-*N*-methylglycine |

(continued)

| Example No. | Structure and name |
|---|---|
| 15-1 | 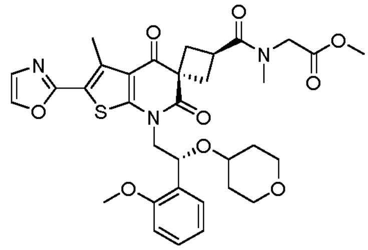 |
| | Methyl *N*-((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)-*N*-methyl glycinate |
| 16 | 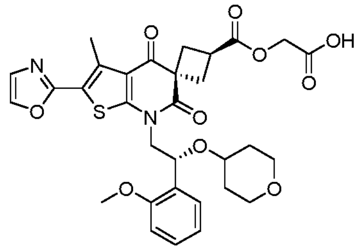 |
| | 2-(((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl) oxy)acetic acid |
| 17 | 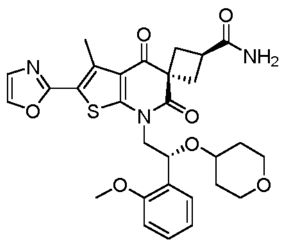 |
| | ((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxamide |
| 18 | 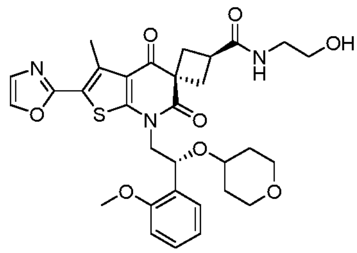 |
| | (1s,3S)-*N*-(2-hydroxyethyl)-1'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxamide |

(continued)

| Example No. | Structure and name |
|---|---|
| 19 | 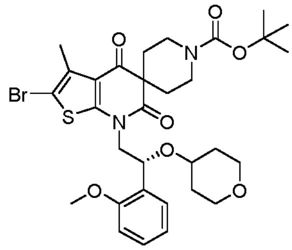 |
| | Tert-butyl(R)-2'-bromo-7'-(2-(2-methoxyphenyl)-2-((tetrahydro-2*H*-pyran-4-yl)oxy)ethyl)-3'-methyl-4',6'-dione-6',7'-dihydro-4'*H*-spiro[piperidine-4,5'-thieno[2,3-d]pyridine]-1-carboxylate |
| 20 | 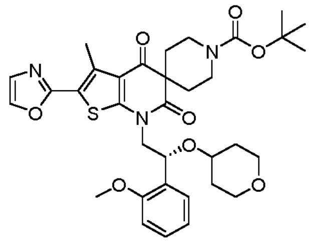 |
| | Tert-butyl(R)-7'-(2-(2-methoxyphenyl)-2-((tetrahydro-2*H*-pyran-4-yl)oxy)ethyl)-3'-methyl-2'-(oxazol-2-yl)-4',6'-dione-6',7'-dihydro-4'*H*-spiro[piperidine-4,5'-thieno[2,3-d]pyridine]-1-carboxylate |
| 21 | 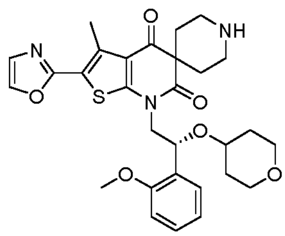 |
| | (R)-7'-(2-(2-methoxyphenyl)-2-((tetrahydro-2*H*-pyran-4-yl)oxy)ethyl)-3'-methyl-2'-(oxazol-2-yl)-4'*H*-spiro[piperidine-4,5'-thieno[2,3-d]pyridine]-4',6'(7'*H*)-dione |
| 22 | 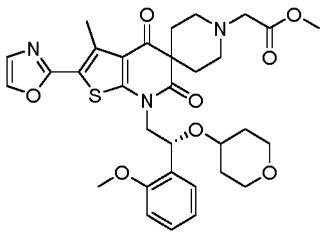 |
| | Methyl(R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)carboxylate |

(continued)

| Example No. | Structure and name |
|---|---|
| 23 | 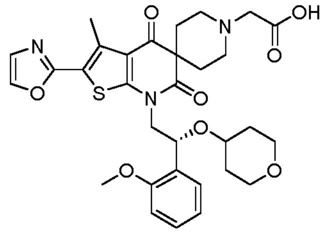 |
| | (R)-2-(1'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)acetic acid |
| 24 | 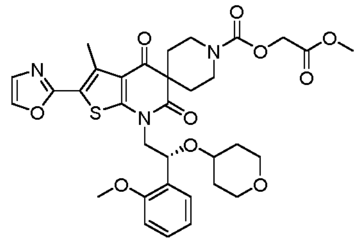 |
| | (2-Methoxy-2-oxoethyl)(R)-7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carboxylate |
| 25-1 | 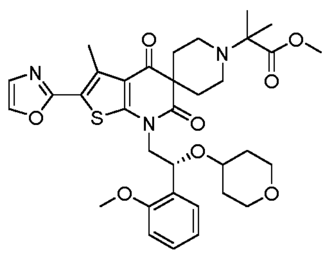 |
| | Methyl (R)-2-(7'-(2-(2-methoxyphenyl)-2-((tetrahydro-2H-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)-2-methylpropanoate |
| 25 | 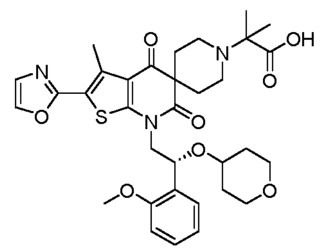 |
| | (R)-2-(1'-(2-(2-methoxypheny1)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)-2-methylpropionic acid |

(continued)

| Example No. | Structure and name |
|---|---|
| 26 | 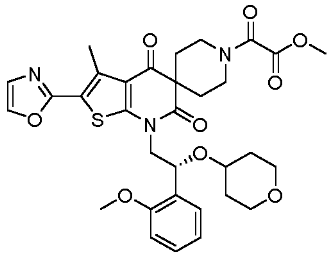 |
| | Methyl (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)-2-oxoacetate |
| 27 | 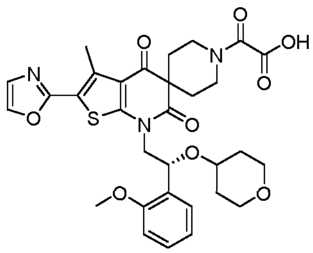 |
| | (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)-2-oxoacetic acid |
| 28 | 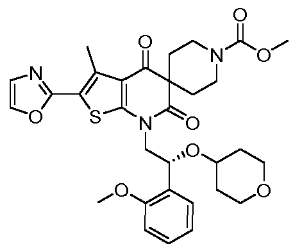 |
| | Methyl (R)-7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carboxylate |
| 29 | 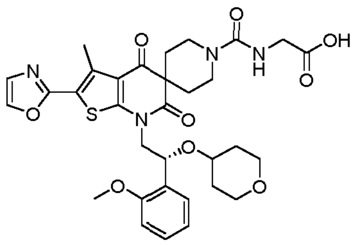 |
| | (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carbonyl)glycine |

(continued)

| Example No. | Structure and name |
|---|---|
| 30 | 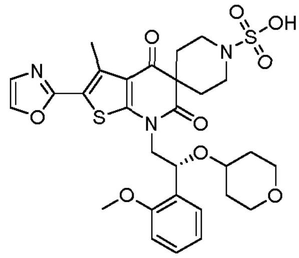 |
| | (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-sulfonic acid |
| 31 | 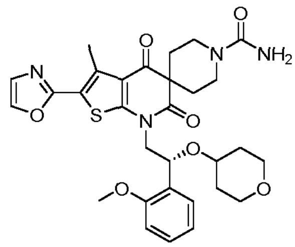 (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carboxamide |
| 32 | 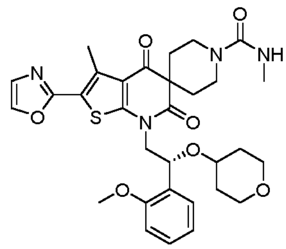 |
| | (R)-(7'-(2-(2-methoxyphenyl)-2-((tetrahydro-2*H*-pyran-4-yl)oxy)ethyl)-N,3'-dimethyl-2'-(oxazol-2-yl)-4',6'-dioxo-6',7'-dihydro-4'*H*-spiro[piperidine-4,5'-thieno[2,3-d]pyridine]-1-carboxamide |
| 33 | 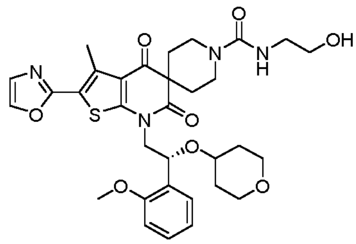 |
| | (R)-*N*-(2-hydroxyethyl)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carboxamide |

(continued)

| Example No. | Structure and name |
|---|---|
| 34 | 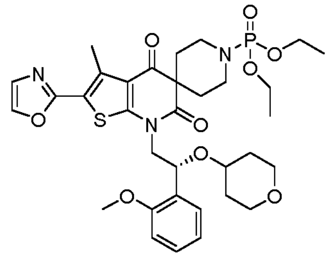 |
| | Diethyl (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)phosphate |

or a mesomer, racemate, enantiomer, diastereomer, or mixture thereof, or a pharmaceutically acceptable salt thereof.

The present invention further provides a method for preparing the compound of formula (I) according to the invention, which comprises the following steps:

when Z is $CR^2$,

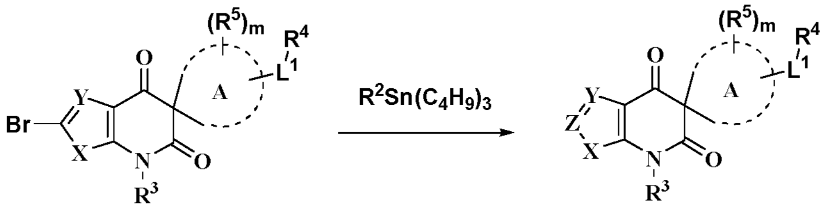


IF

(I)

compound IF is reacted with $R^2Sn(C_4H_9)_3$ in the presence of a catalyst to obtain a compound of formula (I), wherein said catalyst is preferably bis(triphenylphosphine)palladium dichloride;
X, Y, ring A, $L^1$, $R^2$, $R^3$, $R^4$, $R^5$ and m are as defined in formula (I).

The present invention further provides a pharmaceutical composition comprising the compound of formula (I) according to the invention and a pharmaceutically acceptable carrier or excipient.

Another aspect of the present invention provides a use of the compound of formula (I) according to the invention or a pharmaceutical composition comprising the same in the preparation of acetyl-CoA carboxylase inhibitors.

The present invention further provides a use of the compound of formula (I) according to the invention or a pharmaceutical composition comprising the same in the preparation of medicaments for the prevention or treatment of diseases associated with acetyl-CoA carboxylase activity; said disease is preferably metabolic disease, cardiovascular disease or cancer; said metabolic disease is for example dyslipidemia, obesity, diabetes, insulin resistance, metabolic syndrome, fatty liver disease or steatohepatitis, preferably fatty liver disease or steatohepatitis; said cardiovascular disease is for example atherosclerosis, angina pectoris, acute coronary syndrome or heart failure; said cancer is for example breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma or non-small cell lung cancer, preferably liver cancer.

The compound of formula (I) of the invention can form pharmaceutically acceptable acid addition salt with acid according to the conventional method in the field which the present invention belongs to. Said acid includes inorganic acid and organic acid, particularly preferably hydrochloric acid, hydrobromic acid, sulfuric acid, phosphoric acid, methanesulfonic acid, ethanesulfonic acid, p-toluenesulfonic acid, benzenesulfonic acid, naphthalene disulfonic acid, acetic acid, propionic acid, lactic acid, trifluoroacetic acid, maleic acid, citric acid, fumaric acid, oxalic acid, tartaric acid, benzoic acid, and the like.

The compound of formula (I) of the invention can form pharmaceutically acceptable basic addition salt with base according to the conventional method in the field which the present invention belongs to. Said base includes inorganic base and organic base. Acceptable organic base includes diethanolamine, ethanolamine, N-methylglucamine, triethanolamine, tromethamine, and the like, and acceptable inorganic base includes aluminum hydroxide, calcium hydroxide, potassium hydroxide, sodium carbonate and sodium hydroxide, and the like.

The pharmaceutical composition containing the active ingredient can be in a form suitable for oral administration, such as tablets, dragees, lozenges, aqueous or oily suspensions, dispersible powders or granules, emulsions, hard or soft capsules, or syrups or elixirs. The oral composition can be prepared according to any method known in the art for preparing pharmaceutical compositions, and such composition may contain one or more ingredients selected from the group consisting of sweeteners, flavoring agents, coloring agents and preservatives, to provide pleasing and tasty medicinal preparations. Tablets contain the active ingredient and non-toxic pharmaceutically acceptable excipients suitable for mixing for the preparation of tablets. These excipients can be inert excipients, such as calcium carbonate, sodium carbonate, lactose, calcium phosphate or sodium phosphate; granulating and disintegrating agents, such as microcrystalline cellulose, croscarmellose sodium, corn starch or alginic acid; binders such as starch, gelatin, polyvinylpyrrolidone or arabic gum; and lubricants, such as magnesium stearate, stearic acid or talc. These tablets may be uncoated or may be coated by known techniques that can mask the taste of the drug or delay the disintegration and absorption in the gastrointestinal tract, thereby providing a sustained release effect over a longer period of time. For example, water-soluble taste-masking substances such as hydroxypropyl methylcellulose or hydroxypropyl cellulose, or extended release substances such as ethylcellulose, cellulose acetate butyrate can be used.

It is also possible to provide an oral preparation by hard gelatin capsules in which the active ingredient is mixed with an inert solid diluent such as calcium carbonate, calcium phosphate or kaolin, or soft gelatin capsules in which the active ingredient is mixed with a water-soluble carrier such as polyethylene glycol or an oil solvent such as peanut oil, liquid paraffin or olive oil.

The aqueous suspension contains the active substance and excipients for mixing suitable for the preparation of aqueous suspension. Such excipients are suspending agents such as sodium carboxymethylcellulose, methylcellulose, hydroxypropylmethylcellulose, sodium alginate, polyvinylpyrrolidone and arabic gum; dispersing or wetting agents, which may be a naturally occurring phospholipid such as lecithin, or a condensation product of an alkylene oxide with a fatty acid such as polyoxyethylene stearate, or a condensation product of an ethylene oxide with a long chain fatty alcohol, such as heptadecylethyleneoxy cetanol, or a condensation product of an ethylene oxide with a part ester derived from fatty acids and hexitols, such as polyethylene oxide sorbitol monooleate, or a condensation product of an ethylene oxide with a partial ester derived from fatty acids and hexitans, such as polyethylene oxide sorbitan monooleate. The aqueous suspension may also contain one or more preservatives such as ethylparaben or n-propylparaben, one or more coloring agents, one or more flavoring agents, and one or more sweetening agents, such as sucrose, saccharin or aspartame.

The oil suspension can be formulated by suspending the active ingredient in a vegetable oil such as peanut oil, olive oil, sesame oil or coconut oil, or a mineral oil such as liquid paraffin. The oil suspension may contain a thickening agent, such as beeswax, hard paraffin or cetyl alcohol. The above sweeteners and flavoring agents may be added to provide a palatable preparation. The composition can be kept by addition of antioxidants such as butylated hydroxyanisole or alpha-tocopherol.

The active ingredient and dispersing or wetting agents, suspending agents or one or more preservatives can be provided by addition of water to the dispersible powders and granules suitable for the preparation of aqueous suspension. Suitable dispersing or wetting agents and suspending agents are as described above. Other excipients such as sweeteners, flavoring agents and coloring agents may also be added. The composition can be kept by addition of antioxidants such as ascorbic acid.

The pharmaceutical composition of the invention may also be in the form of an oil-in-water emulsion. The oil phase may be a vegetable oil such as olive oil or peanut oil, or a mineral oil such as liquid paraffin or a mixture thereof. Suitable emulsifiers may be naturally occurring phospholipids, such as soy lecithin, and esters or partial esters derived from fatty acids and hexitol anhydrides, such as sorbitan monooleate, and condensation products of said partial esters and ethylene oxide, such as polyethylene oxide sorbitol monooleate. The emulsions may also contain sweeteners, flavoring agents, preservatives and antioxidants. Syrups and elixirs may be formulated with sweeteners such as glycerol, propylene glycol, sorbitol or sucrose. Such preparations may also contain demulcents, preservatives, coloring agents, and antioxidants.

The pharmaceutical composition of the invention may also be in the form of a sterile injectable aqueous solution. Among the acceptable vehicles or solvents that may be employed are water, Ringer's solution and isotonic sodium chloride solution. The sterile injectable preparation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerin to form a microemulsion. The injection solution or microemulsion can be injected into the bloodstream of patients by a local massive injection. Alternatively,

the solution and microemulsion are preferably administered in a manner that maintains a constant circulating concentration of the compound of the invention. To maintain this constant concentration, a continuous intravenous delivery device can be used.

The pharmaceutical composition of the invention may be in the form of a sterile injectable aqueous or oil suspension for intramuscular and subcutaneous administration. The suspension may be formulated with those suitable dispersing or wetting agents and suspending agents indicated above according to known techniques. The sterile injectable preparation may also be a sterile injectable solution or suspension in a non-toxic parenterally acceptable diluent or solvent, such as a solution prepared in 1,3-butanediol. In addition, a sterile fixed oil may conveniently be employed as a solvent or suspension medium. For this purpose, any blended fixed oil including synthetic mono- or diglycerides can be used. In addition, fatty acids such as oleic acid can also used to prepare the injection.

The compound of the invention may be administered in the form of a suppository for rectal administration. These pharmaceutical compositions can be prepared by mixing the drug with a suitable non-irritating excipient which is solid at ordinary temperatures but liquid in the rectum and thus dissolves in the rectum to release the drug. Such materials include cocoa butter, glycerin gelatin, hydrogenated vegetable oil, and a mixture of polyethylene glycols and fatty acid esters of polyethylene glycol with various molecular weights.

As is well known to those skilled in the art, the dosage of a drug depends on a variety of factors including, but not limited to, the activity of the particular compound used, the age, weight, condition, behavior and diet of the patients, time of administration, way of administration, excretion rate, drug combination, and the like. Moreover, the optimal way of treatment, such as treatment mode, the daily dosage of the compound, or the type of the pharmaceutically acceptable salt, may be verified on the basis of the conventional therapeutic regimen.

The present invention may include a composition comprising a compound of formula (I), or a pharmaceutically acceptable salt, hydrate or solvate thereof as an active ingredient, and a pharmaceutically acceptable carrier or excipient, which is formulated into a clinically acceptable prepartion. The derivatives of the present invention can be used in combination with other active ingredients as long as they do not cause other adverse effects such as allergic reactions and the like. The present compound may be used as a single active ingredient or in combination with other agents for the treatment of disease associated with tyrosine kinase activity. Combination therapy can be achieved by administering the individual therapeutic components simultaneously, separately or sequentially.

Detailed description of the terms

Unless otherwise indicated, the terms used in the specification and claims have the following meanings.

The term "alkyl" refers to a saturated linear or branched aliphatic hydrocarbon containing 1 to 20 carbon atoms ($C_1$-$C_{20}$), preferably containing 1 to 12 carbon atoms, more preferably containing 1 to 6 carbon atoms. Examples of alkyl include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-decyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferred alkyl is a lower alkyl group with 1 to 6 carbon atoms including, but not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group may be substituted or unsubstituted, and when substituted, the substituent may be substituted at any available point. Said substituent is preferably selected from one or more groups independently selected from the group consisting of alkyl, alkenyl, alkynyl, alkoxyl, alkylthio, alkylamino, halogen, thiol, hydroxyl, nitro, cyano, cycloalkyl, heterocycloalkyl, aryl, heteroaryl, cycloalkoxyl, heterocycloalkoxyl, cycloalkylthio, heterocycloalkylthio, oxo, carboxyl and carboxylate group.

The term "alkenyl" refers to an alkyl as defined above consisting of at least two carbon atoms and at least one carbon-carbon double bond, such as vinyl, 1-propenyl, 2-propenyl, 1-, 2- or 3-butenyl, and the like. The alkenyl group may be substituted or unsubstituted, and when substituted, the substituent is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocycloalkyl, aryl, heteroaryl, cycloalkoxyl, heterocycloalkoxyl, cycloalkylthio and heterocyclealkylthio group.

The term "alkynyl" refers to an alkyl as defined above consisting of at least two carbon atoms and at least one carbon-carbon triple bond, such as ethynyl, propynyl, butynyl and the like. The alkynyl group may be substituted or

unsubstituted, and when substituted, the substituent is preferably one or more groups independently selected from the group consisting of alkyl, alkenyl, alkynyl, alkoxyl, alkylthio, alkylamino, halogen, thiol, hydroxy, nitro, cyano, cycloalkyl, heterocycloalkyl, aryl, heteroaryl, cycloalkoxyl, heterocycloalkoxyl, cycloalkylthio and heterocyclealkylthio group.

The term "cycloalkyl" refers to a saturated or partially unsaturated monocyclic or polycyclic hydrocarbon groups. The cycloalkyl ring includes 3 to 20 carbon atoms, preferably 3 to 12 carbon atoms, more preferably 3 to 6 carbon atoms. Examples of monocycloalkyl groups include, but are not limited to, cyclopropyl, cyclobutyl, cyclopentyl, cyclopentenyl, cyclohexyl, cyclohexenyl, cyclohexadienyl, cycloheptyl, cycloheptantrienyl, cyclooctyl, and the like; polycyclic cycloalkyl groups include spiro, fused, and bridged cycloalkyl groups.

The term "spiro cycloalkyl" refers to a polycyclic group that shares one carbon atom (referred to as spiro atom) between 5 to 20 membered single rings, which may contain one or more double bonds, but none of the rings have a fully conjugated $\pi$ electronic system. It is preferably 6- to 14-membered, more preferably 7- to 10-membered. The spiro cycloalkyl group is classified into monospirocycloalkyl, bispirocycloalkyl and polyspirocycloalkyl depending on the number of common spiro atoms among the rings, preferably monospirocycloalkyl and bispirocycloalkyl; more preferably, 4-membered/4-membered, 4-membered/5-membered, 4-membered/6-membered, 5-membered/5-membered or 5-membered/6-membered monospirocycloalkyl group. Examples of spiro cycloalkyl include, but are not limited to:

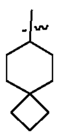 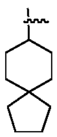 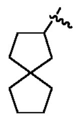 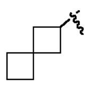 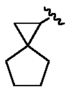 .

The term "fused cycloalkyl" refers to a 5- to 20-membered all-carbon polycyclic group wherein each ring in the system shares an adjacent pair of carbon atoms with other rings in the system, wherein one or more of the rings may contain one or more double bonds, but none of the rings have a fully conjugated $\pi$-electron system. It is preferably 6- to 14-membered, more preferably 7- to 10-membered. Depending on the number of constituent rings, it may be classified into bicyclic, tricyclic, tetracyclic or polycyclic fused cycloalkyl group, preferably a bicyclic or tricyclic cycloalkyl, more preferably a 5-membered/5-membered or 5-membered/6-membered bicycloalkyl group. Examples of fused cycloalkyl include, but are not limited to:

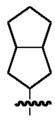 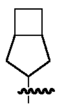 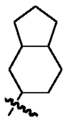 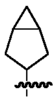 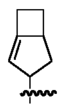 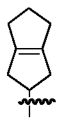 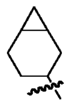 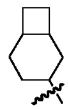 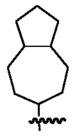 .

The term "bridged cycloalkyl" refers to a 5- to 20-membered all-carbon polycyclic group, wherein any two rings share two carbon atoms which are not directly bonded, which may contain one or more double bonds, but none of the rings have a fully conjugated $\pi$-electron system. It is preferably 6- to 14-membered, more preferably 7- to 10-membered. Depending on the number of constituent rings, it may be classified into bicyclic, tricyclic, tetracyclic or polycyclic bridged cycloalkyl group, preferably a bicyclic, tricyclic or tetracyclic ring, and more preferably a bicyclic or tricyclic ring. Examples of bridged cycloalkyl include, but are not limited to:

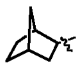 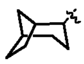 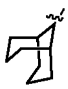 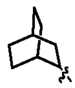 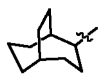

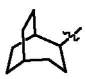 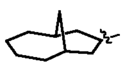 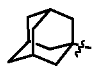 .

Said cycloalkyl ring can be fused to an aryl, heteroaryl or heterocycloalkyl ring, wherein the ring to which the parent structure is attached is a cycloalkyl group. Non-limiting examples include indanyl, tetrahydronaphthyl, benzocycloheptyl and the like. The cycloalkyl group may be optionally substituted or unsubstituted, and when substituted, the substituent is preferably one or more groups independently selected from the group consisting of alkyl, alkenyl, alkynyl, alkoxyl, alkanethio, alkylamino, halogen, thiol, hydroxyl, nitro, cyano, cycloalkyl, heterocycloalkyl, aryl, heteroaryl, cycloalkoxyl, heterocycloalkoxyl, cycloalkylthio, heterocycloalkylthio, oxo, carboxyl and carboxylate group.

The term "heterocyclyl" refers to a saturated or partially unsaturated monocyclic or polycyclic hydrocarbon group containing from 3 to 20 ring atoms, wherein one or more ring atoms are selected from nitrogen, oxygen and $S(O)_m$

(m is an integer from 0 to 2), but excluding the ring moiety of -O-O-, -O-S- or -S-S-, and the remaining ring atoms are carbon. It preferably has 3 to 12 ring atoms with 1 to 4 heteroatoms, more preferably 3 to 8 ring atoms with 1 to 3 heteroatoms, most preferably 5 to 7 ring atoms with 1 to 2 or 1 to 3 heteroatoms. Non-limiting examples of monocyclic heterocyclic groups include pyrrolidinyl, imidazolidinyl, tetrahydrofuranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, preferably 1, 2, 5-oxadiazolyl, pyranyl or morpholinyl. Polycyclic heterocyclic groups include spiro, fused and bridged heterocyclic groups.

The term "spiroheterocyclyl" refers to a polycyclic heterocyclic group that shares one atom (called a spiro atom) between 5- to 20-membered single rings, wherein one or more ring atoms are selected from nitrogen, oxygen or $S(O)_m$ (m is an integer from 0 to 2), and the remaining ring atoms are carbon. It may contain one or more double bonds, but none of the rings have a fully conjugated π-electron system. It is preferably 6- to 14-membered, more preferably 7- to 10-membered. The spiroheterocyclyl group may be classified into a monospiroheterocyclic group, a bispiroheterocyclic group or a polyspirocyclic group depending on the number of spiro atoms between the rings, preferably a monospiroheterocyclic and a bispiroheterocyclic group, more preferably, 4-membered/4-membered, 4-membered/5-membered, 4-membered/6-membered, 5-membered/5-membered or 5-membered/6-membered monospiroheterocyclic group. Non-limiting examples of spiroheterocyclyl groups include:

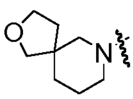 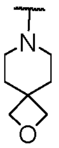 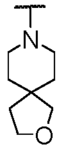 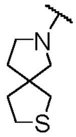 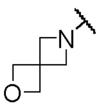 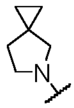 .

The term "fused heterocyclyl" refers to a 5- to 20-membered polycyclic heterocyclic groups wherein each ring in the system shares an adjacent pair of atoms with other rings, and one or more rings may contain one or more double bond, but none of the rings have a fully conjugated π-electron system, wherein one or more ring atoms are heteroatoms selected from nitrogen, oxygen and $S(O)_m$ (m is an integer from 0 to 2), and the remaining ring atoms are carbon. It is preferably 6- to 14-membered, more preferably 7- to 10-membered. It may be classified into a bicyclic, tricyclic, tetracyclic or polycyclic fused heterocyclic group depending on the number of constituent rings, preferably a bicyclic or tricyclic ring, more preferably a 5-membered/5-membered or 5-membered/6-membered bicyclic fused heterocyclic group. Non-limiting examples of fused heterocyclyl groups include:

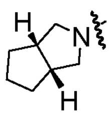 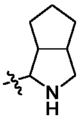 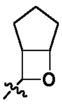 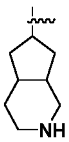 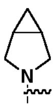 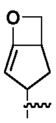 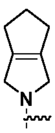 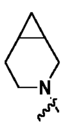

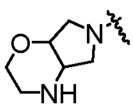 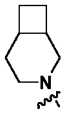 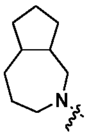 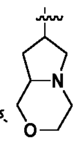 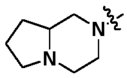 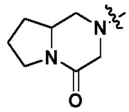 .

The term "bridge heterocyclyl" refers to a 5- to 14-membered polycyclic heterocyclic group in which any two rings share two atoms which are not directly bonded, which may contain one or more double bonds, but none of the rings have a fully conjugated π-electron system, wherein one or more ring atoms are heteroatoms selected from nitrogen, oxygen and $S(O)_m$ (m is an integer from 0 to 2), and the remaining ring atoms are carbon. It is preferably 6- to 14-membered, more preferably 7- to 10-membered. Depending on the number of constituent rings, it may be classified into a bicyclic, tricyclic, tetracyclic or polycyclic bridged heterocyclic group, preferably a bicyclic, tricyclic or tetracyclic ring, and more preferably a bicyclic or tricyclic ring. Non-limiting examples of bridge heterocyclyl groups include:

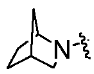 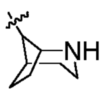 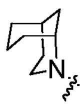 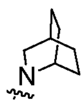

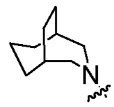 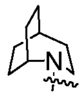 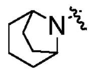 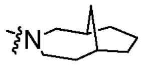 .

Said heterocyclyl ring may be fused to an aryl, heteroaryl or cycloalkyl ring, wherein the ring to which the parent structure is attached is a heterocyclic group. Non-limiting examples include:

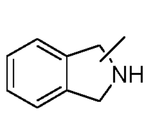 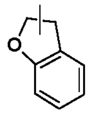 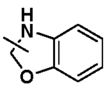 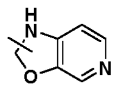 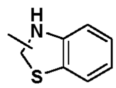

and the like.

The heterocyclic group may be optionally substituted or unsubstituted, and when substituted, the substituent is preferably one or more groups independently selected from the group consisting of alkyl, alkenyl, alkynyl, alkoxyl, alkylthiol, alkylamino, halogen, thiol, hydroxyl, nitro, cyano, cycloalkyl, heterocycloalkyl, aryl, heteroaryl, cycloalkoxyl, heterocycloalkoxyl, cycloalkylthio, heterocycloalkylthio, oxo, carboxyl and carboxylate group.

The term "aryl" refers to a 6- to 14-membered all-carbon monocyclic or fused polycyclic ring (the rings share an adjacent pair of atoms) having a conjugated $\pi$-electron system, preferably 6- to 10-membered, such as phenyl or naphthyl; more preferably phenyl. Said aryl ring may be fused to a heteroaryl, heterocyclyl or cycloalkyl ring, wherein the ring to which the parent structure is attached is an aryl ring. Non-limiting examples include:

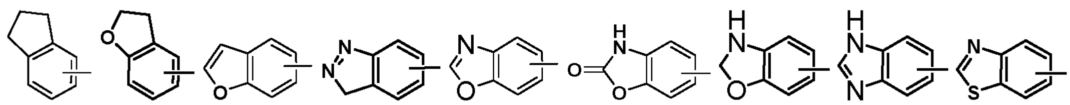

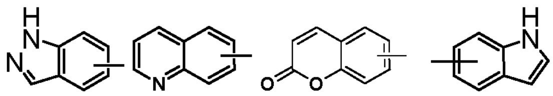 .

The aryl group may be substituted or unsubstituted, and when substituted, the substituent is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocycloalkyl, aryl, heteroaryl, cycloalkoxyl, heterocycloalkoxyl, cycloalkylthio, heterocycloalkylthio, carboxyl and carboxylate group.

The term "heteroaryl" refers to a heteroaromatic system containing 5 to 14 ring atoms and 1 to 4 heteroatoms selected from the group consisting of oxygen, sulfur and nitrogen. The heteroaryl group is preferably 5- to 10-membered with 1 to 3 heteroatoms; more preferably 5- or 6-membered with 1 to 2 heteroatoms. The heteroaryl is preferably for example imidazolyl, furyl, thienyl, thiazolyl, pyrazolyl, oxazolyl, pyrrolyl, tetrazolyl, pyridyl, pyrimidinyl, thiadiazole, pyrazinyl and the like; more preferably imidazolyl, thiazolyl, pyrazolyl or pyrimidinyl, thiazolyl; most preferably pyrazolyl or thiazolyl. Said heteroaryl ring may be fused to an aryl, heterocyclic or cycloalkyl ring, wherein the ring to which the parent structure is attached is a heteroaryl ring. Non-limiting examples include:

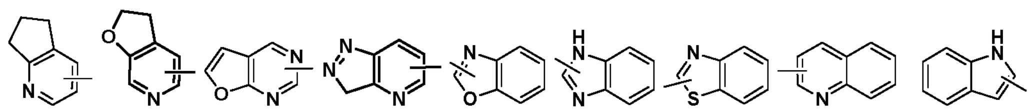 .

The heteroaryl group may be optionally substituted or unsubstituted, and when substituted, the substituent is preferably one or more groups independently selected from the group consisting of alkyl, alkenyl, alkynyl, alkoxyl, alkylthiol, alkylamino, halogen, thiol, hydroxyl, nitro, cyano, cycloalkyl, heterocycloalkyl, aryl, heteroaryl, cycloalkoxyl, heterocycloalkoxyl, cycloalkylthio, heterocycloalkylthio, carboxyl and carboxylate group.

The term "alkoxyl" refers to -O-(alkyl) and -O-(unsubstituted cycloalkyl), wherein alkyl is as defined above. Non-limiting examples of alkoxyl groups include methoxyl, ethoxyl, propoxyl, butoxyl, cyclopropoxyl, cyclobutoxyl, cyclopentyloxyl, cyclohexyloxyl. The alkoxyl group may be optionally substituted or unsubstituted, and when substituted, the substituent is preferably one or more groups independently selected from the group consisting of alkyl, alkenyl, alkynyl,

alkoxyl, alkylthiol, alkylamino, halogen, thiol, hydroxyl, nitro, cyano, cycloalkyl, heterocycloalkyl, aryl, heteroaryl, cycloalkoxyl, heterocycloalkoxyl, cycloalkylthio, heterocycloalkylthio, carboxyl and carboxylate.

The term "haloalkyl" refers to an alkyl group substituted by one or more halogens, wherein alkyl is as defined above.

The term "haloalkoxyl" refers to an alkoxyl group substituted by one or more halogens, wherein alkoxyl is as defined above.

The term "hydroxyl" refers to -OH.

The term "halogen" refers to fluorine, chlorine, bromine or iodine.

The term "amino" refers to $-NH_2$.

The term "cyano" refers to -CN.

The term "nitro" refers to $-NO_2$.

The term "oxo" refers to =O.

The term "carboxyl" refers to -C(O)OH.

The term "thiol" refers to -SH.

The term "ester group" refers to -C(O)O(alkyl) or -C(O)O(cycloalkyl), wherein alkyl and cycloalkyl are as defined above.

The term "acyl" refers to a compound containing a -C(O)R' group, wherein R' is alkyl, cycloalkyl, heterocyclyl, aryl or heteroaryl.

"Optional" or "optionally" means that the subsequently described event or environment may, but need not, occur, including where the event or environment occurs or does not occur. For example, "heterocyclic group optionally substituted with an alkyl group" means that an alkyl group may be, but not necessarily present, and the description includes the case where the heterocyclic group is substituted with an alkyl group and the case where the heterocyclic group is not substituted with an alkyl group.

"Substituted" refers to one or more hydrogen atoms in the group, preferably up to 5, more preferably 1 to 3 hydrogen atoms are independently replaced by a corresponding number of substituents. It goes without saying that the substituents are only in their possible chemical positions. Those skilled in the art will be able to determine (by experiment or theory) substitutions that may or may not be possible without undue effort. For example, an amino group or a hydroxyl group having a free hydrogen may be unstable when combined with a carbon atom having an unsaturated (e.g., olefinic) bond.

"Pharmaceutical composition" means a mixture comprising one or more of the compounds described herein, or a physiologically/pharmaceutically acceptable salt or a prodrug thereof, and other chemical components, as well as other components such as physiological/pharmaceutically acceptable carriers and excipients. The purpose of the pharmaceutical composition is to promote the administration to the organism, which facilitates the absorption of the active ingredient and thereby exerts biological activity.

"Pharmaceutically acceptable salt" refers to a salt of the compound of the invention which is safe and effective for use in mammals and which possesses the desired biological activity.

Methods for synthesizing the compound of the present invention

In order to achieve the purpose of the present invention, the following schemes are applied for the preparation of the compound of formula (I).

When Z is $CR^2$, the compound of formula (I) is synthesized according to Scheme 1:

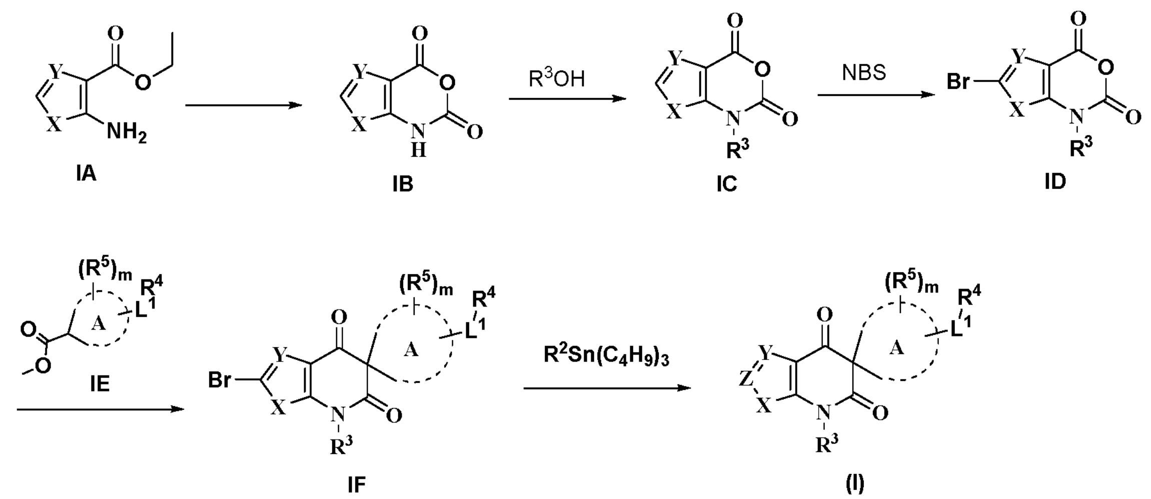


IA, IB, IC, ID, IE, IF, (I)

Scheme 1

Step 1) compound IA is subjected to a hydrolysis reaction, and is then reacted with diphosgene under basic condition to obtain compound IB, wherein said basic condition is preferably potassium hydroxide;

Step 2) Compound IB is reacted with $R^3OH$, diisopropyl azodicarboxylate and triphenylphosphine to obtain compound IC;

Step 3) Compound IC is reacted with NBS to obtain compound ID;

Step 4) Compound ID is reacted with compound IE under basic condition to obtain compound IF, wherein said basic condition is preferably LDA;

Step 5) Compound IF is reacted with $R^2Sn(C_4H_9)_3$ in the presence of a catalyst to obtain a compound of formula (I), wherein said catalyst is preferably bis(triphenylphosphorus)palladium dichloride.

X, Y, ring A, $L^1$, $R^2$, $R^3$, $R^4$, $R^5$ and m are as defined in formula (I).

## DESCRIPTION OF THE DRAWINGS

Figure 1 is a single crystal X-ray diffraction pattern of the compound 8c prepared in Example 8, which is an overlay spectrum of the simulated pattern and the test pattern of the single crystal, wherein the upper line is the test pattern, and the lower line is the simulated pattern.

Figure 2 is the crystal structure of compound 8c, which shows atomic shift parameters with a probability of 50%.

Figure 3 is the crystal structure of compound 8c in one unit crystal cell, with hydrogen omitted for clarity.

## DETAILED DESCRIPTION OF THE INVENTION

The compounds of the present invention and their preparation are further illustrated with reference to the following examples. These examples illustrate some methods of preparing or using said compounds. However, it should be understood that these examples are not intended to limit the scope of the invention. Variations of the invention now known or to be further developed are considered to fall within the scope of the invention described and claimed herein.

The compound of the present invention is prepared by using convenient starting materials and general preparation procedures. The present invention provides typical or preferential reaction conditions, such as reaction temperature, time, solvent, pressure, and molar ratio of reactants. However, unless otherwise specified, other reaction conditions can also be adopted. Optimal conditions may vary with the use of specific reactants or solvents, but under normal circumstances, reaction optimization steps and conditions can be determined.

In addition, some protecting groups may be used in the present invention to protect certain functional groups from unnecessary reactions. The protecting groups suitable for various functional groups and their protection or deprotection conditions are well known to those skilled in the art. For example, "Protective Groups in Organic Synthesis" by T. W. Greene and G. M. Wuts (3rd edition, Wiley, New York, 1999 and citations in the book) describes in detail the protection or deprotection of a large number of protective groups.

The separation and purification of compounds and intermediates adopt appropriate methods and steps according to specific needs, such as filtration, extraction, distillation, crystallization, column chromatography, preparative thin-layer chromatography, preparative high performance liquid chromatography or a combination of the above methods. The examples described in the present invention can be referred for the specific method of use. Of course, other similar separation and purification methods can also be used. They can be characterized using conventional methods (including physical constants and spectral data).

The structure of the compounds are determined by nuclear magnetic resonance (NMR) or/and mass spectrometry (MS). The NMR shift is given in units of $10^{-6}$ (ppm). The NMR is determined by using a Brukerdps 300 nuclear magnetic instrument. The solvent is deuterated dimethyl sulfoxide (DMSO-$d_6$), deuterated chloroform ($CDCl_3$), deuterated methanol ($CD_3OD$), and the internal standard is tetramethylsilane (TMS).

MS is determined using a LC/MS-2020 mass spectrometer (manufacturer: Shimadzu) (QDa Detector).

The preparative liquid chromatography applies Waters high performance liquid chromatograph (Waters 2545 binary gradient pump, 2767 sample manager, 2489 UV/visual detector, single C18, 5 μm, 19 mm×250 mm) (manufacturer: Waters).

The thin layer chromatography (TLC) applies Qingdao Ocean Chemical GF254 silica gel plate, and the specification is 0.15 mm to 0.2 mm for analysis, and 0.4 mm to 0.5 mm for separation and purification.

Column chromatography generally applies Qingdao Ocean Silicone 100 to 200 mesh and 200 to 300 mesh silica gel as carrier.

The known starting materials of the present invention can be synthesized by or according to methods known in the art, or can be purchased from WHmall, Beijing Ouhe, Sigma, J&K Chemicals, Yi Shiming, Shanghai Shuya, Shanghai Innochem, Energy Chemical, Shanghai Bide Pharmatech and other companies.

Unless otherwise specified in the examples, the reactions can all be carried out under nitrogen atmosphere.

Argon atmosphere or nitrogen atmosphere means that the reaction flask is connected to an argon or nitrogen balloon having a volume of about 1 L.

The reaction solvent, organic solvent or inert solvent are each expressed as the solvent used that does not participate in the reaction under the described reaction conditions, including, for example, benzene, toluene, acetonitrile, tetrahydrofuran (THF), dimethylformamide (DMF), chloroform, dichloromethane, ether, methanol, N-methylpyrrolidone (NMP), pyridine, and the like. Unless otherwise specified in the examples, a solution means an aqueous solution.

The chemical reaction described in the present invention is generally carried out under normal pressure. The reaction temperature is between -78°C and 200°C. The reaction time and conditions are, for example, between -78°C and 200°C under one atmospheric pressure, and completed within about 1 to 24 hours. If the reaction is overnight, then the reaction time is generally 16 hours. Unless otherwise specified in the examples, the reaction temperature is room temperature, which is 20°C to 30 °C.

The progress of the reaction in the examples is monitored by thin layer chromatography (TLC). The developing system includes: A: dichloromethane and methanol system, B: petroleum ether and ethyl acetate system, C: acetone. The volume ratio of the solvents is adjusted depending on the polarity of the compound.

The eluent system of column chromatography and the developing system of TLC for the purification of the compound include: A: dichloromethane and methanol system, B: petroleum ether and ethyl acetate system. The volume ratio of the solvents is adjusted depending on the polarity of the compound, and a small amount of basic or acidic reagents such as triethylamine and trifluoroacetic acid may be added for adjustment.

Unless otherwise defined, all professional and scientific terms used herein have the same meaning as those familiar to those skilled in the art. In addition, any methods and materials similar or equivalent to the content described herein can be applied to the method of the present invention.

Abbreviations

μL = microliter
μM = micromole
NMR = nuclear magnetic resonance;
Boc = tert-butoxycarbonyl
br = broad peak
d = doublet
δ = chemical shift
°C = degrees celsius
dd = double doublet
DIPEA = diisopropylethylamine

DMF = N,N-dimethylformamide
DMSO = dimethyl sulfoxide
DCM = dichloromethane
EA = ethyl acetate
EDCI = 1-ethyl-(3-dimethylaminopropyl) carbodiimide hydrochloride
HATU = 2-(7-azobenzotriazole)-N,N,N',N'-tetramethylurea hexafluorophosphate
HOBt= 1-hydroxybenzotriazole
HPLC = high performance liquid chromatography
Hz = hertz
$IC_{50}$ = the concentration that inhibits 50% of the activity
J = coupling constant (Hz)
LC-MS = liquid chromatography-mass spectrometry
LDA = lithium diisopropylamide
m = multiplet
$M+H^+$ = mass of parent compound + one proton
mg = milligram
mL = milliliter
mmol = millimole
MS = mass spectrum
m/z = mass-to-charge ratio
nM = nanomole
NBS = N-bromosuccinimide
PE = petroleum ether
ppm = parts per million
PyBrOP = tripyrrolidinyl phosphonium bromide hexafluorophosphate
s = singlet
t = triplet
TEA = triethylamine
TBDPS = tert-butyl diphenyl silicon
TFA = trifluoroacetic acid
THF = tetrahydrofuran
LDA = lithium diisopropylamide
HMPA = hexamethyl phosphoric acid triamide.

Example 1: Preparation of 3'-methyl-2'-(oxazol-2-yl)-7'-(2-phenyl-2-((tetrahydro-2*H*-pyran-4-yl)oxy)ethyl)-4'*H*-spiro[cyclobutane-1,5'-thieno[2,3-b]pyridine]-4',6'(7'*H*)-dione (1)

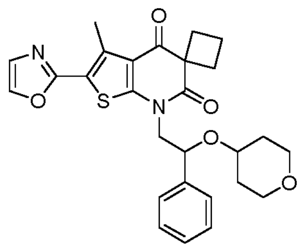

**1**

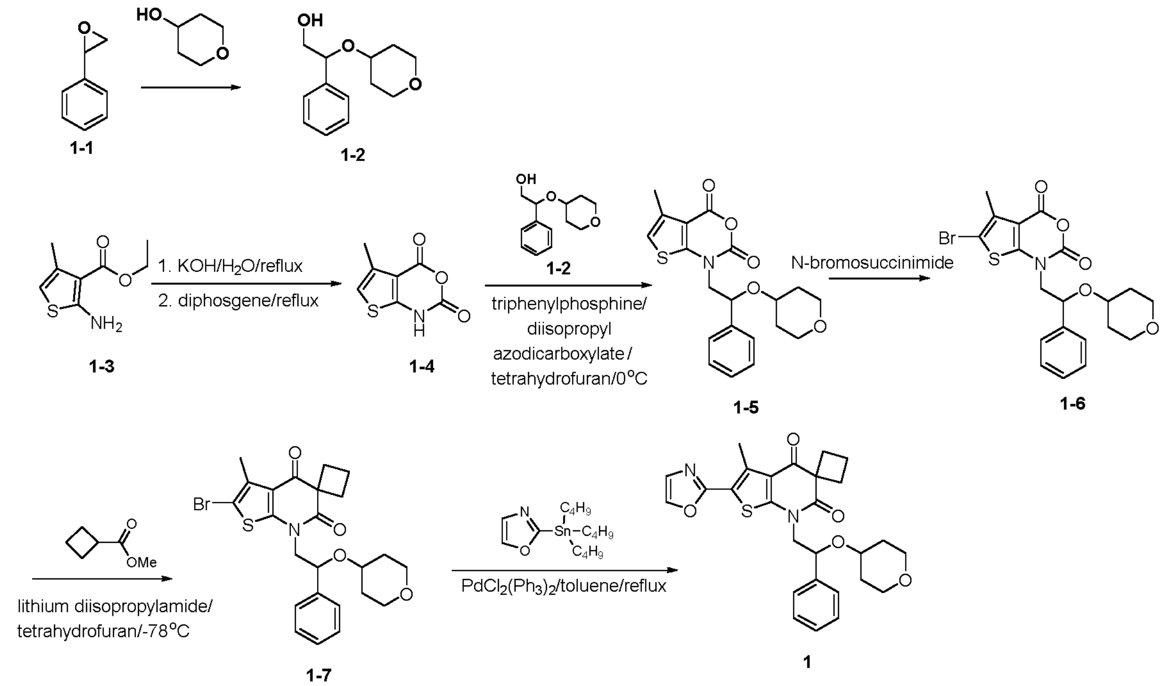


1-1 1-2 1-3 1-4 1-5 1-6 1-7 1

Step 1: Preparation of 2-phenyl-2-((tetrahydro-2*H*-pyran-4-yl)oxy)ethan-1-ol (compound 1-2)

Ferric chloride (12.0 g, 0.750 mol) was added to 4-tetrahydropyranol (102 g, 1.00 mol) at room temperature. The mixed solution was cooled to 0°C, and phenyl oxirane (1-1) (60.0 g, 0.500 mol) was slowly added dropwise. After the addition, the reaction mixture was warmed to room temperature and stirred for 4 hours. The reaction was quenched by adding water (2000 mL) and was extracted with ethyl acetate (3 × 1000 mL). The organic phases were combined and then washed with saturated brine (1000 mL), dried over anhydrous sodium sulfate, and filtered. The filtrate was concentrated under reduced pressure. The residues were purified by silica gel column chromatography (mobile phase: ethyl acetate/petroleum ether = 20% to 50%) to obtain the title compound (18.0 g, 16.2%, a mixture of two optical isomers) as a colorless oil.

$^1$H-NMR ($CDCl_3$) $\delta$: 7.41-7.29 (m, 5 H), 4.85 (m, 1 H), 4.62 (dd, *J* = 4.2, 8.1 Hz, 1 H), 4.03-3.89 (m, 2 H), 3.73-3.50 (m, 3 H), 3.46-3.34 (m, 2 H), 2.03-1.96 (m, 1 H), 1.79-1.61 (m, 3 H).

LC-MS: m/z 245.15 $[M + Na]^+$.

Step 2: Preparation of 5-methyl-2*H*-thieno[2,3-d][1,3]oxazine-2,4(1*H*)-dione (compound 1-4)

The compound ethyl 2-amino-4-methylthiophen-3-carboxylate (1-3) (20.0 g, 0.108 mol) was dissolved in water (400 mL) at room temperature, and potassium hydroxide solid (12.1 g, 0.216 mol) was added. The reaction solution was heated to 100°C and refluxed for 6 hours. The resulting solution was cooled to 0°C, and slowly added with diphosgene (21.2 g, 0.108 mol) dropwise. After addition, the reaction mixture was warmed to room temperature and stirred for 3 hours. A solid was gradually precipitated out during the reaction. After the reaction, the obtained solid was filtered, washed with water to neutrality, then washed with petroleum ether (200 mL), and dried under reduced pressure to obtain the title compound (7.00 g, 35.3%) as a brown solid.

LC-MS: m/z 184.25 $[M + H]^+$.

Step 3: Preparation of 5-methyl-1-(2-phenyl-2-((tetrahydro-2*H*-pyran-4-yl)oxy)ethyl)-2*H*-thieno[2,3-d][1,3]oxazine-2,4(1*H*)-dione (compound 1-5)

5-Methyl-2*H*-thieno[2,3-d][1,3]oxazine-2,4(1*H*)-dione (1-4) (7.00 g, 38.2 mmol) was dissolved in dry tetrahydrofuran (150 mL) under nitrogen atmosphere at room temperature, and 2-phenyl-2-((tetrahydro-2*H*-pyran-4-yl)oxy)ethan-1-ol (1-2) (16.9 g, 76.5 mmol) and triphenylphosphorus (20.0 g, 76.5 mmol) was added successively. The reaction solution was cooled to 0°C, and slowly added with diisopropyl azodicarboxylate (15.5 g, 76.5 mmol) dropwise. After addition, the reaction mixture was warmed to room temperature and reacted for 16 hours. After the reaction, the reaction solution was concentrated under reduced pressure. The residues were purified by silica gel column chromatography (mobile phase: ethyl acetate/petroleum ether = 10% to 50%) to obtain the title compound (4.50 g, 30.4 %) as

a white solid.

LC-MS: m/z 388.15 $[M + H]^+$.

Step 4: Preparation of 6-bromo-5-methyl-1-(2-phenyl-2-((tetrahydro-2*H*-pyran-4-yl)oxy)ethyl)-2*H*-thieno[2,3-d][1,3]oxazine-2,4(1*H*)-dione (compound 1-6)

5-Methyl-1-(2-phenyl-2-((tetrahydro-2*H*-pyran-4-yl)oxy)ethyl)-2*H*-thieno[2,3-d] [1,3]oxazine-2,4(1*H*)-dione (1-5) (4.50 g, 11.6 mmol) was dissolved in chloroform (100 mL) at room temperature, and then added with N-bromosuccimide (2.07 g, 11.6 mmol). The reaction mixture was stirred at room temperature for 3 hours, and then quenched by adding 5% aqueous solution of sodium thiosulfate (10 mL). The mixture was diluted with dichloromethane (200 mL) and water (100 mL). After separation of the organic phase, the aqueous phase was extracted with dichloromethane (3 × 100 mL), and the combined organic phase was washed with saturated brine (50 mL), dried over anhydrous sodium sulfate and filtered. The filtrate was concentrated under reduced pressure. The residues were purified by silica gel column chromatography (mobile phase: ethyl acetate/petroleum ether = 10% to 40%) to obtain the title compound (3.1 g, 57.3 %, a mixture of two optical isomers) as a pale yellow solid.

$^1$H-NMR ($CDCl_3$) $\delta$: 7.47-7.37 (m, 5 H), 5.00 (dd, *J* = 2.4, 7.5 Hz, 1 H), 4.20 (dd, *J* = 1.8, 10.8 Hz, 1 H), 3.80-3.69 (m, 2 H), 3.64 (dd, *J* = 7.5, 10.8 Hz, 1 H), 3.51-3.45 (m, 1 H), 3.38-3.31 (m, 2 H), 1.80-1.71 (m, 2 H), 1.57-1.50 (m, 2 H), 1.42-1.34 (m, 1 H).

LC-MS: m/z 466.10 $[M + H]^+$.

Step 5: Preparation of 2'-bromo-3'-methyl-7'-(2-phenyl-2-((tetrahydro-2*H*-pyran-4-yl)oxy)ethyl)-4'*H*-spiro[cyclobutane-1,5'-thieno[2,3-b]pyridine]-4',6'(7'*H*)-dione (compound 1-7)

Methyl cyclobutanecarboxylate (98.6 mg, 0.860 mmol) was dissolved in dry THF (5 mL) under nitrogen atmosphere, and then added with LDA (0.860 mmol, 2 mol/L) at - 78°C. The mixture was stirred at this temperature for 1 hour, and then added with 6-bromo-5-methyl-1-(2-phenyl-2-((tetrahydro-2*H*-pyran-4-yl)oxy)ethyl)-2*H*-thieno[2,3-d][1,3]oxazine-2,4(1*H*)-dione (1-6) (200 mg, 0.430 mmol). The reaction solution was warmed to room temperature and stirred for 1 hour. After completion of the reaction, the reaction was quenched by adding water. The reaction system was extracted with ethyl acetate (3 × 20 mL), and the combined organic phase was washed with saturated brine (20 mL), dried over anhydrous sodium sulfate, and filtered. The filtrate was concentrated under reduced pressure. The residues were purified by silica gel column chromatography (mobile phase: ethyl acetate/petroleum ether = 10% to 40%) to obtain the title compound (110 mg, 50.8 %) as a white solid.

LC-MS: m/z 504.05 $[M + H]^+$.

Step 6: Preparation of 3'-methyl-2'-(oxazol-2-yl)-7'-(2-phenyl-2-((tetrahydro-2*H*-pyran-4-yl)oxy)ethyl)-4'*H*-spiro[cyclobutane-1,5'-thieno[2,3-b]pyridine]-4',6'(7'*H*)-dione (compound 1)

2-(Tributylstannyl)oxazole (53 mg, 0.15 mmol) and bis(triphenylphosphorus) palladium dichloride (0.01 mmol, 7.1 mg) were added to 2'-bromo-3'-methyl-7'-(2-phenyl-2-((tetrahydro-2*H*-pyran-4-yl)oxy)ethyl)-4'*H*-spiro[cyclobutane-1,5'-thieno[2,3-b]pyridine]-4',6'(7'*H*)-dione (1-7) (50 mg, 0.099 mmol) in a dry toluene solution (3 mL) under nitrogen atmosphere at room temperature. The reaction solution was heated and refluxed overnight. After completion of the reaction, the reaction was quenched by adding water. The reaction system was extracted with ethyl acetate (3 × 10 mL), and the combined organic phase was washed with saturated brine (20 mL), dried over anhydrous sodium sulfate, and filtered The filtrate was concentrated under reduced pressure. The residues were purified by preparative column chromatography (column type: XSelect CSH Prep C18 OBD column 5 μm, 19 × 150 mm; mobile phase A: water (10 mmol/L ammonium bicarbonate), mobile phase B: acetonitrile; flow rate: 25 mL/min; gradient: 50% to 53% acetonitrile; duration: 8 minutes; 254 nm) to obtain the title compound (10 mg, 20.4%, a mixture of two optical isomers) as a white solid.

$^1$H-NMR ($CDCl_3$) $\delta$: 8.00 (s, 1 H), 7.53-7.36 (m, 5 H), 7.30 (s, 1 H), 5.02-4.97 (m, 1 H), 4.31-4.25 (m, 1 H), 3.95-3.87 (m, 1 H), 3.77-3.61 (m, 2 H), 3.55-3.47 (m, 1 H), 3.42-3.33 (m, 2 H), 2.84 (s, 3 H), 2.70-2.47 (m, 4 H), 2.24-2.13 (m, 2 H), 1.83-1.64 (m, 2 H), 1.55-1.38 (m, 2 H).

LC-MS: m/z 493.15 $[M + H]^+$.

Example 2: Preparation of 3'-methyl-2'-(oxazol-2-yl)-7'-(2-phenyl-2-((tetrahydro-2*H*-pyran-4-yl)oxy)ethyl)-4'*H*-spiro[cyclopentane-1,5'-thieno[2,3-b]pyridine]-4',6'(7'*H*)-dione (2)

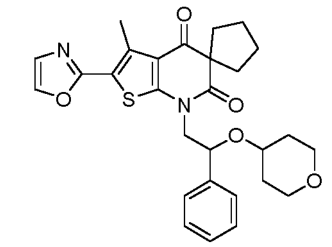

2

The preparation method was the same as in Example 1, except that methyl cyclopentanecarboxylate was used instead of methyl cyclobutanecarboxylate, to obtain the title compound 2.

$^1$H-NMR ($CDCl_3$) $\delta$: 7.73 (d, *J* = 0.6 Hz, 1 H), 7.44-7.50 (m, 2 H), 7.36-7.42 (m, 3 H), 7.24 (d, *J* = 0.6 Hz, 1 H), 4.96 (dd, *J* = 3.9, 9.6 Hz, 1 H), 4.26 (dd, *J* = 3.6, 14.4 Hz, 1 H), 3.70-3.88 (m, 3 H), 3.41-3.50 (m, 1 H), 3.30-3.36 (m, 2 H), 2.84 (s, 3 H), 2.17-2.27 (m, 3 H), 2.03-2.07 (m, 1 H), 1.88-1.93 (m, 4 H), 1.67-1.77 (m, 2 H), 1.49-1.61 (m, 1 H), 1.37-1.47 (m, 1 H).

LC-MS: m/z 507.35 [M + H]$^+$.

Example 3: Preparation of 3'-methyl-2'-(oxazol-2-yl)-4',6'-dioxo-7'-(2-phenyl-2-((tetrahydro-2*H*-pyran-4-yl)oxy)ethyl)-6',1'-dihydro-4'*H*-spiro[cyclopentane-1,5'-thieno[2,3-b]pyridine]-3-carboxylic acid (3)

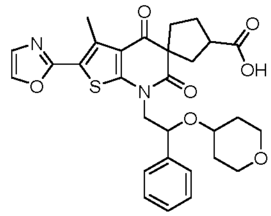

3

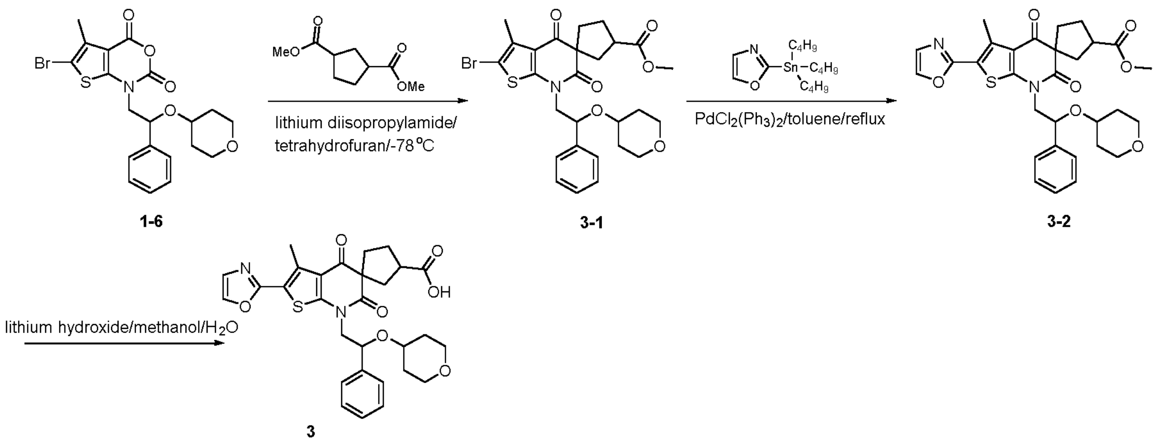


Step 1: Preparation of methyl 2'-bromo-3'-methyl-2'-4',6'-dioxo-7'-(2-phenyl-2-((tetrahydro-2*H*-pyran-4-yl)oxy)ethyl)-6',1'-dihydro-4'*H*-spiro[cyclopentane-1,5'-thieno[2,3-b]pyridine]-3-carboxylate (3-1)

Methyl cyclopentane-1,3-dicarboxylate (120 mg, 0.643 mmol) was dissolved in dry THF (5 mL) under nitrogen atmosphere, and then added with LDA (0.643 mmol, 2 mol/L) at -78°C. The mixture was stirred at this temperature for 1 hour and then added with 6-bromo-5-methyl-1-(2-phenyl-2-((tetrahydro-2*H*-pyran-4-yl)oxy)ethyl)-2*H* -thieno[2,3-d][1,3]oxazine-2,4(1*H*)-dione (1-6) (250 mg, 0.536 mmol). The reaction solution was warmed to room temperature and stirred overnight. After completion of the reaction, the reaction was quenched by adding water. The reaction system was extracted with ethyl acetate (3 × 20 mL), and the combined organic phase was washed with saturated brine (20 mL), dried over anhydrous sodium sulfate, and filtered. The filtrate was concentrated under reduced pressure. The residues

were purified by column chromatography silica gel (mobile phase: ethyl acetate/petroleum ether = 30% to 70%) to obtain the title compound (136 mg, 44.0 %) as a white solid.

LC-MS: m/z 598.15 $[M + Na]^+$.

Step 2: Preparation of methyl 3'-methyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylate (3-2)

2-(Tributylstannyl)oxazole (348 mg, 0.971 mmol) and bis(triphenylphosphorus) palladium dichloride (170 mg, 0.243 mmol) were added to methyl 2'-bromo-3'-methyl-2'-4',6'-dioxo-7'-(2-phenyl-2-((tetrahydro-2*H*-pyran-4-yl)oxy)ethyl)-4'*H*-spiro[cyclopentane-1, 5'-thieno[2,3-b]pyridine]-3-carboxylate (3-1) (280 mg, 0.486 mmol) in a dry toluene solution (10 mL) under nitrogen atmosphere at room temperature. The reaction solution was heated and refluxed overnight. After completion of the reaction, the reaction was quenched by adding water. The reaction system was extracted with ethyl acetate (3 × 20 mL), and the combined organic phase was washed with saturated brine (30 mL), dried over anhydrous sodium sulfate and filtered. The filtrate was concentrated under reduced pressure. The residues were purified by column chromatography silica gel (mobile phase: ethyl acetate/petroleum ether = 20% to 40%) to obtain the title compound (136 mg, 50.0 %) as a pale yellow oil.

LC-MS: m/z 579.35 $[M + H]^+$.

Step 3: Preparation of 3'-methyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylic acid (3)

Lithium hydroxide (3.4 mg, 0.14 mmol) was added to methyl 3'-methyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylate (3-2) (40 mg, 0.071 mmol) in a methanol/water mixed solution (2 mL, methanol/water = 1/1) at room temperature. The reaction solution was stirred overnight at room temperature. After completion of the reaction, the organic solvent was removed under reduced pressure, then the reaction solution was adjusted to pH 2 with dilute hydrochloric acid (pH = 1). The reaction system was extracted with ethyl acetate (3×20 mL), and the combined organic phase was washed with saturated brine (20 mL), dried over anhydrous sodium sulfate, and filtered. The filtrate was concentrated under reduced pressure. The residues were purified by silica gel column chromatography (mobile phase: ethyl acetate/petroleum ether = 20% to 60%) to obtain the title compound (14 mg, 36.0 %, a mixture of eight optical isomers) as a white solid.

$^1H$-NMR ($CDCl_3$) $\delta$: 7.75 (d, *J* = 0.6 Hz, 1 H), 7.35-7.49 (m, 5 H), 7.28 (d, *J* = 0.6 Hz, 1 H), 4.94-5.01 (m, 1 H), 4.16-4.40 (m, 1 H), 3.71-3.83 (m, 3 H), 3.42-3.49 (m, 1 H), 3.26-3.38 (m, 2 H), 2.84 (m, 3 H), 2.48-2.70 (m, 2 H), 2.18-2.30 (m, 4 H), 1.70-1.83 (m, 2 H), 1.56-1.58 (m, 1 H), 1.41-1.45 (m, 1 H).

LC-MS m/z 551.35 $[M + H]^+$.

Example 4: Preparation of 3,3'-dimethyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylic acid (4)

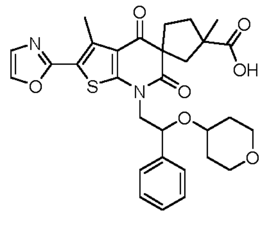

**4**

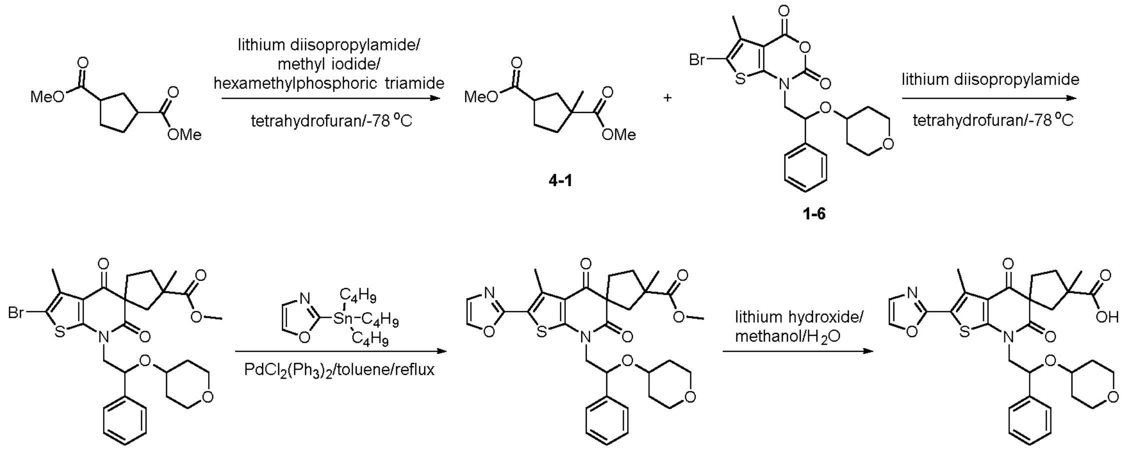


4-1 + 1-6

4-2 4-3 4

Step 1: Preparation of methyl 1-methyl-cyclopentane-1,3-dicarboxylate (4-1)

Methyl cyclopentane-1,3-dicarboxylate (0.552 g, 5.52 mmol) and hexamethyl phosphate triamide (3.10 g, 17.2 mmol) were dissolved in dry THF (10 mL) under nitrogen atmosphere, and then added with LDA (16.5 mmol, 2 mol/L) at -78°C. The mixture was stirred at this temperature for 1 hour and then added with methyl iodide (1.22 g, 8.59 mmol). The reaction solution was warmed to room temperature and stirred overnight. After completion of the reaction, the reaction was quenched by adding water. The reaction system was extracted with ethyl acetate (3×40 mL), and the combined organic phase was washed with saturated brine (40 mL), dried over anhydrous sodium sulfate, and filtered. The filtrate was concentrated under reduced pressure. The residues were purified by silica gel column chromatography (mobile phase: ethyl acetate/petroleum ether = 20% to 40%) to obtain the title compound (630 mg, 73.0 %) as a pale yellow oil.

$^1$H-NMR ($CDCl_3$) $\delta$: 3.69 (s, 6 H), 2.87-3.00 (m, 1 H), 2.37-2.52 (m, 1 H), 2.12-2.31 (m, 1 H), 1.83-2.02 (m, 2 H), 1.49-1.77 (m, 2 H), 1.30 (s, 3 H).

Step 2: Preparation of methyl 2'-bromo-3,3'-dimethyl-2'-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylate (4-2)

Methyl 1-methyl-cyclopentane-1,3-dicarboxylate (4-1) (322 mg, 1.61 mmol) was dissolved in dry THF (20 mL) under nitrogen atmosphere, and then added with LDA (2.04 mmol, 2 mol/L) at -78°C. The mixture was stirred at this temperature for 1 hour and then added with 6-bromo-5-methyl-1-(2-phenyl-2-((tetrahydro-2*H*-pyran-4-yl)oxy)ethyl)-2*H*-thieno[2,3-d][1,3]oxazine-2,4(1*H*)-dione (1-6) (250 mg, 0.536 mmol). The reaction solution was warmed to room temperature and stirred overnight. After completion of the reaction, the reaction was quenched by adding water. The reaction system was extracted with ethyl acetate (3×40 mL), and the combined organic phase was washed with saturated brine (20 mL), dried over anhydrous sodium sulfate, and filtered. The filtrate was concentrated under reduced pressure. The residues were purified by silica gel column chromatography (mobile phase: ethyl acetate/petroleum ether = 20% to 40%) to obtain the title compound (260 mg, 82.0 %) as a pale yellow oil.

LC-MS: m/z 590.25 $[M + H]^+$.

Step 3: Preparation of methyl 3,3'-dimethyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylate (4-3)

2-(Tributylstannyl)oxazole (303 mg, 0.847 mmol) and bis(triphenylphosphorus) palladium dichloride (149 mg,

0.212 mmol) were added to methyl 2'-bromo-3,3'-dimethyl-2'-4',6'-dioxo-7'-(2-phenyl-2-((tetrahydro-2*H*-pyran-4-yl)oxy)ethyl)-4'*H*-spiro[cyclopentane-2,5'-thieno[2,3-b]pyridine]-3-carboxylate (4-2) (0.423 mmol, 250 mg) in a dry toluene solution (10 mL) under nitrogen atmosphere at room temperature. The reaction solution was heated and refluxed overnight. After completion of the reaction, the reaction was quenched by adding water. The reaction system was extracted with ethyl acetate (3×20 mL), and the combined organic phase was washed with saturated brine (30 mL), dried over anhydrous sodium sulfate, and filtered. The filtrate was concentrated under reduced pressure. The residues were purified by silica gel column chromatography (mobile phase: ethyl acetate/petroleum ether = 20% to 40%) to obtain the title compound (170 mg, 69.0 %) as a pale yellow oil.

$^1$H-NMR (MeOD) $\delta$: 7.74 (m, 1 H), 7.37-7.50 (m, 5 H), 7.28 (m, 1 H), 4.89-5.02 (m, 1 H), 4.29-4.37 (m, 1 H), 4.12-4.19 (m, 1 H), 3.71-3.87 (m, 5 H), 3.42-3.49 (m, 1 H), 3.28-3.37 (m, 2 H), 2.88-3.01 (m, 1 H), 2.82 (s, 3 H), 2.43-2.52 (m, 1 H), 2.18-2.35 (m, 2 H), 2.03-2.14 (m, 1 H), 1.68-1.84 (m, 4 H), 1.54-1.61 (m, 1 H), 1.40 (s, 3 H).

LC-MS: m/z 579.25 $[M + H]^+$.

Step 4: Preparation of 3,3'-dimethyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylic acid (4)

Lithium hydroxide (13 mg, 0.56 mmol) was added to 3,3'-dimethyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclopentane-1,5'-thieno[2,3-b]pyridine]-3-carboxylate (4-3) (0.16 g, 0.28 mmol) in a methanol/water mixed solution (6 mL, methanol/water = 1/1) at room temperature. The reaction solution was stirred overnight at room temperature. After completion of the reaction, the organic solvent was removed under reduced pressure, then the reaction solution was adjusted to pH 2 with dilute hydrochloric acid (pH = 1). The reaction system was extracted with ethyl acetate (3×20 mL), and the combined organic phase was washed with saturated brine (20 mL), dried over anhydrous sodium sulfate and filtered. The filtrate was concentrated under reduced pressure. The residues were purified by silica gel column chromatography (mobile phase: ethyl acetate/petroleum ether = 20% to 60%) to obtain the title compound (60 mg, 38 %, a mixture of eight optical isomers) as a white solid.

$^1$H-NMR ($CDCl_3$) $\delta$: 7.74 (m, 1 H), 7.37-7.50 (m, 5 H), 7.28 (m, 1 H), 4.92-4.97 (m, 1 H), 4.35-4.41 (m, 1 H), 3.67-3.83 (m, 3 H), 3.43-3.48 (m, 1 H), 3.29-3.38 (m, 2 H), 2.90-2.97 (m, 1 H), 2.79 (s, 3 H), 2.39-2.47 (m, 2 H), 2.23-2.34 (m, 2 H), 2.09-2.18 (m, 1 H), 1.78-1.99 (m, 2 H), 1.43-1.62 (m, 2 H), 1.54 (s, 3 H).

LC-MS: m/z 565.35 $[M + H]^+$.

Example 5: Preparation of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclopentane-1,5'-thieno[2,3-b]pyridine]-3-carboxylic acid (5)

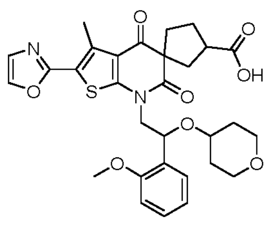

**5**

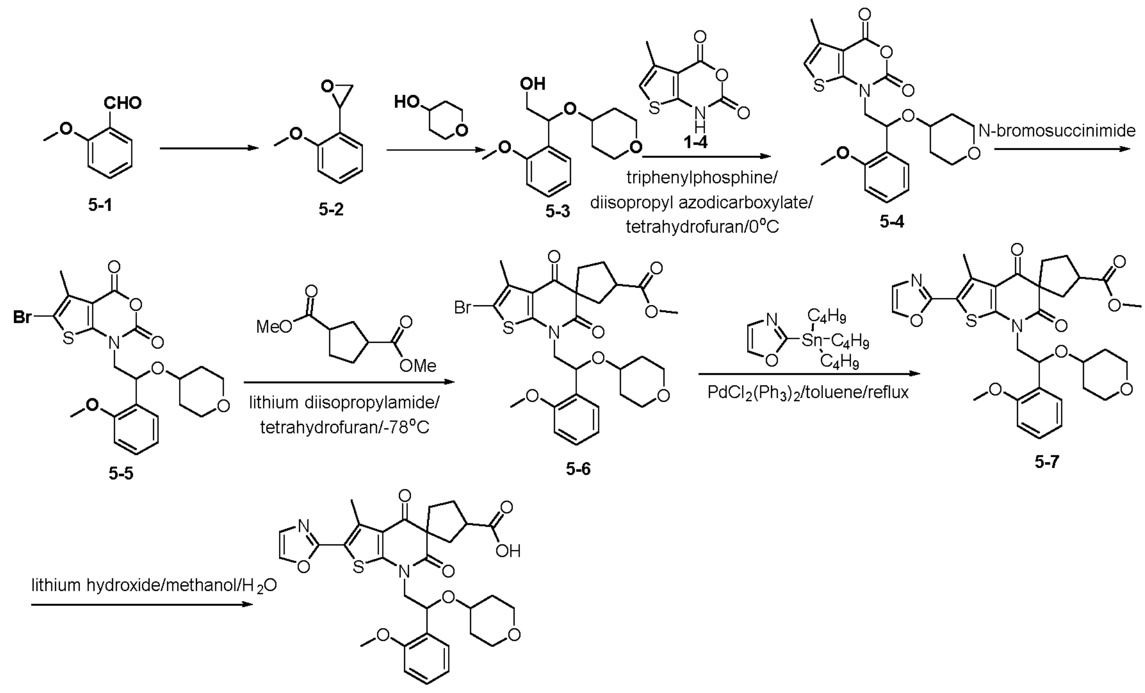


5-1 5-2 5-3 1-4 5-4 5-5 5-6 5-7 5

Step 1: Preparation of 2-methoxyphenyl oxirane (5-2)

Sodium hydride (35.3 g, 0.882 mol) was dissolved in dry dimethyl sulfoxide (800 mL) at room temperature. The mixed solution was cooled to 0°C, and then added with trimethylsulfoxide iodide (194 g, 0.882 mol). After stirring for 2 hours at 0°C, o-methoxybenzaldehyde (5-1) (100 g, 0.735 mol) was slowly added. The mixture was stirred at room temperature for 3 hours, and quenched by adding water (3000 mL). The reaction was extracted with ethyl acetate (3×3000 mL). The organic phases were combined and then washed with saturated brine (2000 mL), dried over anhydrous sodium sulfate and filtered. The filtrate was concentrated under reduced pressure. The title compound (105 g) was obtained as a pale yellow oil. The crude product was directly used in the next reaction without purification.

Step 2: Preparation of 2-phenyl-2-((tetrahydro-2*H*-pyran-4-yl)oxy)ethane-1-ol (5-3)

Ferric chloride (16.9 g, 0.105 mol) was added to 4-tetrahydropyranol (204 g, 1.40 mol) at room temperature. The mixed solution was cooled to 0°C, and then slowly added with crude 2-methoxyphenyl oxirane (5-2) (105 g, 0.700 mol) dropwise. The reaction mixture was warmed to room temperature and stirred for 4 hours. The reaction was quenched by adding water (2000 mL). The reaction system was extracted with ethyl acetate (3×1000 mL). The organic phases were combined and then washed with saturated brine (1000 mL), dried over anhydrous sodium sulfate and filtered. The filtrate was concentrated under reduced pressure. The residues were separated and purified by silica gel column chromatography (mobile phase: ethyl acetate/petroleum ether = 20% to 50%). The title compound (30.0 g, 17.0 %, a mixture of two optical isomers) was obtained as a colorless oil.

$^1$H-NMR ($CD_3OD$) $\delta$: 7.43 (dd, *J* = 1.8, 7.5 Hz, 1 H), 7.32-7.27 (m, 1 H), 7.00 (t, *J* = 7.5 Hz, 1 H), 6.90 (d, *J* = 7.5 Hz, 1 H), 5.09 (dd, *J* = 3.6, 8.4 Hz, 1 H), 4.03-3.90 (m, 2 H), 3.85 (s, 3 H), 3.70 (dd, *J* = 3.6, 11.4 Hz, 1 H), 3.59-3.51 (m, 2 H), 3.46-3.37 (m, 3 H), 2.07-1.98 (m, 1 H), 1.84-1.79 (m, 1 H), 1.72-1.61 (m, 2 H).

LC-MS: m/z 275.15 [M + Na]$^+$.

Step 3: Preparation of 1-(2-(2-methoxyphenyl-2-((tetrahydro-2*H*-pyran-4-yl)oxy)ethyl)-5-methyl-2*H*-thieno[2,3-d][1,3]oxazine-2,4(1*H*)-dione (5-4)

5-Methyl-2*H*-thieno[2,3-d][1,3]oxazine-2,4(1*H*)-dione (1-4) (5.00 g, 27.3 mmol) was dissolved in dry tetrahydrofuran (150 mL) under nitrogen atmosphere at room temperature, and added with 2-phenyl-2-((tetrahydro-2*H*-pyran-4-yl)oxy)ethane-1-ol (5-3) (13.77 g, 54.6 mmol) and triphenylphosphorus (14.3 g, 54.6 mmol) successively. The reaction solution was cooled to 0°C, and then slowly added with diisopropyl azodicarboxylate (11.0 g, 54.6 mmol) dropwise. After addition, the reaction solution was warmed to room temperature and reacted for 16 hours. After completion of the reaction, the reaction solution was concentrated under reduced pressure. The residues were purified by silica gel column chromatography (mobile phase: ethyl acetate/petroleum ether = 10% to 50%) to obtain the title compound (3.50 g, 30.6%) as a white solid.

LC-MS: m/z 418.15 $[M + H]^+$.

Step 4: Preparation of 6-bromo-1-(2-(2-methoxyphenyl-2-((tetrahydro-2*H*-pyran-4-yl)oxy)ethyl)-5-methyl-2*H*-thieno[2,3-d][1,3]oxazine-2,4(1*H*)-dione (5-5)

1-(2-(2-Methoxyphenyl-2-((tetrahydro-2*H*-pyran-4-yl)oxy)ethyl)-5-methyl-2*H*-thieno[2,3-d][1,3]oxazine-2,4(1*H*)-dione (5-4) (3.50 g, 8.37 mmol) was dissolved in chloroform (100 mL) at room temperature, and then added with N-bromosuccimide (1.49 g, 8.37 mmol). The mixture was reacted at room temperature for 3 hours. The reaction was quenched by adding 5% aqueous solution of sodium thiosulfate (10 mL), and diluted with dichloromethane (200 mL) and water (100 mL). After separation of the organic phase, the aqueous phase was extracted with dichloromethane (3×100 mL), and the combined organic phase was washed with saturated brine (100 mL), dried over anhydrous sodium sulfate and filtered. The filtrate was concentrated under reduced pressure. The residues were purified by silica gel column chromatography (mobile phase: ethyl acetate/petroleum ether = 10% to 40%) to obtain the title compound (2.50 g, 60.2 %, a mixture of two optical isomers) as a pale yellow solid.

$^1$H-NMR (d$^6$-DMSO) $\delta$: 7.46 (dd, *J* = 1.8, 7.5 Hz, 1 H), 7.32 (dt, *J* = 1.85, 8.1 Hz, 1 H), 7.06-6.99 (m, 2 H), 5.24 (dd, *J* = 4.8, 8.4 Hz, 1 H), 4.01-3.97 (m, 2 H), 3.79 (s, 3 H), 3.67-3.56 (m, 2 H), 3.46-3.48 (m, 1 H), 3.29-3.21 (m, 2 H), 2.52-2.50 (m, 1 H), 1.71-1.63 (m, 2 H), 1.40-1.18 (m, 2 H).

LC-MS: m/z 518.10 $[M + Na]^+$.

Step 5: Preparation of methyl 2'-bromo-7'-(2-(2-methoxyphenyl)-2-((tetrahydro-2*H*-pyran-4-yl)oxy)ethyl)-3'-methyl-4',6'-dioxo-6',7'-dihydro-4'*H*-spiro[cyclopentane-1,5'-thieno[2,3-b]pyridine]-3-carboxylate (5-6)

Methyl cyclopentane-1,3-dicarboxylate (563 mg, 3.02 mmol) was dissolved in dry THF (20 mL) under nitrogen atmosphere, and then added with LDA (3.02 mmol, 2 mmol/L) at -78°C. The mixture was stirred at this temperature for 1 hour and then added with 6-bromo-1-(2-(2-methoxyphenyl-2-((tetrahydro-2*H*-pyran-4-yl)oxy)ethyl)-5-methyl-2*H*-thieno[2,3-d][1,3]oxazine-2,4(1*H*)-dione (5-5) (500 mg, 1.01 mmol). The reaction solution was warmed to room temperature and stirred overnight. After completion of the reaction, the reaction was quenched by adding water. The reaction system was extracted with ethyl acetate (3×40 mL), and the combined organic phase was washed with saturated brine (50 mL), dried over anhydrous sodium sulfate and filtered. The filtrate was concentrated under reduced pressure. The residues were purified by silica gel column chromatography (mobile phase: ethyl acetate/petroleum ether = 20% to 40%) to obtain the title compound (392 mg, 64.0 %) as a white solid.

LC-MS: m/z 628.15 $[M + Na]^+$.

Step 6: Preparation of methyl 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclopentane-1,5'-thieno[2,3-b]pyridine]-3-carboxylate (5-7)

2-(Tributylstannyl)oxazole (463 mg, 1.29 mmol) and bis(triphenylphosphorus) palladium dichloride (227 mg, 0.323 mmol) were added to methyl 2'-bromo-7'-(2-(2-methoxyphenyl-2-((tetrahydro-2*H*-pyran-4-yl)oxy)ethyl)-3'-methyl-4',6'-dioxo-6',7'-dihydro-4'*H*-spiro[cyclopentane-1,5'-thieno[2,3-b]pyridine]-3-carboxylate (5-6) (392 mg, 0.646 mmol) in a dry toluene solution (10 mL) under nitrogen atmosphere at room temperature. The reaction solution was heated and refluxed overnight. After completion of the reaction, the reaction was quenched by adding water. The reaction system was extracted with ethyl acetate (3×20 mL), and the combined organic phase was washed with saturated brine (30 mL), dried over anhydrous sodium sulfate and filtered. The filtrate was concentrated under reduced pressure. The residues were separated and purified by silica gel column chromatography (mobile phase: ethyl acetate/petroleum ether = 20% to 40%) to obtain the title compound (162 mg, 42.0 %) as a pale yellow oil.

LC-MS: m/z 617.35 $[M + Na]^+$.

Step 7: Preparation of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclopentane-1,5'-thieno[2,3-b]pyridine]-3-carboxylic acid (5)

Methyl 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[cyclopentane-1,5'-thieno[2,3-b]pyridine]-3-carboxylate (5-7) (160 mg, 0.277 mmol) was dissolved in 6N hydrochloric acid (5 mL) at room temperature. The reaction solution was stirred at room temperature overnight. After completion of the reaction, the reaction solution was concentrated under reduced pressure to obtain a crude product as colorless oil which was purified by silica gel column chromatography (mobile phase: ethyl acetate/petroleum ether = 20% to 60%) to obtain the title compound (60.1 mg, 37.1 %, a mixture of eight optical isomers) as a white solid.

$^1$H-NMR ($CD_3OD$) $\delta$: 7.73 (d, *J* = 0.9 Hz, 1 H), 7.57 (t, *J* = 4.5 Hz, 1 H), 7.29-7.34 (m, 2H), 7.04 (t, *J* = 6 Hz, 1 H), 6.89 (t, *J* = 6.3 Hz, 1 H), 5.38-5.45 (m, 1 H), 4.17-4.27 (m, 1 H), 3.98-4.10 (m, 1 H), 3.81 (s, 3 H), 3.77-3.84 (m, 2 H), 3.43-3.47 (m, 1 H), 3.23-3.36 (m, 3 H), 2.84 (s, 3 H), 2.53-2.72 (m, 2 H), 2.13-2.48 (m, 5 H), 1.73-1.94 (m, 2 H), 1.53-1.59 (m, 1 H), 1.44-1.48 (m, 1 H).

LC-MS: m/z 581.15 [M + H]$^+$.

Step 8: Seperation of optical isomers of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclopentane-1,5'-thieno[2,3-b]pyridine]-3-carboxylic acid (5)

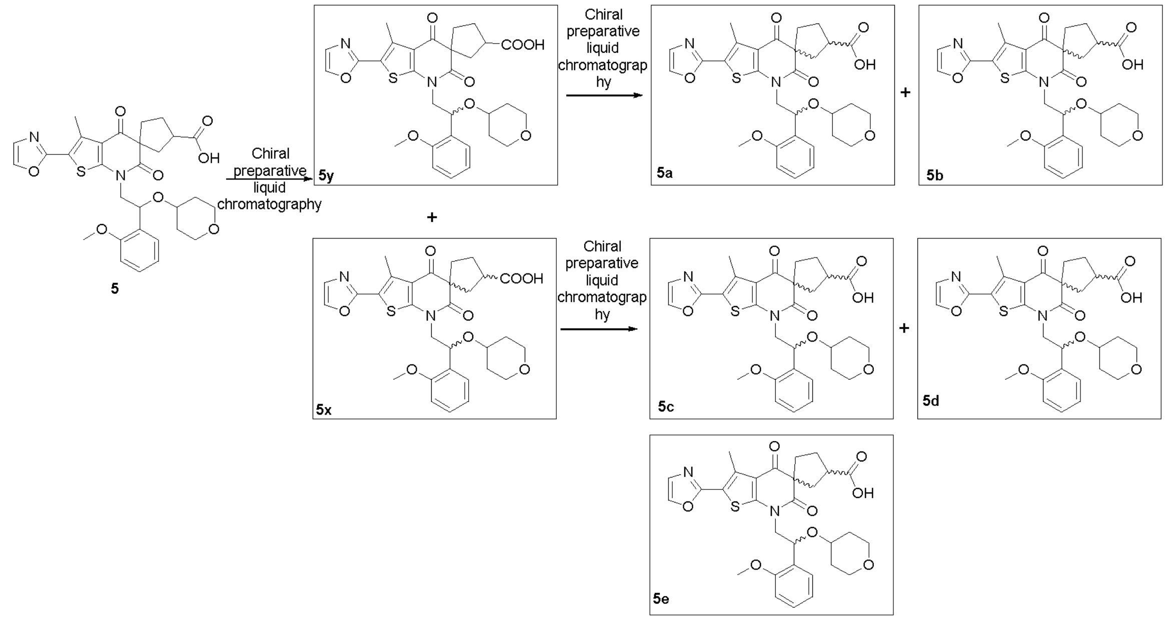


5 5y 5x 5a 5b 5c 5d 5e

7'-(2-(2-Methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[cyclopentane-1,5'-thieno[2,3-b]pyridine]-3-carboxylic acid (compound 5) (465 mg) was separated by preparative chiral column chromatography (column type: XSelect CSH Prep C18 OBD column, 5 $\mu$m, 19×150 mm; mobile phase A: water (0.1% formic acid), mobile phase B: acetonitrile; flow rate: 25 mL/min; gradient: 68% to 73% acetonitrile; duration: 15 minutes; detection wavelength: 254/220 nm), to obtain compound 5x (119 mg, a mixture of 6 optical isomers) as a white solid and compound 5y (100 mg, a mixture of 2 optical isomers) as a white solid.

Compound 5x: $^1$H-NMR ($CD_3OD$) $\delta$ : 7.99 (s, 1 H), 7.56-7.53 (m, 1 H), 7.32-7.28 (m, 2 H), 7.05-6.96 (m, 2 H), 5.49-5.42 (m, 2 H), 4.16-4.13 (m, 2 H), 3.87 (s, 3 H) 3.86-3.75 (m, 2 H), 3.50-3.47 (m, 1 H), 3.39-3.36 (m, 2 H), 3.15-3.09 (m, 1 H), 2.78 (s, 3 H), 2.56-2.53 (m, 1 H), 2.16-2.09 (m, 3 H), 1.86-1.80 (m, 1 H), 1.76-1.71 (m, 1 H), 1.56-1.44 (m, 2 H).

LC-MS: m/z 581.15 [M + H]$^+$.

Compound 5y: $^1$H-NMR ($CD_3OD$) $\delta$: 7.99 (s, 1 H), 7.55 (dd, *J* = 1.2, 5.4 Hz, 1 H), 7.33-7.29 (m, 2 H), 7.03 (t, *J* = 5.7 Hz, 1 H), 6.98 (d, *J* = 6.3 Hz, 1 H), 5.46-5.43 (m, 1 H), 4.18-4.09 (m, 1 H), 3.89 (s, 3 H), 3.80-3.73 (m, 2 H), 3.50-3.46 (m, 1 H), 3.38-3.36 (m, 2 H), 3.11-3.09 (m, 1 H), 2.79 (s, 3 H), 2.44-2.42 (m, 2 H), 2.30-2.23 (m, 2 H), 2.16-2.12 (m, 2 H), 1.83-1.81 (m, 1 H), 1.76-1.72 (m, 1 H), 1.54-1.44 (m, 2 H).

LC-MS: m/z 581.15 [M + H]$^+$.

Step 9: Separation of optical isomers of compound 5y

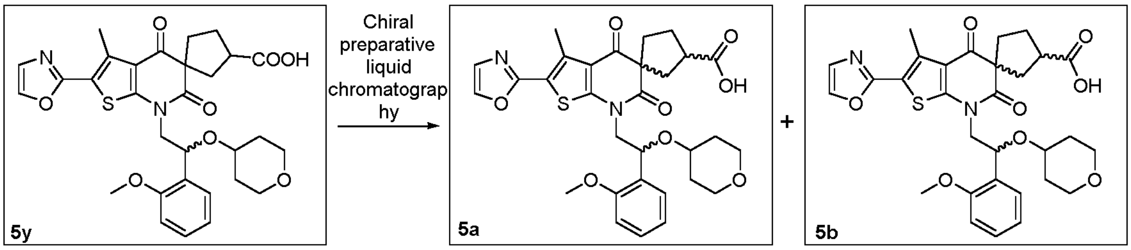


5y 5a 5b

Compound 5y (100 mg) was separated by preparative chiral column chromatograph (column type: CHIRALPAK IF-3, 2.0×25 cm L (5 μm); mobile phase A: tert-butyl methyl ether (0.1% formic acid), mobile phase B: ethanol; flow rate: 20 mL/min; gradient: 10% isogradient; duration: 40 minutes; detection wavelength: 220/320 nm), to obtain compound 5a (22.1 mg, single configuration) as a white solid and compound 5b (18.3 mg, single configuration) as a white solid, respectively.

The $^1$H NMR and LC/MS data of compounds 5a and 5b were completely consistent with those of compound 5y.

Chiral analysis: Column type: CHIRALPAK IF-3, 10×0.46 cm L (3 μm); mobile phase A: tert-butyl methyl ether (0.1% formic acid), mobile phase B: ethanol; flow rate: 1 mL/min; gradient: 10% isogradient; detection wavelength 220/320 nm, retention time: 5a: 1.35 min; 5b: 1.55 min.

Step 10: Separation of optical isomers of compound 5x

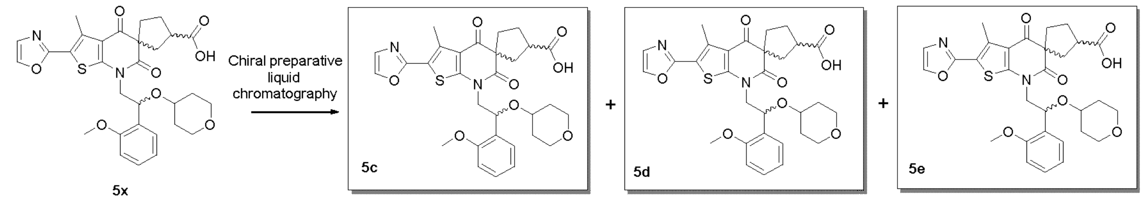


5x 5c 5d 5e

Compound 5x (119 mg) was separated by preparative chiral column chromatography (column type: CHIRALPAK ID-3, 2.0×25cm L (5 μm); mobile phase A: n-hexane (0.1% formic acid), mobile phase B: ethanol; flow rate: 20 mL/min; gradient: 15% isogradient; duration: 40 min; detection wavelength: 220/320 nm), to obtain compound 5c (9.1 mg, single configuration) as a white solid, compound 5d (4.6 mg, single configuration) as a white solid, and compound 5e (20 mg, a mixture of two optical isomers) as a white solid, respectively.

Compound 5c: $^1$H-NMR ($CD_3OD$) $\delta$: 7.97 (s, 1 H), 7.54 (dd, *J* = 1.5, 9 Hz, 1 H), 7.32-7.27 (m, 2 H), 7.05-6.91 (m, 2 H), 5.44-5.40 (m, 1 H), 4.12-4.09 (m, 2 H), 3.86 (s, 3 H), 3.80-3.72 (m, 2 H), 3.50-3.44 (m, 1 H), 3.39-3.36 (m, 1 H), 3.10-3.07 (m, 1 H), 2.76 (s, 3 H), 2.53-2.50 (m, 2 H), 2.20-2.10 (m, 4 H), 1.85-1.70 (m, 2 H), 1.56-1.42 (m, 2 H), 1.35-1.30 (m, 2 H).

LC-MS: m/z 581.15 $[M + H]^+$.

Compound 5d: $^1$H-NMR ($CD_3OD$) $\delta$: 7.98 (s, 1 H), 7.54 (dd, *J* = 1.5, 7.5 Hz, 1 H), 7.33-7.27 (m, 2 H), 7.05-6.96 (m, 2 H), 5.45-5.41 (m, 1 H), 4.15-4.12 (m, 2 H), 3.86 (s, 3 H), 3.81-3.73 (m, 2 H), 3.51-3.45 (m, 1 H), 3.40-3.37 (m, 1 H), 3.13-3.08 (m, 1 H), 2.77 (s, 3 H), 2.60-2.53 (m, 1 H), 2.45-2.38 (m, 1 H), 2.25-2.04 (m, 4 H), 1.86-1.72 (m, 2 H), 1.57-1.45 (m, 2 H), 1.35-1.33 (m, 1 H).

LC-MS: m/z 581.15 $[M + H]^+$.

Compound 5e: $^1$H-NMR ($CD_3OD$) $\delta$: 7.97 (s, 1 H), 7.54-7.52 (m, 1 H), 7.32-7.27 (m, 2 H), 7.05-6.95 (m, 2 H), 5.44-5.40 (m, 1 H), 4.13-4.10 (m, 1 H), 3.87 (s, 3 H), 3.80-3.72 (m, 2 H), 3.48-3.45 (m, 1 H), 3.10-3.02 (m, 1 H), 2.76 (s, 3 H), 2.55-2.50 (m, 2 H), 2.23-2.03 (m, 4 H), 1.81-1.71 (m, 2 H), 1.56-1.40 (m, 2 H), 1.32-1.30 (m, 1 H).

LC-MS: m/z 581.15 $[M + H]^+$.

Chiral analysis: Column type: CHIRALPAK ID-3, 10×0.46cm L (3 μm); mobile phase A: n-hexane (0.1% formic acid), mobile phase B: ethanol; flow rate: 1.5 mL/min; gradient: 15% isogradient; detection wavelength: 220/320 nm, retention time: 5c: 9.1 min; 5d: 10.2 min; 5e: 12.5 min.

Example 6: Preparation of 3'-methyl-2'-(oxazol-2-yl)-4',6'-dioxo-7'-(2-phenyl-2-((tetrahydro-2*H*-pyran-4-yl)oxy)ethyl)-6',7'-dihydro-4'*H*-spiro[cyclohexane-1,5'-thieno[2,3-b]pyridine]-4-carboxylic acid (6)

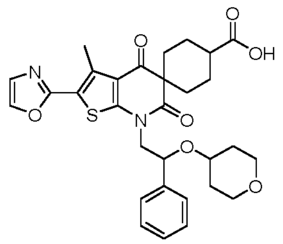

6

The preparation method was the same as in Example 3, except that methyl cyclohexane-1,4-dicarboxylate was used instead of methyl cyclopentane-1,3-dicarboxylate, to obtain the title compound 6 (a mixture of four optical isomers).

$^1$H-NMR ($CDCl_3$) 9:7.73 (1 H, d, *J* = 0.9 Hz), 7.49 (1 H, dd, *J* = 1.5, 7.5 Hz), 7.37-7.46 (3 H, m), 7.25 (1 H, d, *J* = 0.9 Hz), 4.93 (1 H, dd, *J* = 6, 9.6 Hz), 4.28 (1 H, dd, *J* = 3.6, 14.4 Hz), 3.73-3.84 (3 H, m), 3.42-3.48 (1 H, m), 3.28-3.70 (2 H, m), 2.82 (3 H, s), 2.45-2.49 (1 H, m), 2.06-2.25 (5 H, m), 2.01 (3 H, m), 1.70-1.82 (2 H, m), 1.50-1.62 (1 H, m), 1.36-1.47 (1 H, m).

LC-MS: m/z 565.40 $[M + H]^+$.

Example 7: Preparation of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclohexane-1,5'-thieno[2,3-b]pyridine]-3-carboxylic acid (7)

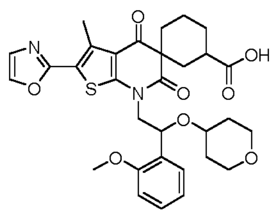

7

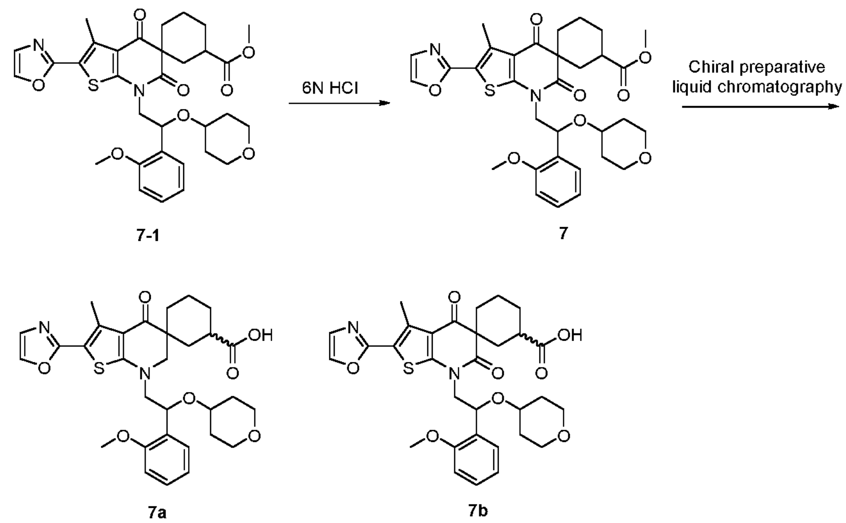


7-1

7

7a

7b

Step 1: Preparation of compound 7-1

The preparation method was the same as for compound 5-7 in Example 5, except that methyl cyclohexane-1,3-dicarboxylate was used instead of methyl cyclopentane-1,3-dicarboxylate to obtain the title compound 7-1.

Step 2: Preparation of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclohexane-1,5'-thieno[2,3-b]pyridine]-3-carboxylic acid (7)

Methyl 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[cyclohexane-1,5'-thieno[2,3-b]pyridine]-3-carboxylate (50 mg, 0.086 mmol) was dissolved in 6N hydrochloric acid (3 mL). The reaction solution was stirred at room temperature overnight. After completion of the reaction, the reaction solution was concentrated under reduced pressure to obtain the title compound as a colorless oil.

Step 3: Seperation of optical isomers of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclohexane-1,5'-thieno[2,3-b]pyridine]-3-carboxylic acid (7)

7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[cyclohexane-1,5'-thieno[2,3-b]pyridine]-3-carboxylic acid was separated by preparative column chromatography (column type: XSelect CSH Prep C18 OBD column, 5 μm, 19×150 mm; mobile phase A: water (10 mmol/L ammonium bicarbonate), mobile phase B: acetonitrile; flow rate: 25 mL/min; gradient: 25% to 75% acetonitrile; duration: 8 min; detection wavelength: 254 nm), to obtain compound 7a (5.6 mg, 11.2%, a mixture of optical isomers) as a white solid and compound 7b (2.5 mg, 5.0%, a mixture of optical isomers) as a white solid, respectively.

Compound 7a: $^1$H-NMR ($CDCl_3$) $\delta$: 7.73 (1 H, s), 7.48-7.50 (2 H, m), 7.37-7.44 (3 H, m), 7.25 (1 H, s), 3.42-3.47 (1 H, m), 3.31-3.39 (3 H, m), 2.81 (3H, s), 2.31 (1 H, t, 9.6 Hz), 2.10-2.17 (2 H, m), 1.91-1.95 (1 H, m), 1.70-1.82 (5 H, m), 1.53-1.61 (1 H, m), 1.42-1.45 (2 H, m).

LC-MS: m/z 565.25 $[M + H]^+$.

Compound 7b: $^1$H-NMR ($CDCl_3$) $\delta$: 7.73 (1 H, s), 7.48-7.50 (2 H, m), 7.37-7.44 (3 H, m), 7.24 (1 H, s), 4.95-4.97 (1 H, m), 4.12-4.16 (1 H, s), 3.90-3.96 (1 H, m), 3.73-3.80 (2 H, m), 3.41-3.46 (1 H, m), 3.27-3.32 (2 H, m), 3.13-3.19 (1 H, m), 2.82 (3 H, s), 2.10-2.23 (4 H, m), 1.91-1.97 (4 H, m), 1.76-1.84 (3 H, m), 1.67-1.73 (2 H, m), 1.38-1.57 (4 H, m), 1.28-1.30 (1 H, m), 0.88-0.91 (1 H, m).

LC-MS: m/z 565.25 $[M + H]^+$.

Example 8: Preparation of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclohexane-1,5'-thieno[2,3-b]pyridine]-3-carboxylic acid (8)

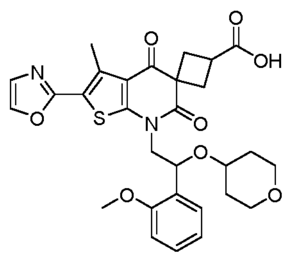

**8**

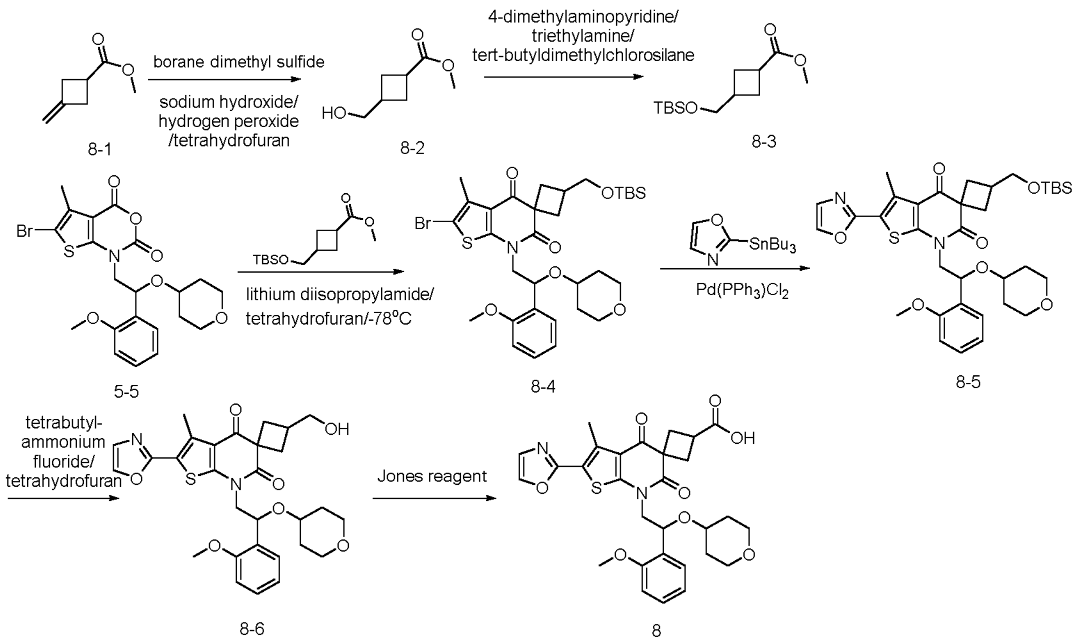


8-1 8-2 8-3 5-5 8-4 8-5 8-6 8

Step 1: Preparation of methyl 3-hydroxymethylcyclobutane-carboxylate (8-2)

Methyl 3-methylenecyclobutane-1-carboxylate (8-1) (10.0 g, 78.1 mmol) was dissolved in dry tetrahydrofuran (100 mL) under a nitrogen atmosphere, and borane dimethyl sulfide (32.0 mL, 64.0 mmol) was slowly added dropwise at -10°C. The reaction solution was warmed to room temperature and stirred for 3 hours to obtain a colorless transparent liquid. Anhydrous methanol (5 mL) was added to the reaction solution at - 10°C, and the reaction solution was warmed to room temperature and stirred for 30 minutes. Sodium hydroxide solution (15.0 mL, 2 mol/L) and hydrogen peroxide (8.96 g, 78.1 mol, 30%) were slowly added dropwise at -10°C, stirred at room temperature for 2 hours, and the reaction solution was evaporated under reduced pressure to dryness to obtain the title compound (5.60 g, 49.0%) as a colorless oil.
LC-MS: m/z 145.15 $[M + H]^+$.

Step 2: Preparation of methyl 3-tert-butyldimethylsiloxymethylcyclobutane-carboxylate (8-3)

Methyl 3-hydroxymethylcyclobutane-carboxylate (8-2) (5.60 g, 38.7 mmol) was dissolved in dichloromethane (50 mL) at room temperature. The reaction solution was cooled to -10°C and then slowly added with tert-butyl dimethylchlorosilane (7.00 g, 46.7 mmol), triethylamine (5.89 g, 58.3 mmol) and 4-dimethylaminopyridine (0.470 g, 3.87 mmol) . The reaction solution was warmed to room temperature and stirred for 12 hours, and then quenched by adding water (100 mL). The reaction system was extracted with ethyl acetate (3 × 150 mL), and the combined organic phase was washed with saturated brine (100 mL), dried over anhydrous sodium sulfate and filtered. The filtrate was concentrated under reduced pressure to obtain the title compound (5.00g, 50.0%) as a colorless oil. The obtained product was directly used in the next step without purification.
LC-MS: m/z 259.20 $[M + H]^+$.

Step 3: Preparation of 2'-bromo-3-(((tert-butyldimethylsilyl)methyl)-7'-(2-(2-methoxyphenyl)-2-((tetrahydro-2*H*-pyran-4-yl)oxy)ethyl)-3'-methyl-4'*H*-spiro[cyclobutane-1,5'-thieno[2,3-b]pyridine]-4',6'(7'*H*)-dione (8-4)

Methyl 3-tert-butyldimethylsiloxymethylcyclobutane-carboxylate (2.30 g, 9.00 mmol) was dissolved in dry THF (5 mL) under nitrogen atmosphere, and then added with LDA (9.00 mmol, 2 mol/L) at -78°C. The mixture was stirred at this temperature for 1 hour and then added with 6-bromo-1-(2-(2-methoxyphenyl-2-((tetrahydro-2*H*-pyran-4-yl)oxy)ethyl)-5-methyl-2*H*-thieno[2,3-d][1,3]oxazine-2,4(*1*H)-dione (5-5) (3.00 g, 6.00 mmol). The reaction solution was warmed to room temperature and stirred for 2 hours. After completion of the reaction, the reaction was quenched by adding water. The reaction system was extracted with ethyl acetate (3 × 50 mL), and the combined organic phase was washed with saturated brine (50 mL), dried over anhydrous sodium sulfate and filtered. The filtrate was concentrated under reduced pressure. The residues were purified by silica gel column chromatography (mobile phase: ethyl acetate/petroleum ether = 30% to 70%) to obtain the title compound (1.40 g, 34.0 %) as a yellow oil.
LC-MS: m/z 702.00 $[M + H]^+$.

Step 4: Preparation of 3-(((tert-butyldimethylsilyl)methyl)-7'-(2-(2-methoxyphenyl)-2-((tetrahydro-2*H*-pyran-4-yl)oxy)ethyl)-3'-methyl-2'-(oxazol-2-yl)-4'*H*-spiro[cyclobutane-1,5'-thieno[2,3-b] pyridine]-4',6'(7'*H*)-dione(8-5)

2-(Tributylstannyl)oxazole (0.810 g, 2.30 mmol) and bis(triphenylphosphorus) palladium dichloride (0.730 g, 1.00 mmol) were added to 2'-bromo-3-(((tertbutyldimethylsilyl)methyl)-7'-(2-(2-methoxyphenyl)-2-((tetrahydro-2*H*-pyran-4-yl)oxy)ethyl)-3'-methyl-4'*H*-spiro[cyclobutane-1,5'-thieno[2,3-b]pyridine]-4',6'(7'*H*)-dione (8-4) (1.40 g, 2.10 mmol) in a dry toluene solution (10 mL) under nitrogen atmosphere at room temperature. The reaction solution was heated and refluxed overnight. After completion of the reaction, the reaction was quenched by adding water. The reaction system was extracted with ethyl acetate (3×30 mL), and the combined organic phase was washed with saturated brine (50 mL), dried over anhydrous sodium sulfate and filtered. The filtrate was concentrated under reduced pressure. The residues were purified by silica gel column chromatography (mobile phase: ethyl acetate/petroleum ether = 40% to 70%) to obtain the title compound (0.800 g, 58.0 %) as a pale yellow oil.

LC-MS: m/z 689.30 $[M + H]^+$.

Step 5: Preparation of 3-(hydroxymethyl)-7'-(2-(2-methoxyphenyl) -2-((tetrahydro-2*H*-pyran-4-yl)oxy)ethyl)-3'-methyl-2'-(oxazol-2-yl)-4'*H*-spiro[cyclobutane-1,5'-thieno[2,3-b]pyridine]-4',6'(7'*H*)-dione(8-6)

3-(((Tert-butyldimethylsilyl)methyl)-7'-(2-(2-methoxyphenyl)-2-((tetrahydro-2*H*-pyran-4-yl)oxy)ethyl)-3'methyl-2'-(oxazol-2-yl)-4'*H*-spiro[cyclobutane-1,5'-thieno[2,3-b]pyridine]-4',6'(7'*H*)-dione (8-5) (0.800 g, 1.20 mmol) was dissolved in dry tetrahydrofuran (5 mL) at room temperature, and then added with tetrabutylamine fluoride (0.370 g, 1.40 mmol). The mixture was stirred at room temperature for 12 hours, and then quenched by adding water (50 mL). The reaction mixture was extracted with ethyl acetate (3×50 mL). The organic phases were combined and then washed with saturated brine (40 mL), dried over anhydrous sodium sulfate and filtered. The filtrate was concentrated under reduced pressure. The residues were purified by silica gel column chromatography (mobile phase: ethyl acetate/petroleum ether = 50% to 90%) to obtain the title compound (0.500 g, 75.0 %) as a colorless oil.

LC-MS: m/z 553.20 $[M + H]^+$.

Step 6: Preparation of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxylic acid (8)

Jones reagent (0.43 mL, 2 mol/L) was slowly added dropwise to a solution of 3-(hydroxymethyl)-7' -(2-(2-methoxyphenyl)-2-((tetrahydro-2*H*-pyran-4-yl)oxy)ethyl)-3'-methyl-2'-(oxazol-2-yl)-4'*H*-spiro[cyclobutane-1,5'-thieno[2,3-b]pyridine]-4',6'(7'*H*)-dione (8-6) (220 mg, 0.400 mmol) in acetone (5 mL) at 0°C, and stirred at this temperature for 1.5 hours. The reaction was quenched with isopropanol (5 mL). The reaction solution was concentrated under reduced pressure, and added with water (20 mL). The mixture was extracted with ethyl acetate (3×20 mL). The organic phases were combined and then washed with saturated brine (20 mL), dried over anhydrous sodium sulfate and filtered. The filtrate was concentrated under reduced pressure. The residues were purified by high performance liquid chromatography (column type: XBridge Shiels RP18 OBD column, 5 μm, 19×150 nm; mobile phase A: water (0.1% formic acid), mobile phase B: acetonitrile; flow rate: 25 mL/min; gradient: 45% to 65% in 8 min; detection wavelength: 254/220 nm) to obtain the title compound (82 mg, 36.3%, a mixture of four optical isomers) as a white powder.

LC-MS: m/z 567.15 $[M + H]^+$.

Step 7: Seperation of optical isomers of 7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxylic acid (8)

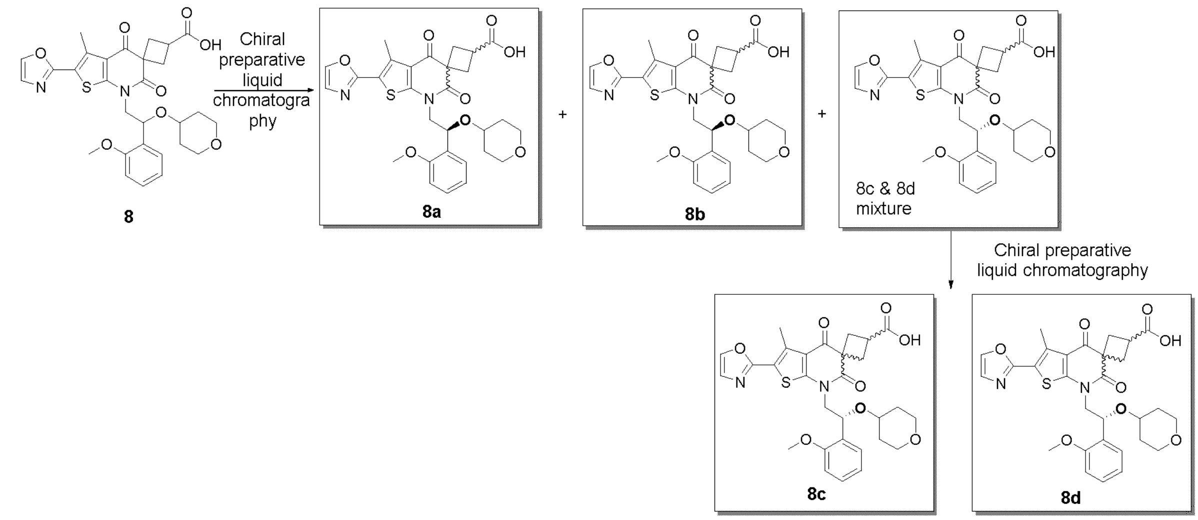


8 8a 8b 8c 8d

7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxylic acid (compound 8) (82 mg) was separated by preparative column chromatography (column type: CHIRALPAK IC, 2×25 cm, 5 μm; mobile phase A: n-hexane (0.1% formic acid), mobile phase B: ethanol; flow rate: 18 mL/min; gradient: 18%; duration: 40 min; detection wavelength: 210 nm), to obtain compound 8a (13.8 mg, single configuration) and 8b (5.8 mg, single configuration) and a mixture of 8c and 8d (30 mg, a mixture of two optical isomers) as white solid, respectively.

Compound 8a: $^1$H-NMR ($CD_3OD$) $\delta$: 7.99 (s, 1 H), 7.56-7.53 (m, 1 H), 7.33-7.28 (s, 2 H), 7.06-6.97 (m, 2 H), 5.43 (t, *J* = 6.9 Hz, 1 H), 4.13 (d, *J* = 6.9 Hz, 2 H), 3.87 (s, 3 H), 3.81-3.69 (m, 2 H), 3.50-3.44 (m, 1 H), 3.40-3.34 (m, 3 H), 2.95-2.88 (m, 1 H), 2.84 (s, 3 H), 2.81-2.62 (m, 3 H), 1.84-1.70 (m, 2 H), 1.57-1.40 (m, 2 H).

LC-MS: m/z 567.15 $[M + H]^+$.

Compound 8b: $^1$H-NMR ($CD_3OD$) $\delta$: 7.99 (s, 1 H), 7.56-7.53 (m, 1 H), 7.34-7.28 (s, 2 H), 7.07-6.97 (m, 2 H), 5.47 (t, *J* = 6.9 Hz, 1 H), 4.22-4.12 (m, 2 H), 3.87 (s, 3 H), 3.81-3.69 (m, 2 H), 3.54-3.44 (m, 1 H), 3.40-3.34 (m, 3 H), 2.99-2.83 (m, 2 H), 2.80 (s, 3 H), 2.81-2.62 (m, 2 H), 1.86-1.72 (m, 2 H), 1.57-1.42 (m, 2 H).

LC-MS: m/z 567.15 $[M + H]^+$.

The mixture of compound 8c and 8d (30 mg) was separated by preparative column chromatography (column type: CHIRALPAK IE, 2×25 cm, 5 μm; mobile phase A: n-hexane (0.1% formic acid), mobile phase B: ethanol; flow rate: 20 mL/min; gradient: 20%; duration: 15 min; detection wavelength: 210 nm), to obtain compound 8c (10.2 mg, retention time: 8.55 min, single configuration) and compound 8d (8.3 mg, retention time: 10.10 min, single configuration) as a white solid, respectively.

The NMR and LC/MS data of compound 8c were completely consistent with those of compound 8a.

The NMR and LC/MS data of compound 8d were completely consistent with those of compound 8b.

Chiral analysis: Column type: CHIRALPAK IC, 2×25 cm, 5 μm; mobile phase A: n-hexane (0.1% formic acid), mobile phase B: ethanol; flow rate: 1 mL/min; gradient: 30%; detection wavelength: 210 nm, retention time: 8a: 5.83 min; 8b: 7.38 min; 8c: 10.09 min; 8d: 10.09 min.

Step 8: Confirmation of the configuration of compound 8c

About 10 mg of compound 8c isolated in Step 7 was dissolved in 1 mL of MeOH solvent, and the solvent was naturally evaporated at room temperature to obtain a rodshaped single crystal. The single crystal was analyzed by X-ray diffraction. The X-ray diffraction pattern (XRD) is shown in Figure 1. The simulated and test XRD patterns of the single crystal were overlaped and compared in Figure 1. Between the simulated and test XRD patterns, the main peaks are well matched.

X-ray diffraction analysis parameters are as follows:

Instrument model: Rigaku XtaLAB PRO 007HF (Mo)
Ray: Mo Kα radiation (λ = 0.71073 Å)
Temperature: 180 K
Scanning method: 2θ/θ
Scanning range: 3.000°~40.006°

The crystal structure data is summarized in Table 1 below.

Table 1 Crystal data and structure data of compound 8c

| | |
|---|---|
| Empirical formula | $C_{29}H_{30}N_2O_8S$ |
| Molecular weight | 566.61 |
| Temperature/K | 180.00(10) |
| Crystal system | Orthogonal |
| Space group | $P2_12_12_1$ |
| $a$/Å | 5.83670(10) |
| $b$/Å | 17.8465(3) |
| $c$/Å | 25.7535(4) |
| $\alpha$/° | 90 |
| $\beta$/° | 90 |
| $\gamma$/° | 90 |
| volume/Å$^3$ | 2682.60(8) |
| Z | 4 |
| $\rho_{calc}$g/cm$^3$ | 1.403 |
| $\mu$/mm$^{-1}$ | 0.176 |
| $F$(000) | 1192.0 |
| Crystal size/mm$^3$ | 0.51 × 0.07 × 0.03 |
| Radiation | Mo K$\alpha$ ($\lambda$ = 0.71073 Å) |
| Data collection range of 2$\theta$/° | 4.564 to 54.97 |
| Index range | $-7 \le h \le 7$, $-23 \le k \le 23$, $-33 \le l \le 33$ |
| Diffraction points collected | 62490 |
| Independent diffraction spot | 6172 [$R_{int}$ = 0.0397, $R_{sigma}$ = 0.0181] |
| Data/limits/parameters | 6172/1/366 |
| Goodness of fit on $F^2$ | 1.061 |
| Final R index [I≥ 2$\sigma$ (I)] | $R_1$ = 0.0323, $wR_2$ = 0.0853 |
| Final R index [all data] | $R_1$ = 0.0338, $wR_2$ = 0.0862 |
| Maximum difference peak/hole/e Å$^{-3}$ | 0.29 /-0.15 |
| Flack parameter | 0.014 (14) |

The CrysAlisPro program was used for data reduction and empirical absorption correction. The SHELXT program was used to analyze the structure through a dual-space algorithm. All non-hydrogen atoms can be located directly from the differential Fourier diagram. The skeleton hydrogen atoms were geometrically placed and a riding model was used to confine them to the parent atoms. The SHELXL program was used to complete the refinement of the final structure by using the full matrix technique to minimize the sum of the square deviations of $F^2$.

The fine single crystal structure is shown in Figure 2 and Figure 3. The chemical formula unit of the single crystal is $C_{32}H_{42}N_2O_4S$, which crystallizes in an orthogonal form in the $P2_12_12_1$ space group. Each asymmetric unit has one molecule, which shows a *cis* conformation, and the chiral carbon is shown as "S". Each unit cystal cell has four molecules. The hydrogen bond of O5-H5...O4 was found through symmetrical operation.

From the above analysis, it can be determined that the configurational formula of compound **8c** is as follows:

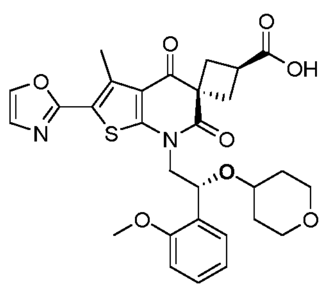

**8c**

Example 9: Preparation of (1s,3S)-7'-((R)-2-(2-methoxyphenyl)-2-((tetrahydro-2*H*-pyran-4-yl)oxy)ethyl)-N,3'-dimethyl-2'-(oxazol-2-yl)-4',6'-dioxo-6',7'-dihydro-4'*H*-spiro[cyclohexane-1,5'-thieno[2,3-b]pyridine]-3-carboxyamide (9)

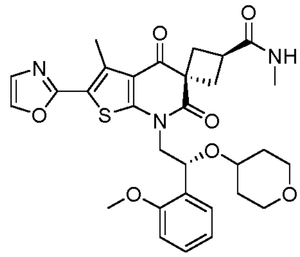

**9**

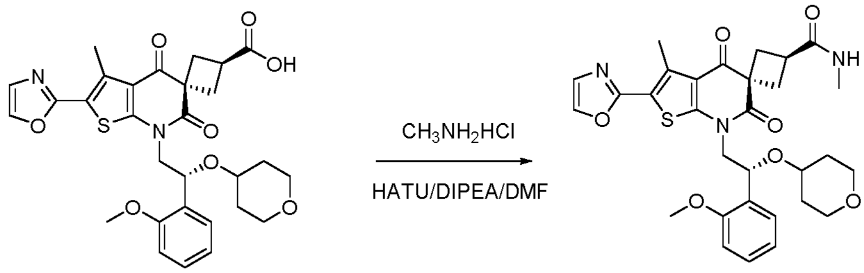


**8c** **9**

Compound **8c** (30 mg, 0.035 mmol) was dissolved in DMF (2 mL) at room temperature. Methylamine hydrochloride (7.2 mg, 0.107 mmol), HATU (20.0 mg, 0.053 mmol) and DIPEA (23.1 mg, 0.178 mmol) were added to the reaction solution. The mixture was stirred at room temperature overnight, and quenched by adding water (5 mL). The reaction system was extracted with ethyl acetate (3 × 10 mL). The organic phases were combined and then washed with saturated brine (10 mL), dried over anhydrous sodium sulfate and filtered. The organic phase was concentrated under reduced pressure. The residues were purified by silica gel column chromatography (mobile phase: petroleum ether/ethyl acetate = 40% to 70%) to obtain the title compound (7 mg, 32.7 %) as a white solid.

$^1$H-NMR ($CD_3OD$) δ: 7.98 (d, *J* = 0.9 Hz, 1H), 7.54 (dd, *J* = 1.8, 7.8 Hz, 1H); 7.34-7.28 (m, 2H), 7.06-8.97 (m, 2H), 5.44-5.39 (m, 1H), 4.12-4.09 (m, 2H), 3.87 (s, 3H), 3.80-3.67 (m, 2H), 3.51-3.42 (m, 1H), 3.40-3.37 (m, 3H), 2.96-2.90 (m, 1H), 2.82 (s, 3H), 2.77 (s, 3H), 2.71-2.54 (m, 2H), 1.82-1.71 (m, 2H), 1.57-1.37 (m, 2H).

LC-MS: m/z 580.20 $[M + H]^+$.

Example 10: Preparation of ((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclohexane-1,5'-thieno[2,3-b]pyridine]-3-carbonyl)glycine(10)

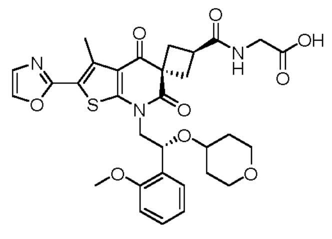

10

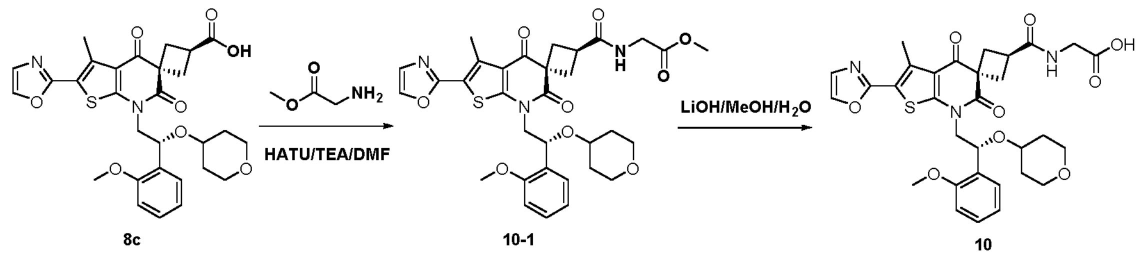


Step 1: Preparation of methyl ((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)glycinate (**10-1**)

Compound **8c** (30 mg, 0.053 mmol) was dissolved in DMF (2 mL) at room temperature. Methyl glycinate hydrochloride (9.4 mg, 0.106 mmol), HATU (30.2 mg, 0.079 mmol) and triethylamine (21.4 mg, 0.212 mmol) were added to the reaction solution. The mixture was stirred at room temperature overnight, and quenched by adding water (5 mL). The reaction system was extracted with ethyl acetate (3 × 10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 40% to 70%) to obtain the title compound (5 mg, 15 %) as a white solid.

$^1$H-NMR ($CD_3OD$) δ: 7.99 (d, *J* = 0.9 Hz, 1H), 7.54 (dd, *J* = 1.8, 7.5 Hz, 1H), 7.34-7.28 (m, 2H), 7.06-6.97 (m, 2H), 5.44-5.40 (m, 1H), 4.13-4.10 (m, 2H), 4.00-3.98 (m, 2H), 3.88 (s, 1H), 3.80-3.67 (m, 5H), 3.51-3.42 (m, 2H), 3.40-3.35 (m, 2H), 3.01-2.94 (m, 1H), 2.89-2.83 (m, 4H), 2.74-2.58 (m, 2H), 1.81-1.72 (m, 2H), 1.57-1.41 (m, 1H).

LC-MS: m/z 638.30 $[M + H]^+$.

Step 2: Preparation of ((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-l,5'-thieno[2,3-b]pyridine]-3-carbonyl)glycine (**10**)

Compound **10-1** (30 mg, 0.047 mmol) was dissolved in methanol/water mixed solution (1/1, 2 mL) at room temperature. Lithium hydroxide (4.0 mg, 0.094 mmol) was added to the reaction solution. The reaction mixture was reacted at room temperature for 2 hours and then added with diluted hydrochloric acid until the pH of the reaction solution reached 2. The organic solvent was removed under reduced pressure, and the system was extracted with ethyl acetate (3×10 mL). The organic phases were combined and then washed with saturated brine (10 mL), dried over anhydrous sodium sulfate and filtered. The organic phase was concentrated under reduced pressure. The residues were purified by silica gel column chromatography (mobile phase: petroleum ether/ethyl acetate = 60% to 70%) to obtain the title compound (11 mg, 61.2 %) as a white solid.

$^1$H-NMR ($CD_3OD$) δ: 7.99 (d, *J* = 0.6 Hz, 1H), 7.54 (dd, *J* = 1.2, 5.7 Hz, 1H), 7.33-7.29 (m, 2H), 7.03 (td, *J*= 0.6, 5.4 Hz, 1H), 6.98 (dd, *J* = 0.6, 6.3 Hz, 1H), 5.44-5.40 (m, 1H), 4.17-4.06 (m, 2H), 3.96 (s, 3H), 3.88 (s, 3H), 3.79-3.67 (m, 2H), 3.50-3.43 (m, 2H), 3.39-3.35 (m, 2H), 3.00-2.96 (m, 1H), 2.88-2.79 (m, 4H), 2.73-2.67 (m, 2H), 2.65-2.59 (m, 2H).

LC-MS: m/z 624.20 $[M + H]^+$.

Example 11: Preparation of 2-((ls,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxyamido)-2-methyl-propionic acid (11)

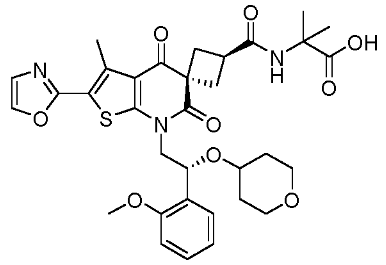

11

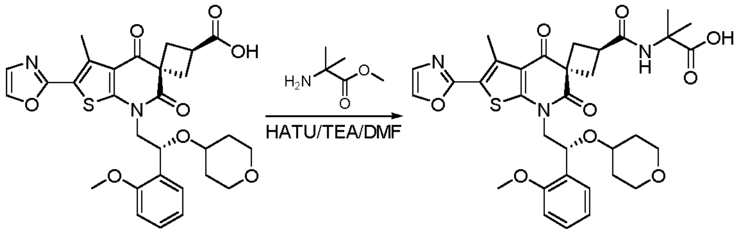


8c 11

Compound **8c** (20 mg, 0.035 mmol) was dissolved in DMF (2 mL) at room temperature. HATU (20.1 mg, 0.053 mmol) and triethylamine (10.7 mg, 0.106 mmol) was added to the reaction solution. The mixture was stirred at room temperature for 1 hour. Methyl 2-aminoisobutyrate hydrochloride (7.3 mg, 0.071 mmol) was added and then stirred at room temperature overnight. After completion of the reaction, the reaction was quenched by adding water (5 mL). The reaction system was extracted with ethyl acetate (3 × 10 mL), and the organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 40% to 70%) to obtain the title compound (3.6 mg, 15.3 %) as a white solid.

$^1$H-NMR ($CD_3OD$) δ: 7.97 (d, *J*= 0.9 Hz, 1H), 7.52 (dd, *J*= 1.5, 7.5 Hz, 1H), 7.33-7.25 (m, 2H), 7.05-6.96 (m, 2H), 5.41-5.37 (m, 1H), 4.14-4.00 (m, 1H), 3.86 (s, 3H), 3.79-3.67 (m, 2H), 3.48-3.43 (m, 1H), 3.37-3.32 (m, 3H), 3.02-2.80 (m, 4H), 2.68-2.54 (m, 2H), 1.80-1.70 (m, 2H), 1.53-1.31 (m, 8H).

LC-MS: m/z 652.40 [M + H]$^+$.

Example 12: Preparation of (1s,3S)-7'-((R)-2-(2-methoxyphenyl)-2-((tetrahydro-2*H*-pyran-4-yl)oxy)ethyl)-N,N,3'-tiimethyl-2'-(oxazol-2-yl)-4',6'-dioxo-6',7'-dihydro-4'*H*-spiro[cyclohexane-1,5'-thieno[2,3-b]pyridine]-3-carboxamide (12)

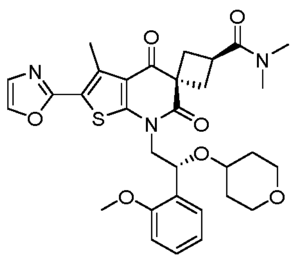

12

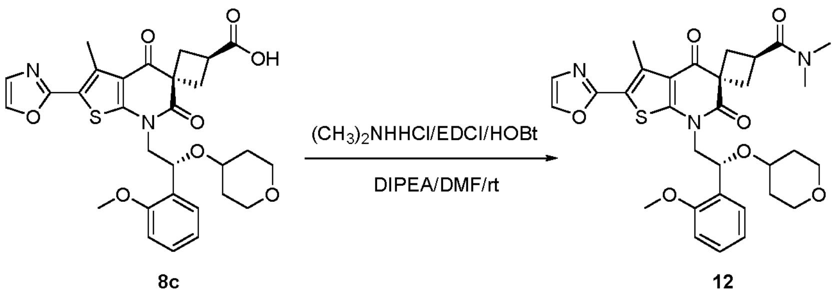


8c 12

Compound **8c** (30 mg, 0.053 mmol) was dissolved in DMF (2 mL) at room temperature. Dimethylamine hydrochloride (14.3 mg, 0.176 mmol), 1-(3-dimethylaminopropyl)-3-ethylcarbodiimide hydrochloride (EDCI) (8.0 mg, 0.042 mmol), 1-hydroxybenzotriazole (HOBt) (6.0 mg, 0.044 mmol) and DIPEA (36.1 mg, 0.279 mmol) were added to the reaction solution. The reaction mixture was stirred at room temperature overnight, and quenched by adding water (5 mL). The reaction system was extracted with ethyl acetate (3 × 10 mL), and the organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 40% to 70%) to obtain the title compound (7.6 mg, 35.1 %) as a white solid.

$^1$H-NMR ($CD_3OD$) $\delta$: 7.99 (d, $J$ = 0.9 Hz, 1H), 7.54 (dd, $J$= 1.8, 7.5 Hz, 1H), 7.30-7.28 (m, 2H), 7.06-6.97 (m, 2H), 5.44-5.40 (m, 1H), 4.16-4.09 (m, 2H), 3.86 (s, 3H), 3.80-3.59 (m, 3H), 3.51-3.44 (m, 1H), 3.40-3.37 (m, 3H), 3.23 (dd, $J$= 1.2, 14.7 Hz, 1H), 3.03 (s, 3H), 2.98 (s, 3H), 2.92-2.88 (m, 1H), 2.84 (s, 3H), 2.06-2.03 (m, 1H), 1.83-1.71 (m, 2H), 1.57-1.43 (m, 2H).

LC-MS: m/z 594.30 [M + H]$^+$.

Example 13: Preparation of ((1R,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclohexane-1,5'-thieno[2,3-b]pyridine]-3-carbonyl)-L-alanine (13)

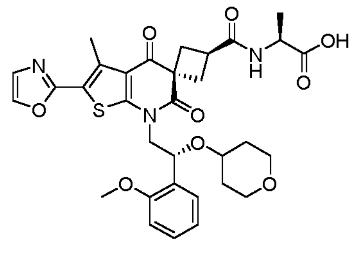

**13**

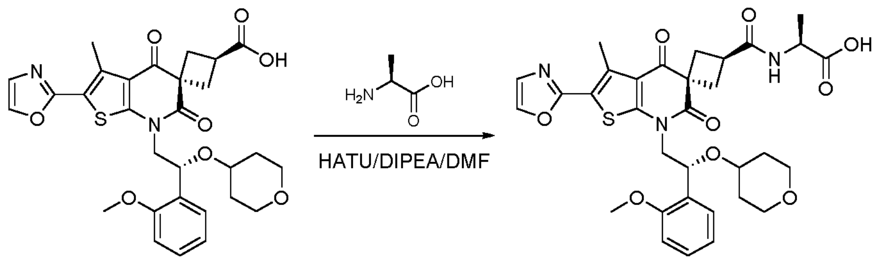


**8c** **13**

Compound **8c** (20 mg, 0.035 mmol) was dissolved in DMF (2 mL) at room temperature. HATU (20.1 mg, 0.053 mmol) and DIPEA (10.7 mg, 0.106 mmol) was added to the reaction solution. The mixture was stirred at room temperature for 1 hour, and then added with L-alanine (7.3 mg, 0.071 mmol) and stirred overnight at room temperature. After completion of the reaction, the reaction was quenched by adding water (5 mL). The reaction system was extracted with ethyl acetate (3×10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 40% to 70%) to obtain the title compound (3.6 mg, 15.3 %) as a white solid.

$^1$H-NMR ($CD_3OD$) $\delta$: 7.99 (s, 1H), 7.54 (dd, $J$ = 1.8, 7.5 Hz, 1H), 7.33-7.28 (m, 2H), 7.06-6.97 (m, 2H), 5.44-5.40 (m, 1H), 4.43 (q, $J$ = 7.5 Hz, 1H), 4.13-4.11 (m, 2H), 3.87 (s, 3H), 3.80-3.68 (m, 2H), 3.50-3.36 (m, 3H), 3.00-2.94 (m, 1H), 2.88-2.79 (m, 4H), 2.73-2.57 (m, 2H), 1.84-1.72 (m 2H), 1.57-1.38 (m, 5H).

LC-MS: m/z 638.35 [M + H]$^+$.

Example 14: Preparation of ((1S,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)-D-alanine (14)

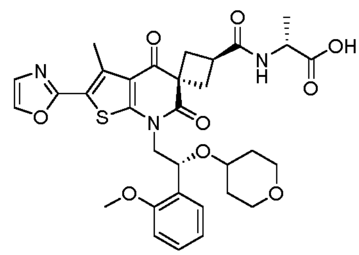

**14**

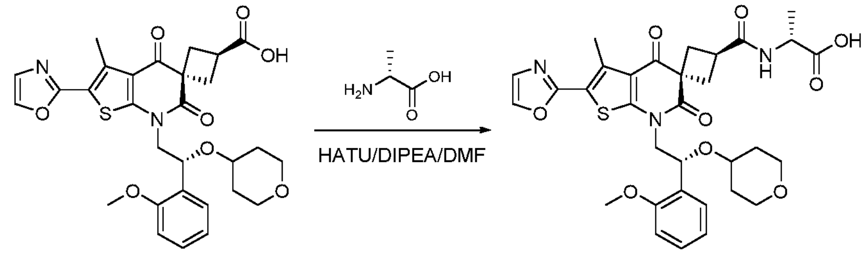


8c 14

Compound 8c (20 mg, 0.035 mmol) was dissolved in DMF (2 mL) at room temperature. HATU (26.8 mg, 0.071 mmol) and DIPEA (18.3 mg, 0.14 mmol) was added to the reaction solution. The mixture was stirred at room temperature for 1 hour, and then added with D-alanine (6.3 mg, 0.071 mmol) and stirred overnight at room temperature. After completion of the reaction, the reaction was quenched by adding water (5 mL). The reaction system was extracted with ethyl acetate (3×10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 40% to 70%) to obtain the title compound (5 mg, 21.7 %) as a white solid.

$^1H$-NMR ($CD_3OD$) δ: 7.98 (s, 1H), 7.53 (dd, *J* = 1.8, 7.5 Hz, 1H), 7.33-7.28 (m, 1H), 7.05-6.96 (m, 2H), 5.41 (dd, *J* = 4.8, 7.2 Hz, 1H), 4.44 (q, *J* = 7.2 Hz, 1H), 4.17-4.02 (m, 2H), 3.87-3.67 (m, 2H), 3.49-3.36 (m, 3H), 2.96 (t, *J* = 9 Hz, 1H), 2.88-2.78 (m, 4H), 2.73-2.56 (m, 2H), 1.82-1.71 (m, 2H), 1.55-1.40 (m, 5H).

LC-MS: m/z 638.35 $[M + H]^+$.

Example 14-1: Preparation of Methyl ((1S,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)-D-alaninate

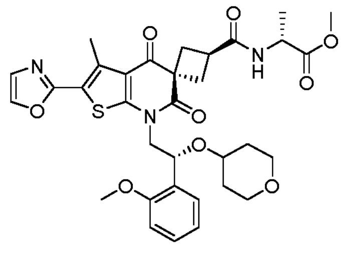

**14-1**

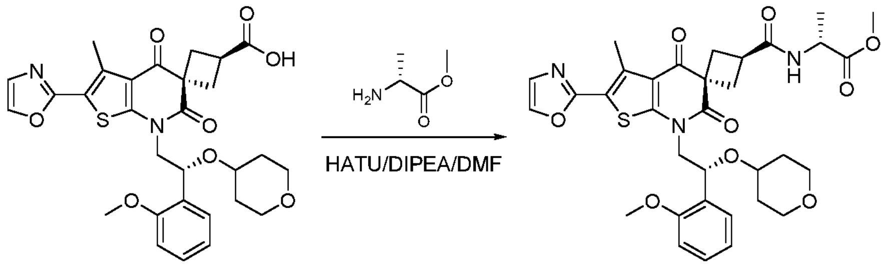


**8c** **14-1**

Compound **8c** (20 mg, 0.035 mmol) was dissolved in DMF (2 mL) at room temperature. HATU (26.8 mg, 0.071 mmol) and DIPEA(18.3 mg, 0.14 mmol) was added to the reaction solution. The mixture was stirred at room temperature for 1 hour, and then added with methyl D-alaninate (9.9 mg, 0.071 mmol) and stirred overnight at room temperature. After completion of the reaction, the reaction was quenched by adding water (5 mL). The reaction system was extracted with ethyl acetate (3×10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 30% to 50%) to obtain the title compound (6.7 mg, 29.1 %) as a white solid.

$^1$H-NMR ($CD_3OD$) $\delta$: 7.99 (s, 1H), 7.55 (dd, *J* = 1.8, 7.5 Hz, 1H), 7.34-7.28 (m, 2H), 7.06-6.97 (m, 2H), 5.44-5.39 (m, 1H), 4.45 (q, *J* = 7.2 Hz, 1H), 4.12-4.03 (m, 2H), 3.88 (s, 3H), 3.75 (s, 3H), 3.80-3.67 (m, 2H), 3.48-3.36 (m, 4H), 3.03-2.93 (m, 1H), 2.87-2.81 (m, 1H), 2.83 (s, 3H), 2.73-2.56 (m, 2H), 1.82-1.72 (m, 2H), 1.57-1.45 (m, 2H), 1.42 (d, *J* = 7.5 Hz, 3H).

LC-MS: m/z 652.25 $[M + H]^+$.

Example 15: Preparation of *N*-((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)-*N*-methylglycine(15)

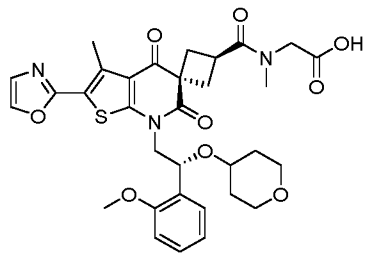

**15**

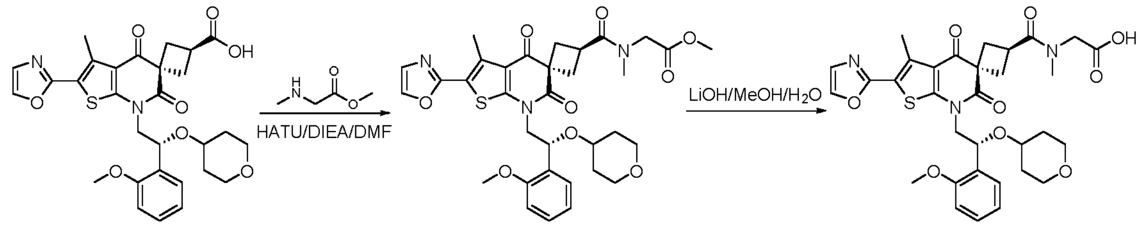


**8c** **15-1** **15**

Step 1: Preparation of *N*-((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)-N-methylglycine(**15-1**)

Compound **8c** (30 mg, 0.053 mmol) was dissolved in DMF (2 mL) at room temperature. Methyl sarcosinate hydrochloride (10.9 mg, 0.106 mmol), HATU (30.2 mg, 0.079 mmol) and triethylamine (21.4 mg, 0.212 mmol) were added to the reaction solution. The reaction mixture was stirred at room temperature overnight, and then quenched by adding water (5 mL). The reaction system was extracted with ethyl acetate (3×10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 40% to 50%) to obtain the title compound (8.0 mg, 23.1 %) as a white solid.

$^1H$-NMR ($CD_3OD$) δ: 7.99 (d, *J* = 0.9 Hz, 1H), 7.55 (dd, *J* = 1.5, 7.5 Hz, 1H), 7.34-7.29 (m, 2H), 7.06-8.97 (m, 2H), 5.45-5.40 (m, 1H), 4.21-4.11 (m, 4H), 3.89 (s, 3H), 3.80-3.66 (m, 6H), 3.56-3.46 (m, 2H), 3.10 (s, 3H), 3.02-2.96 (m, 2H), 2.92-2.83 (m, 4H), 2.79-2.63 (m, 2H), 1.84-1.72 (m, 2H), 1.55-1.40 (m, 2H).

LC-MS: m/z 652.35 $[M + H]^+$.

Step 2: Preparation of *N*-((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[cyclobutane-1,5'-thieno[2,3-b]pyridine]-3-carbonyl)-*N*-methylglycine (15)

Compound **15-1** (8.0 mg, 0.014 mmol) was dissolved in methanol/water mixed solution (1/1, 1 mL) at room temperature. Lithium hydroxide (0.7 mg, 0.028 mmol) was added to the reaction solution. The reaction mixture was stirred at room temperature for 2 hours, and then added with diluted hydrochloric acid until the pH of the reaction solution reached 2. The organic solvent was removed under reduced pressure, and the system was extracted with ethyl acetate (3×10 mL). The organic phases were combined and then washed with saturated brine (10 mL), dried over anhydrous sodium sulfate and filtered. The organic phase was concentrated under reduced pressure. The residues were purified by silica gel column chromatography (mobile phase: petroleum ether/ethyl acetate = 40% to 70%) to obtain the title compound (3.4 mg, 42.1 %) as a white solid.

$^1H$-NMR ($CD_3OD$) δ: 7.99-7.98 (m, 1H), 7.55 (dd, *J* = 1.2, 7.8 Hz, 1H), 7.34-7.28 (m, 2H), 7.06-6.97 (m, 2H), 5.44-5.40 (m, 1H), 4.13-4.04 (m, 4H), 3.87 (s, 3H), 3.80-3.70 (m, 3H), 3.57-3.45 (m, 2H), 3.40-3.37 (m, 1H), 3.09-3.08 (m, 2H), 3.04-2.97 (m, 2H), 2.93-2.89 (m, 1H), 2.87-2.83 (m, 3H), 2.78-2.63 (m, 2H), 1.83-1.72 (m, 2H), 1.58-1.40 (m, 2H).

LC-MS: m/z 638.40 $[M + H]^+$.

Example 16: Preparation of 2-(((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carbonyl)oxy)acetic acid (16)

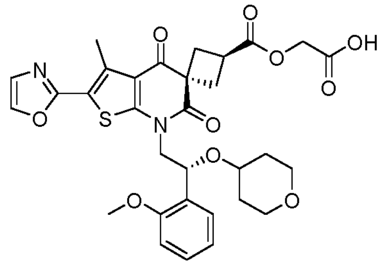

**16**

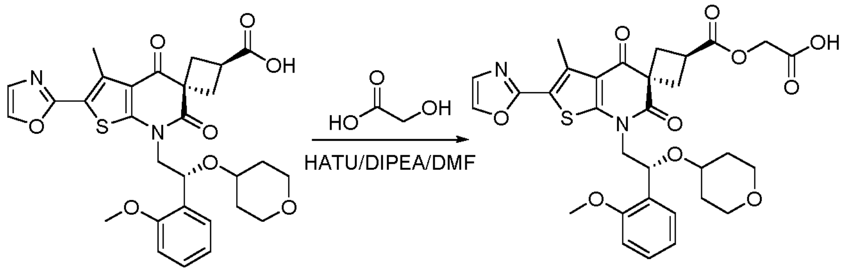


**8c** **16**

Compound **8c** (20 mg, 0.035 mmol) was dissolved in DMF (2 mL) at room temperature. HATU (26.8 mg, 0.071 mmol) and DIPEA(18.3 mg, 0.14 mmol) was added to the reaction solution. The mixture was stirred at room temperature for 1 hour, and then added with glycolic acid (5.4 mg, 0.071 mmol) and stirred at room temperature overnight. After completion of the reaction, the reaction was quenched by adding water (5 mL). The reaction system was extracted with ethyl acetate (3×10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 60% to 70%) to obtain the title compound (5.0 mg, 22.7 %) as a white solid.

$^1$H-NMR ($CD_3OD$) δ: 7.99 (s, 1H), 7.56-7.54 (m, 1H), 7.34-7.29 (m, 2H), 7.06-6.97 (m, 2H), 5.45-5.41 (m, 1H), 4.65 (m, 2H), 4.14-4.12 (m, 2H), 3.88 (s, 3H), 3.88-3.70 (m, 3H), 3.62-3.34 (m, 5H), 3.27-3.20 (m, 1H), 3.11-3.09 (m, 1H), 3.02-2.96 (m, 1H), 2.90-2.71 (m, 7H), 1.84-1.71 (m, 2H), 1.46-1.31 (m, 2H).

LC-MS: m/z 625.25 $[M + H]^+$.

Example 17: Preparation of ((1s,3S)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxamide (17)

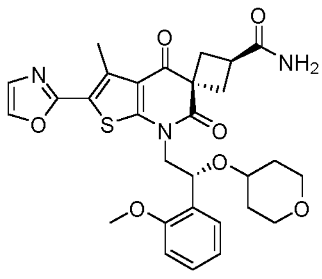

17

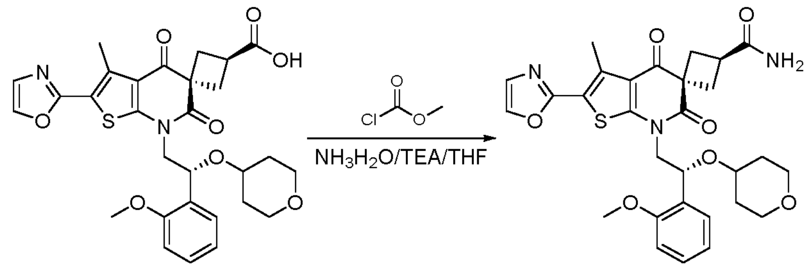


8c 17

Compound 8c (20 mg, 0.035 mmol) was dissolved in THF (2 mL) at room temperature. Triethylamine (10.7 mg, 0.106 mmol) and methyl chloroformate (6.7 mg, 0.07 mmol) were added to the reaction solution at 0°C successively. The mixture was stirred at room temperature for 1 hour, and then added with ammonia (7.42 mg, 0.212 mmol) and stirred at room temperature overnight. After completion of the reaction, the reaction was quenched by adding water (5 mL). The reaction system was extracted with ethyl acetate (3×10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 60% to 70%) to obtain the title compound (6 mg, 30.1 %) as a white solid.

$^1$H-NMR ($CD_3OD$) δ: 7.99 (s, 1H), 7.56-7.53 (m, 1H), 7.33-7.29 (m, 2H), 7.06-6.97 (m, 2H), 5.44-5.40 (m, 1H), 4.14-4.11 (m, 2H), 3.88 (s, 3H), 3.79-3.69 (m, 2H), 3.51-3.33 (m, 4H), 2.97-2.90 (m, 1H), 2.83-2.74 (m, 4H), 2.70-2.58 (m, 2H), 1.83-1.72 (m, 2H), 1.56-1.42 (m, 2H).

LC-MS: m/z 566.15 [M + H]$^+$.

Example 18: Preparation of (1s,3S)-*N*-(2-hydroxyethyl)-7'-((R)-2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[cyclobutane-1,5'-thieno[2,3-b]pyridine]-3-carboxamide (18)

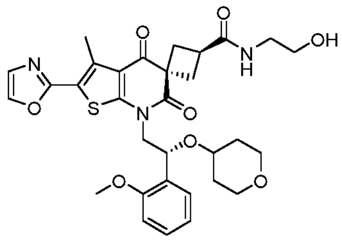

**18**

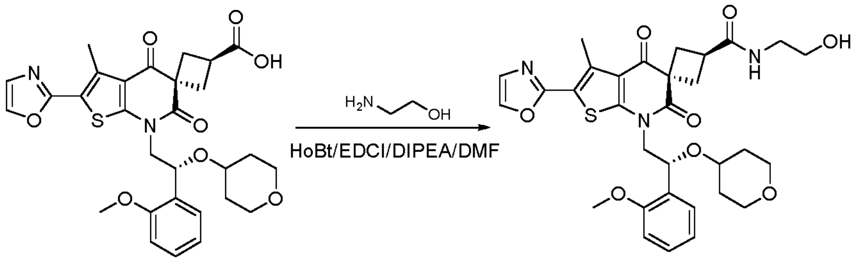


8c

18

Compound 8c (30 mg, 0.053 mmol) was dissolved in DMF (2 mL) at room temperature. Ethanolamine (4.3 mg, 0.071 mmol), 1-(3-dimethylaminopropyl)-3-ethylcarbodiimide hydrochloride (EDCI) (11.5 mg, 0.06 mmol), HOBt (8.1 mg, 0.06 mmol) and DIPEA (20.6 mg, 0.159 mmol) were added to the reaction solution. The reaction mixture was stirred at room temperature overnight, and quenched by adding water (5 mL). The reaction system was extracted with ethyl acetate (3×10 mL). The organic phases were combined and then washed with saturated brine (10 mL), dried over anhydrous sodium sulfate and filtered. The filtrate was concentrated under reduced pressure. The residues were purified by silica gel column chromatography (mobile phase: petroleum ether/ethyl acetate = 40% to 70%) to obtain the title compound (5.9 mg, 26.3 %) as a white solid.

$^1$H-NMR ($CD_3OD$) δ: 7.99 (s, 1H), 7.56-7.54 (m, 1H), 7.34-7.29 (m, 2H), 7.06-6.97 (m, 2H), 5.45-5.40 (m, 1H), 4.18-4.10 (m, 2H), 3.88 (s, 3H), 3.80-3.56 (m, 4H), 3.50-3.32 (m, 4H), 2.99-2.92 (m, 1H), 2.87-2.80 (m, 4H), 2.72-2.56 (m, 2H), 1.82-1.71 (m, 2H), 1.57-1.41 (m, 2H).

LC-MS: m/z 610.55 $[M + H]^+$.

Example 19: Preparation of tert-butyl (R)-2'-bromo-7'-(2-(2-methoxyphenyl)-2-((tetrahydro-2*H*-pyran-4-yl)oxy)ethyl)-3'-methyl-4',6'-dione-6',7'-dihydro-4'*H*-spiro[piperidine-4,5'-thieno[2,3-d]pyridine]-1-carboxylate (19)

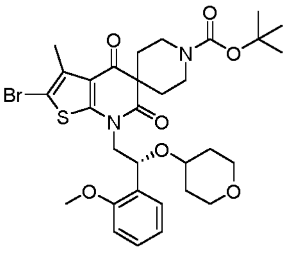

19

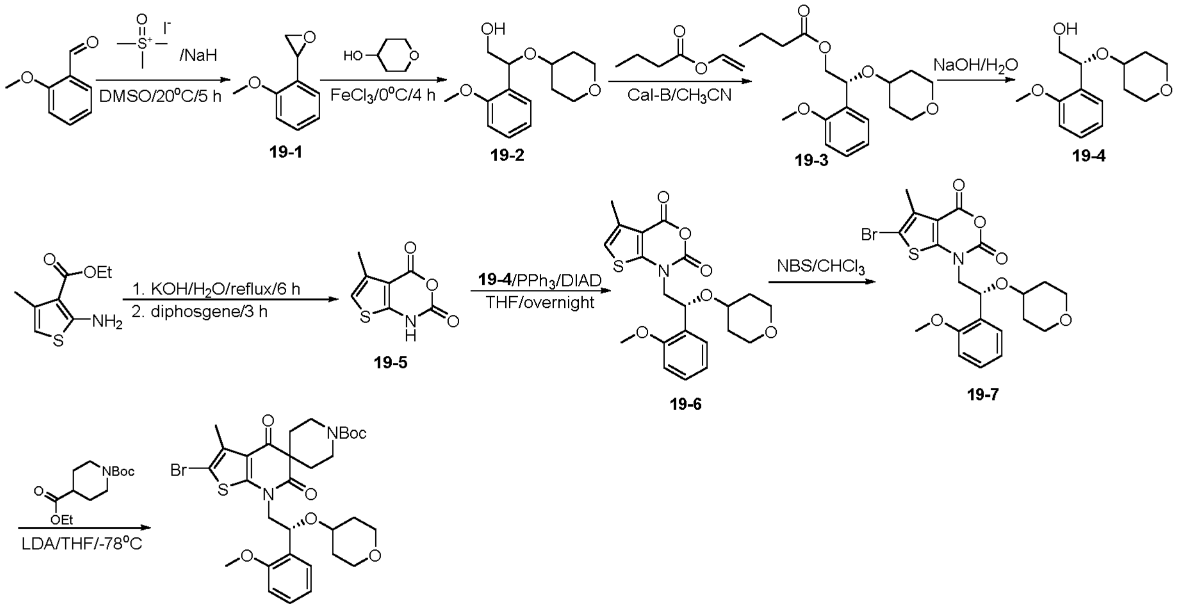


19-1 19-2 19-3 19-4 19-5 19-6 19-7 19

Step 1: Preparation of 2-methoxyphenyl oxirane (19-1)

Sodium hydride (35.3 g, 0.882 mol) was dissolved in dry dimethyl sulfoxide (800 mL) at room temperature. The mixed solution was cooled to 0°C, and then added with trimethylsulfoxide iodide (194 g, 0.882 mol). After stirring for 2 hours at 0°C, o-methoxybenzaldehyde (100 g, 0.735 mol) was slowly added. The mixture was stirred at room temperature for 3 hours, and then quenched by adding water (3000 mL). The reaction mixture was extracted with ethyl acetate (3×3000 mL). The organic phases were combined and then washed with saturated brine (2000 mL), dried over anhydrous sodium sulfate and filtered. The organic phase was concentrated under reduced pressure to obtain the crude title compound as a pale yellow oil (105 g). The crude product was used in the next step directly without purification.

Step 2: Preparation of 2-(2-methoxyphenyl)-2-((tetrahydro-2*H*-pyran-4-yl)oxy)ethan-1-ol (**19-2**)

Ferric chloride (16.9 g, 0.105 mol) was added to 4-tetrahydropyranol (204 g, 1.40 mol) at room temperature. The mixed solution was cooled to 0°C, and then slowly added with crude 2-methoxyphenyl oxirane (**19-1**) (105 g, 0.700 mol) dropwise. The reaction mixture was warmed to room temperature and stirred for 4 hours. The reaction was quenched by adding water (2000 mL). The reaction system was extracted with ethyl acetate (3×1000 mL). The organic phases were combined and then washed with saturated brine (1000 mL), dried over anhydrous sodium sulfate and filtered. The organic phase was concentrated under reduced pressure. The residues were purified by silica gel column chromatography (mobile phase: ethyl acetate/petroleum ether = 20% to 50%). The title compound (30.0 mg, 17.0%, a mixture of two optical isomers) was obtained as a colorless oil.

$^1$H-NMR ($CD_3OD$) δ: 7.43 (dd, *J*= 1.8, 7.5 Hz, 1H), 7.32-7.27 (m, 1H), 7.00 (t, *J*= 7.5 Hz, 1H), 6.90 (d, *J*= 7.5 Hz, 1H), 5.09 (dd, *J*= 3.6, 8.4 Hz, 1H), 4.03-3.90 (m, 2H), 3.85 (s, 3H), 3.70 (dd, *J*= 3.6, 11.4 Hz, 1H), 3.59-3.51 (m, 2H), 3.46-3.37 (m, 3H), 2.07-1.98 (m, 1H), 1.84-1.79 (m, 1H), 1.72-1.61 (m, 2H).

LC-MS: m/z 275.15 [M + Na]$^+$.

Step 3: Preparation of (R)-2-(2-methoxyphenyl)-2-((tetrahydro-2*H*-pyran-4-yl)oxy)ethyl butyrate (**19-3**)

Compound **19-2** (30.0 g, 0.12 mol), *Candida antarctica* enzyme (Beijing Cliscent Technology Co., Ltd.) (3.00 g) and vinyl n-butyrate (7.50 g, 0.07 mol) were dissolved in dry acetonitrile (150 mL) at room temperature. The reaction solution was stirred at room temperature for 2 hours and filtered. The organic phase was concentrated under reduced pressure. The residues were purified by silica gel column chromatography (mobile phase: ethyl acetate/petroleum ether = 20% to 40%) to obtain the title compound (15.0 g, 39.1%) as a colorless oil.

$^1$H-NMR ($CDCl_3$) $\delta$: 7.50 (d, *J* = 7.2 Hz, 1H), 7.33-7.27 (m, 1H), 7.10-6.98 (m, 1H), 6.92-6.87 (m, 1H), 5.23-5.04 (m, 1H), 4.21-4.13 (m, 2H), 4.02-3.85 (m, 5H), 3.51-3.40 (m, 3H), 2.38-2.30 (m, 2H), 1.98-1.66 (m, 6H), 1.02-0.95(m, 3H).

LC-MS: m/z 345.18 $[M + Na]^+$.

Step 4: Preparation of (R)-2-(2-methoxyphenyl)-2-((tetrahydro-2*H*-pyran-4-yl)oxy)ethan-l-ol (**19-4**)

Compound **19-3** (15.0 g, 0.05 mol) and sodium hydroxide (3.10 g, 0.08 mol) were dissolved in a mixed solution of methanol (30 mL) and water (30 mL) at room temperature. The reaction solution was stirred at room temperature for 2 hours. The organic phase was concentrated under reduced pressure and then filtered to obtain the title compound (10.0 g, 85.2%) as a white solid.

$^1$H-NMR ($CD_3OD$) $\delta$: 7.43 (dd, *J* = 1.8, 7.5 Hz, 1H), 7.32-7.27 (m, 1H), 7.00 (t, *J* = 7.5 Hz, 1H), 6.90 (d, *J* = 7.5 Hz, 1H), 5.09 (dd, *J* = 3.6, 8.4 Hz, 1H), 4.03-3.90 (m, 2H), 3.85 (s, 3H), 3.70 (dd, *J* = 3.6, 11.4 Hz, 1H), 3.59-3.51 (m, 2H), 3.46-3.37 (m, 3H), 2.07-1.98 (m, 1H), 1.84-1.79 (m, 1H), 1.72-1.61 (m, 2H).

LC-MS: m/z 275.15 $[M + Na]^+$.

Step 5: Preparation of 5-methyl-2*H*-thieno[2,3-d][1,3]oxazine-2,4(1*H*)-dione (**19-5**)

Ethyl 2-amino-methylthiophene-3-carboxylate (20.0 g, 0.108 mol) was dissolved in water (400 mL) at room temperature, and then added with potassium hydroxide solid (12.1 g, 0.216 mol). The reaction solution was heated to 100°C and refluxed for 6 hours. The resulting solution was cooled to 0°C, and then slowly added with diphosgene (21.2 g, 0.108 mol) dropwise. After the addition, the reaction mixture was stirred for 3 hours at room temperature. A solid was gradually precipitated out during the reaction. After completion of the reaction, the resulted solid was filtered, washed with water to neutrality, and then washed with petroleum ether (200 mL). The solid was dried under reduced pressure to obtain the title compound (7.00 g, 35.3%) as a brown solid.

LC-MS: m/z 184.25 $[M + H]^+$.

Step 6: Preparation of (R)-1-(2-(2-methoxyphenyl-2-((tetrahydro-2*H*-pyran-4-yl)oxy)ethyl)-5-methyl-2*H*-thieno[2,3-d][1,3]oxazine-2,4(1*H*)-dione (**19-6**)

Compound **19-5** (5.00 g, 27.3 mmol) was dissolved in anhydrous tetrahydrofuran (150 mL) under nitrogen protection at room temperature, and and then added with compound **19-4** (13.77 g, 54.6 mmol) and triphenylphosphine (14.3 g, 54.6 mmol) successively. The reaction solution was cooled to 0°C, and then slowly added with diisopropyl azodicarboxylate (11.0 g, 54.6 mmol) dropwise. After the addition, the reaction mixture was warmed to room temperature and stirred for 16 hours. After completion of the reaction, the reaction solution was concentrated under reduced pressure. The residues were purified by silica gel column chromatography (mobile phase: ethyl acetate/petroleum ether = 10% to 50%) to obtain the title compound (3.50 g, 30.6%) as a white solid.

LC-MS: m/z 418.15 $[M + H]^+$.

Step 7: Preparation of (R)-6-bromo-1-(2-(2-methoxyphenyl-2-((tetrahydro-2*H*-pyran-4-yl)oxy)ethyl)-5-methyl-2*H*-thieno[2,3-d][1,3]oxazine-2,4(1*H*)-dione (**19-7**)

Compound **19-6** (3.50 g, 8.37 mmol) was dissolved in chloroform (100 mL) at room temperature. N-bromosuccinimide (1.49 g, 8.37 mmol) was added to the solution. The reaction solution was stirred at room temperature for 3 hours, and then quenched by adding 5% aqueous solution of sodium thiosulfate (10 mL). The solution was diluted with dichloromethane (200 mL) and water (100 mL). After separation of the organic phase, the aqueous phase was extracted with dichloromethane ($3\times100$ mL), and the combined organic phase was washed with saturated brine (100 mL), dried over anhydrous sodium sulfate and filtered. The organic phase was concentrated under reduced pressure. The residues were purified by silica gel column chromatography (mobile phase: ethyl acetate/petroleum ether = 10% to 40%) to obtain the title compound (2.50 g, 60.2 %) as a pale yellow solid.

$^1$H-NMR (d6-DMSO) $\delta$: 7.46 (dd, J = 1.8, 7.5 Hz, 1H), 7.32 (dt, J = 1.85, 8.1 Hz, 1H), 7.06-6.99 (m, 2H), 5.24 (dd, J = 4.8, 8.4 Hz, 1H), 4.01-3.97 (m, 2H), 3.79 (s, 3H), 3.67-3.56 (m, 2H), 3.46-3.48 (m, 1H), 3.29-3.21 (m, 2H),

2.52-2.50 (m, 1H), 1.71-1.63 (m, 2H), 1.40-1.18 (m, 2H).

LC-MS: m/z 518.10 $[M + Na]^+$.

Step 8: Preparation of tert-butyl (R)-2'-bromo-7'-(2-(2-methoxyphenyl)-2-((tetrahydro-2*H*-pyran-4-yl)oxy)ethyl)-3'-methyl-4',6'-dione-6',7'-dihydro-4'*H*-spiro[piperidine-4,5'-thieno[2,3-d]pyridine]-1-carboxylate (**19**)

Methyl N-Boc-4-piperidine-carboxylate (243.3 mg, 1.21 mmol) was dissolved in dry THF (5 mL) under nitrogen atmosphere, and then added with LDA (1.29 mmol, 2 mol/L) at -78°C. The mixture was stirred at this temperature for 1 hour, and then added with compound **19-7** (200 mg, 0.40 mmol). The reaction solution was warmed to room temperature and stirred for 1 hour. After completion of the reaction, the reaction was quenched by adding water. The reaction system was extracted with ethyl acetate (3×20 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 10% to 40%) to obtain the title compound (243.0 mg, 90.5 %) as a white solid.

$^1$H-NMR ($CD_3OD$) $\delta$: 7.49 (dd, *J* = 3, 7.5 Hz, 1H), 7.32 (td, *J* = 1.8, 7.5 Hz, 1H), 7.05-6.97 (m, 2H), 5.38 (t, *J* = 6.6 Hz, 1H), 4.09-4.06 (m, 2H), 3.86 (s, 3H), 3.86-3.73 (m, 2H), 3.72-3.60 (m, 2H), 3.50-3.35(m, 5H), 2.33 (s, 3H), 2.03-1.70 (m, 6H), 1.56-1.39 (m, 11H).

LC-MS: m/z 686.95 $[M + H]^+$.

Example 20: Preparation of tert-butyl (R)-7'-(2-(2-methoxyphenyl)-2-((tetrahydro-2*H*-pyran-4-yl)oxy)ethyl)-3'-methyl-2'-(oxazol-2-yl)-4',6'-dione-6',7'-dihydro-4*H*-spiro[piperidine-4,5'-thieno[2,3-d]pyridine]-1-carboxylate (20)

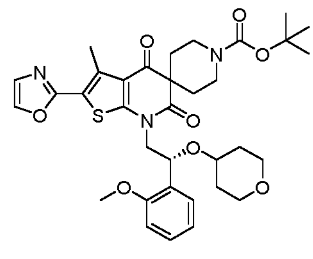

**20**

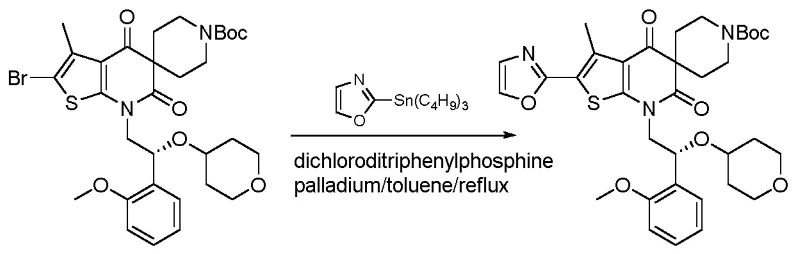


**19** **20**

2-(Tributylstannyl)oxazole (43.2 mg, 0.12 mmol) and bis(triphenylphosphorus)palladium dichloride (0.03 mmol, 21.2 mg) were added to a solution (3 mL) of compound **19** in dry toluene (40.0 mg, 0.06 mmol) under nitrogen atmosphere at room temperature. The reaction solution was heated and refluxed overnight. After completion of the reaction, the reaction was quenched by adding water. The reaction system was extracted with ethyl acetate (3×20mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 10% to 40%) to obtain the title compound (38.1 mg, 95.6 %) as a white solid.

$^1$H-NMR ($CD_3OD$) $\delta$: 7.**99** (s, 1H), 7.53 (dd, *J* = 1.5, 7.5 Hz, 1H), 7.35-7.29 (m, 2H), 7.06-6.98 (m, 2H), 5.45 (t, *J*= 6.3 Hz, 1H), 4.15 (m, 2H), 3.88 (s, 3H), 3.82-3.76 (m, 2H), 3.72-3.61 (m, 2H), 3.60-3.53(m, 1H), 3.51-3.43 (m, 2H), 3.40-3.36 (m, 2H), 2.00-1.72 (m, 6H), 1.56-1.40 (m, 11H).

LC-MS: m/z 652.25 $[M + H]^+$.

Example 21: Preparation of (R)-7'-(2-(2-methoxyphenyl)-2-((tetrahydro-2*H*-pyran-4-yl)oxy)ethyl)-3'-methyl-2'-(oxazol-2-yl)-4'*H*-spiro[piperidine-4,5'-thieno[2,3-d]pyridine]-4',6'(7'*H*)-dione (21)

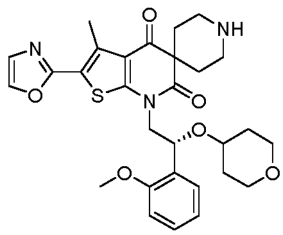

**21**

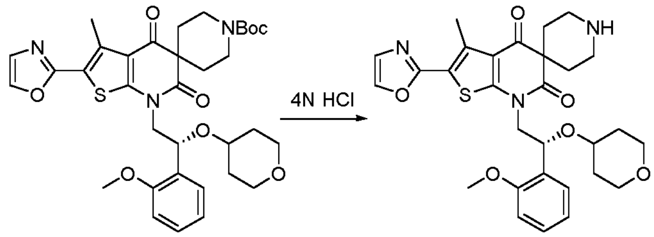


20

21

Compound **20** (35.0 mg, 0.054 mmol) was added to a solution of hydrochloric acid in 1,4-dioxane (4 mol/L, 2 mL) at room temperature. After stirring at room temperature for 1 hour, the reaction solution was concentrated under reduced pressure to remove the hydrochloric acid and organic solvent. The residues were purified by silica gel column chromatography (mobile phase: ethyl acetate/petroleum ether = 50% to 60%) to obtain compound **21** (28.1 mg, 90.2%) as a white solid.

$^1$H-NMR ($CD_3OD$) $\delta$: **8.00** (s, 1H), 7.53 (dd, *J* = 1.5, 7.5 Hz, 1H), 7.35-7.29 (m, 2H), 7.06-6.98 (m, 2H), 5.46 (t, *J*= 6.3 Hz, 1H), 4.12-4.11 (m, 2H), 3.88 (s, 3H), 3.83-3.76 (m, 2H), 3.68-3.35 (m, 3H), 3.22-3.08 (m, 4H ), 2.77 (m, 3H), 2.21-2.06 (m, 3H), 2.03-1.93 (m, 1H), 1.89-1.83 (m, 1H), 1.78-1.73 (m, 1H), 1.56-1.39 (m, 2H).

LC-MS: m/z 552.30 $[M + H]^+$.

Example 22: Preparation of methyl (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-yl)carboxylate(22)

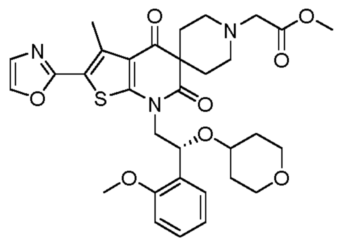

22

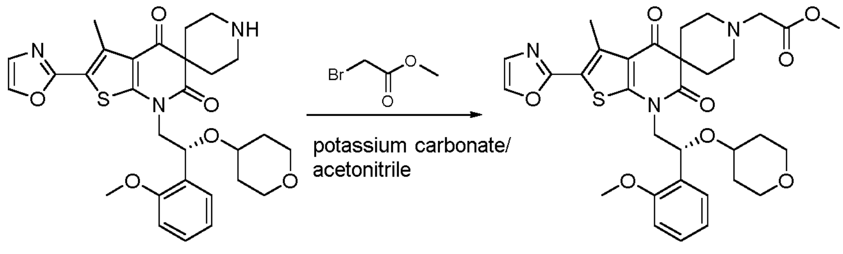


21 22

Methyl bromoacetate (6.1 mg, 0.036 mmol) and potassium carbonate (10.0 mg 0.073 mmol) were added to a solution (3 mL) of compound **21** in dry acetonitrile (20.0 mg, 0.036 mmol) under nitrogen atmosphere at room temperature. The reaction solution was stirred for 2 hours at room temperature, and then quenched by adding water. The reaction system was extracted with ethyl acetate (3×20 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 30% to 40%) to obtain compound 22 (15.3 mg, 62.9 %) as a white solid.

$^1$H-NMR ($CD_3OD$) $\delta$: 7.99 (d, *J* = 0.9 Hz, 1H), 7.53 (dd, *J* = 1.8, 7.5 Hz, 1H), 7.34-7.28 (m, 2H), 7.06-6.97 (m, 2H), 5.46-5.42 (m, 2H), 7.25 (q, *J* = 4.8 Hz, 2H), 4.17-4.12 (m, 2H), 3.86 (s, 3H), 3.81-3.77 (m, 2H), 3.51-3.42 (m, 1H), 3.39-3.35 (m, 4H), 2.93-2.88 (m, 2H), 2.85-2.81 (m, 2H), 2.76 (s, 3H), 2.27-2.12 (m, 3H), 2.09-1.98 (m, 1H), 1.87-1.73 (m, 2H), 1.58-1.40 (m, 2H), 1.31 (t, *J* = 7.2 Hz, 3H).

LC-MS: m/z 638.20 $[M + H]^+$.

Example 23: Preparation of (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-l-yl)acetic acid (23)

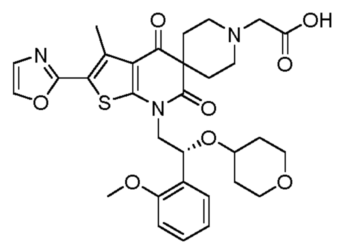

23

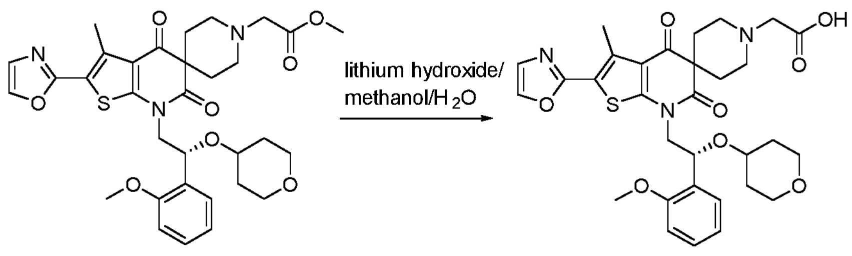


22 23

Compound 22 (20.0 mg, 0.03 mmol) and lithium hydroxide (1.4 mg, 0.06 mmol) were dissolved in a methanol/water mixed solution (6 mL, methanol/water = 1/1) under nitrogen atmosphere at room temperature. The reaction solution was stirred overnight at room temperature. After completion of the reaction, the organic solvent was removed under reduced pressure. The reaction solution was adjusted to pH 2 with dilute hydrochloric acid (pH = 1). The system was extracted with ethyl acetate (3×20 mL), and the combined organic phase was washed with saturated brine (20 mL), dried over anhydrous sodium sulfate and filtered. The filtrate was concentrated under reduced pressure. The residues were purified by silica gel column chromatography (mobile phase: ethyl acetate/petroleum ether = 30% to 40%) to obtain compound **23** (8.5 mg, 43.3 %) as a white solid.

$^1$H-NMR ($CD_3OD$) δ: 8.01 (d, *J* = 0.6 Hz, 1H), 7.52 (dd, *J* = 1.5, 7.5 Hz, 1H), 7.35-7.30 (m, 2H), 7.06-7.00 (m, 2H), 5.49-5.45 (m, 1H), 4.34-4.25 (m, 1H), 4.19-4.11 (m, 3H), 3.89 (s, 3H), 3.94-3.77 (m, 2H), 3.62-3.44 (m, 5H), 3.40-3.35 (m, 2H), 2.79 (s, 3H), 2.46-2.33 (m, 4H), 1.90-1.86 (m, 1H), 1.79-1.74 (m, 1H), 1.57-1.39 (m, 2H).

LC-MS: m/z 610.20 [M + H]$^+$.

Example 24: Preparation of (2-methoxy-2-oxoethyl) (R)-7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carboxylate (24)

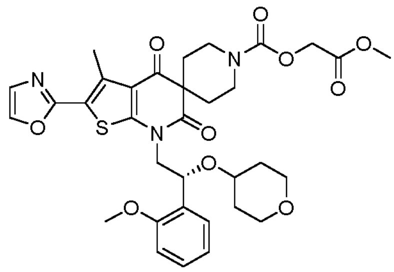

**24**

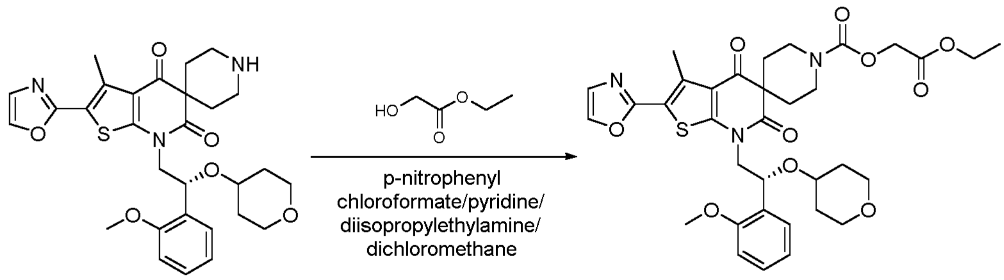


**21** **24**

P-nitrophenyl chloroformate (21.9 mg, 0.109 mmol) was added to a solution (3 mL) of 2-hydroxyethyl acetate (11.3 mg, 0.109 mmol) and pyridine (0.01 mL, 0.126 mmol) in dry dichloromethane at 0°C under nitrogen atmosphere. The reaction solution was stirred at 0°C for 2 hours, and then concentrated under reduced pressure to obtain a crude product. The crude product was added to a solution (3 mL) of compound **21** (30.0 mg, 0.054 mmol) and diisopropylethylamine (0.02 mL, 0.155 mmol) in dry dichloromethane. The reaction solution was stirred for 2 hours at room temperature, and then quenched by adding water. The system was extracted with ethyl acetate (3×20 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 50% to 70%) to obtain compound **24** (7.8 mg, 20.8 %) as a white solid.

$^1$H-NMR ($CD_3OD$) δ: 7.99 (d, *J* = 0.9 Hz, 1H), 7.54 (dd, *J* = 1.5, 7.5 Hz, 1H), 7.32-7.29 (m, 2H), 7.06-6.98 (m, 2H), 5.48-5.44 (m, 2H), 4.86 (s, 3H), 4.27 (q, *J* = 7.2 Hz, 4.20-4.16 (m, 2H), 3.88 (s, 3H), 3.84-3.75 (m, 3H), 3.71-3.57 (m, 2H), 3.50-3.43 (m, 1H), 3.39-3.33 (m, 2H), 2.77 (s, 3H), 2.11-2.01 (m, 3H), 1.94-1.84 (m, 2H), 1.80-1.73 (m, 1H), 1.57-1.40 (m, 2H), 1.32 (t, *J* = 6.9 Hz, 3H).

LC-MS: m/z 682.25 [M + H]$^+$.

Example 25: Preparation of (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)-2-methylpropionic acid (25)

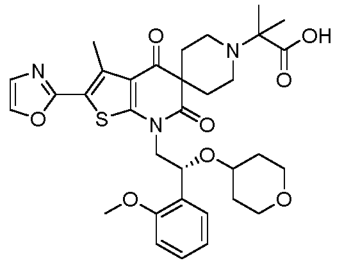

**25**

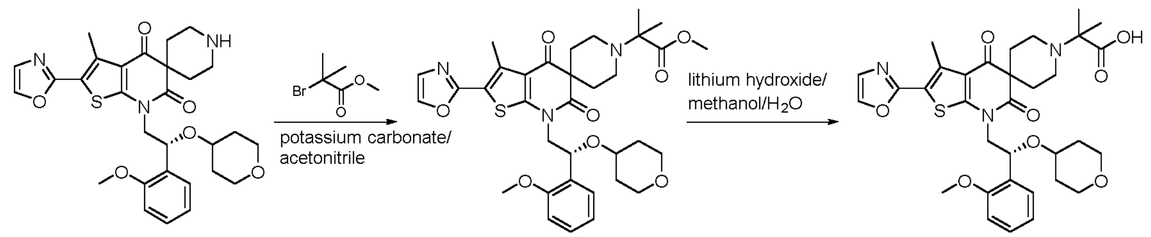


**21** **25-1** **25**

Step 1: Preparation of methyl (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)-2-methylpropanoate (**25-1**)

Methyl 2-bromoisobutyrate (26.5 mg, 0.136 mmol) and potassium carbonate (15.0 mg 0.109 mmol) were added to a solution (3 mL) of compound 21 in dry acetonitrile (15.0 mg, 0.027 mmol) under nitrogen atmosphere at room temperature. The reaction solution was stirred at 80°C for 16 hours, and then quenched by adding water. The reaction system was extracted with ethyl acetate (3×20 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 30% to 40%) to obtain the title compound (8.5 mg, 46.0 %) as a white solid.

$^1$H-NMR ($CD_3OD$) δ: 7.98 (d, *J* = 0.9 Hz, 1H), 7.53 (dd, *J* = 1.8, 7.5 Hz, 1H), 7.34-7.28 (m, 2H), 7.05-8.97 (m, 2H), 5.46-5.42 (t, *J* = 6.6 Hz, 1H), 4.25 (q, *J* = 6.9 Hz, 2H), 4.18-4.10 (m, 2H), 3.87 (s, 3H), 3.83-3.76 (m, 2H), 3.49-3.38 (m, 3H), 2.93-2.83 (m, 4H), 2.76 (s, 3H), 2.24-1.96 (m, 4H), 1.87-1.73 (m, 2H), 1.57-1.42 (m, 2H), 1.38-1.33 (m, 9H).

LC-MS: m/z 666.50 $[M + H]^+$.

Step 2: Preparation of (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[piperidine-4,5'-thieno[2,3-d]pyridin]-1-yl)-2-methylpropionic acid (compound **25**)

Compound **25-1** (13.0 mg, 0.019 mmol) was added to a solution of hydrochloric acid in 1,4-dioxane (3 mL) under nitrogen atmosphere at room temperature. After stirring at room temperature for 2 hours, the reaction solution was concentrated under reduced pressure to remove the hydrochloric acid and organic solvent. The residues were purified by silica gel column chromatography (mobile phase: ethyl acetate/petroleum ether = 50% to 70%) to obtain compound **25** (3.0 mg, 25.1 %) as a white solid.

$^1$H-NMR ($CD_3OD$) δ: 8.01 (s, 1H), 7.52 (d, *J* = 7.5 Hz, 1H), 7.36-7.31 (m, 2H), 7.05-6.98 (m, 2H), 5.48-5.44 (m, 1H), 4.34-4.18 (m, 2H), 3.87-3.80 (m, 5H), 3.52-3.45 (m, 1H), 3.42-37 (m, 5H), 3.27-3.10 (m, 1H), 2.79 (s, 3H), 2.48-2.03 (m, 4H), 1.93-1.89 (m, 1H), 1.78-1.73 (m, 1H), 1.58-1.49 (m, 8H).

LC-MS: m/z 638.20 $[M + H]^+$.

Example 26: Preparation of methyl (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-1-yl)-2-oxoacetate (26)

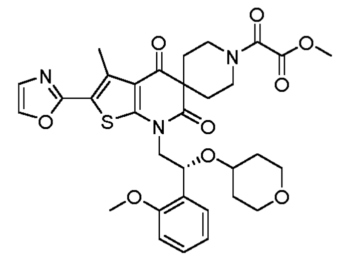

**26**

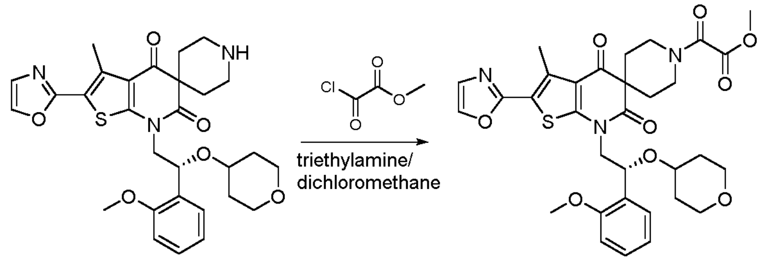


21 26

Methyl 2-chloro-2-oxoacetate (5.0 mg, 0.041 mmol) was added to a solution (3 mL) of compound **21** (15.0 mg, 0.027 mmol) and triethylamine (5.5 mg, 0.054 mmol) in dry dichloromethane at 0°C under nitrogen atmosphere. The reaction solution was stirred at room temperature for 3 hours, and then quenched by adding water. The reaction system was extracted with ethyl acetate (3 × 10 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 30% to 40%) to obtain compound **26** (8.5 mg, 46.0 %) as a white solid.

$^1$H-NMR ($CD_3OD$) δ: 8.00 (d, *J* = 0.9 Hz, 1H), 7.54 (dd, *J* = 1.8, 7.5 Hz, 1H), 7.35-7.29 (m, 2H), 7.06-6.99 (m, 2H), 5.48-5.43 (m, 1H), 4.29-4.08 (m, 2H), 3.92-3.87 (m, 7H), 3.84-3.77 (m, 3H), 3.71-3.61 (m, 3H), 3.51-3.45 (m, 3H), 2.78 (s, 3H), 2.22-2.15 (m, 1H), 2.10-2.04 (m, 2H), 1.94-1.74 (m, 3H), 1.47-1.45 (m, 2H), 1.36-1.25 (m, 2H).

LC-MS: m/z 638.20 $[M + H]^+$.

Example 27: Preparation of (R)-2-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridin]-l-yl)-2-oxoacetic acid (27)

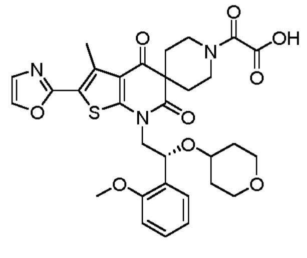

**27**

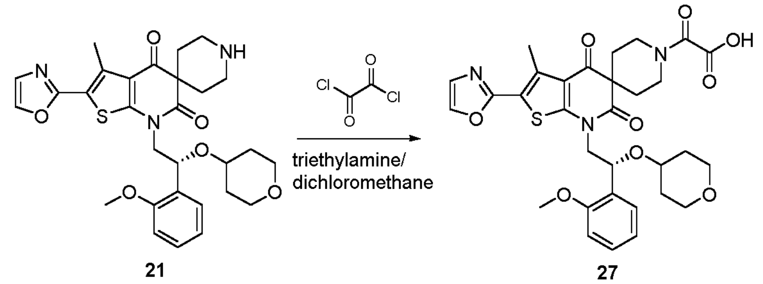


21 27

Oxalyl chloride (5.2 mg, 0.041 mmol) was added to a solution (3 mL) of compound **21** (28.0 mg, 0.051 mmol) and triethylamine (10.3 mg, 0.102 mmol) in dry dichloromethane at -10°C under nitrogen atmosphere. The reaction solution was stirred at -10°C for 20 minutes, and then warmed to room temperature and further stirred for 1 hour. The reaction was quenched by adding water. The reaction system was extracted with ethyl acetate (3×10 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 70% to 80%) to obtain compound **27** (15.0 mg, 46.4 %) as a white solid.

$^1$H-NMR ($CD_3OD$) δ: 7.99 (d, *J* = 0.9 Hz, 1H), 7.54 (dd, *J* = 1.8, 7.5 Hz, 1H), 7.34-7.28 (m, 2H), 7.05-6.97 (m, 2H), 5.45 (t, *J* = 6.6 Hz, 1H), 4.19-4.17 (m, 1H), 3.87 (s, 3H), 3.86-3.78 (m, 4H), 3.66-3.45 (m, 3H), 3.36-3.33 (m, 2H), 2.76 (s, 3H), 3.19-2.07 (m, 3H), 1.99-1.85 (m, 3H), 1.77-1.72 (m, 1H), 1.56-1.31 (m, 2H).

LC-MS: m/z 624.40 $[M + H]^+$.

Example 28: Preparation of methyl (R)-7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carboxylate (28)

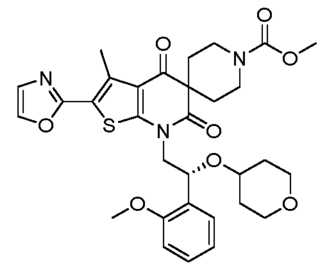

**28**

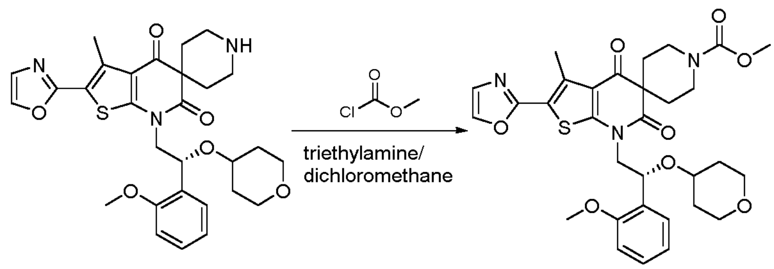


21 28

The preparation method was the same as in Example 26, except that methyl chloroformate was used instead of methyl 2-chloro-2-oxoacetate to obtain compound 28.

$^1$H-NMR ($CD_3OD$) δ: 7.99 (d, *J* = 0.9 Hz, 1H), 7.54 (dd, *J* = 1.8, 7.8 Hz, 1H), 7.35-7.27 (m, 2H), 7.06-6.98 (m, 2H), 5.45 (t, *J* = 6.6 Hz, 1H), 4.49-4.16 (m, 1H), 3.88 (m, 3H), 3.83-3.76 (m, 3H), 3.73-3.70 (m, 4H), 3.57-3.36 (m, 5H), 2.77 (s, 3H), 2.09-1.99 (m, 3H), 1.88-1.83 (m, 2H), 1.77-1.73 (m, 1H), 1.56-1.40 (m, 2H).

LC-MS: m/z 610.25 $[M + H]^+$.

Example 29: Preparation of (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carbonyl)glycine (29)

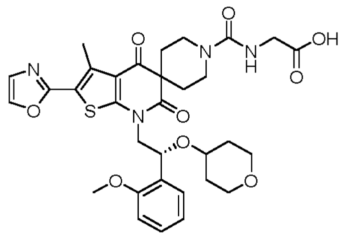

**29**

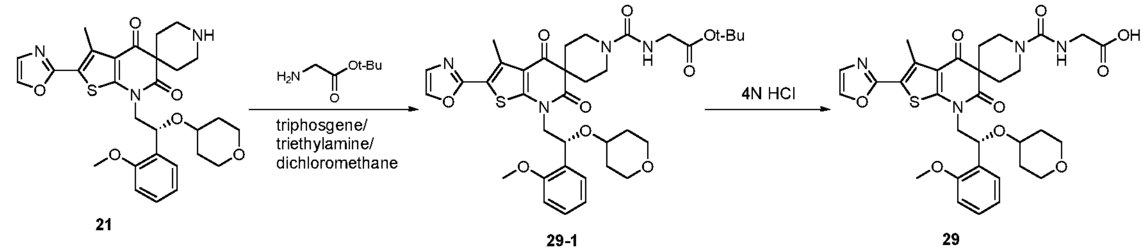


21 29-1 29

Step 1: Preparation of tert-butyl (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carbonyl)glycinate (**29-1**)

Triphosgene (8.0 mg, 0.027 mmol) was added to a solution (3 mL) of tert-butyl glycinate (14.2 mg, 0.108 mmol) and triethylamine (27.3 mg, 0.27 mmol) in dry dichloromethane at 0°C under nitrogen atmosphere. The reaction solution was stirred at 0°C for 3 hours, and then added with compound **21** (30.0 mg, 0.054 mmol). The reaction solution was warmed to room temperature and stirred overnight. The reaction was quenched by adding water and the reaction system was extracted with ethyl acetate (3 × 10 mL). The combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 30% to 40%) to obtain compound **29-1** (24.1 mg, 65.2 %) as a white solid.

$^1$H-NMR ($CD_3OD$) δ: 7.99 (d, *J* = 0.9 Hz, 1H), 7.54 (dd, *J* = 1.5, 7.5 Hz, 1H), 7.35-7.29 (m, 2H), 7.08-6.98 (m, 2H), 5.45 (t, *J* = 6.6 Hz, 1H), 4.17-4.15 (m, 2H), 3.88 (s, 3H), 3.84-3.71 (m, 5H), 3.68-3.44 (m, 4H), 2.77 (s, 3H), 2.12-2.04 (m, 3H), 1.94-1.84 (m, 2H), 1.78-1.73 (m, 1H), 1.54-1.39 (m, 11H).

LC-MS: m/z 708.60 $[M + H]^+$.

Step 2: Preparation of (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[piperidine-4,5'-thieno[2,3-d]pyridine]-1-carbonyl)glycine (**29**)

Compound **29-1** (20.0 mg, 0.028 mmol) was dissolved in a solution of hydrochloric acid in 1,4-dioxane (3 mL) under nitrogen atmosphere at room temperature. After stirring at room temperature for 2 hours, the reaction solution was concentrated under reduced pressure to remove the hydrochloric acid and organic solvent. The residues were purified by silica gel column chromatography (mobile phase: ethyl acetate/petroleum ether = 50% to 70%) to obtain compound **29** (13.0 mg, 64.4 %) as a white solid.

$^1$H-NMR ($CD_3OD$) δ: 7.99 (d, *J* = 0.9 Hz, 1H), 7.54 (dd, *J* = 1.5, 7.5 Hz, 1H), 7.35-7.29 (m, 2H), 7.08-6.98 (m, 2H), 5.47-5.43 (m, 1H), 4.18-4.15 (m, 2H), 3.89-3.88 (m, 4H), 3.84-3.71 (m, 4H), 3.68-3.44 (m, 4H), 2.77 (s, 3H), 2.12-2.04 (m, 3H), 1.94-1.84 (m, 2H), 1.78-1.73 (m, 1H), 1.54-1.39 (m, 2H).

LC-MS: m/z 653.20 $[M + H]^+$.

Example 30: Preparation of (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[piperidine-4,5'-thieno[2,3-d]pyridine]-1-sulfonic acid (30)

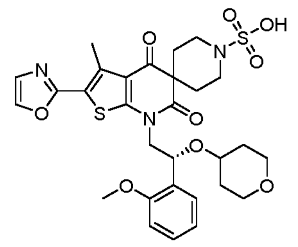

**30**

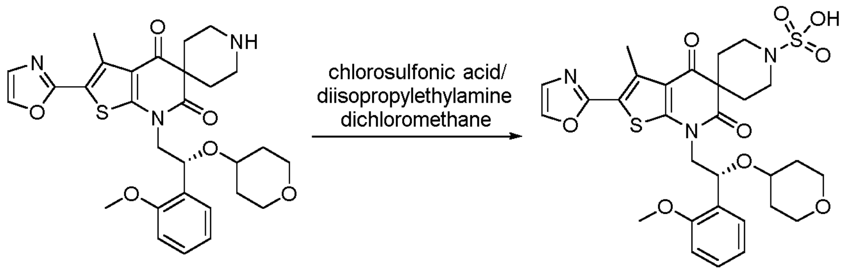


**21** **30**

Chlorosulfonic acid (8.5 mg, 0.073 mmol) was added to a solution (3 mL) of compound **21** (20.0 mg, 0.036 mmol) and diisopropyl ethyl amine (14.1 mg, 0.109 mmol) in dry dichloromethane at 0°C under nitrogen atmosphere. The reaction solution was stirred at room temperature overnight, and then quenched by adding water. The reaction system was extracted with ethyl acetate (3×10 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 60% to 80%) to obtain compound 30 (5.1 mg, 21.8 %) as a white solid.

$^1$H-NMR ($CD_3OD$) δ: 8.00 (s, 1H), 7.52-7.51 (d, *J* = 7.5 Hz, 1H), 7.35-7.30 (m, 2H), 7.05-6.98 (m, 2H), 5.48-5.43 (m, 1H), 4.30-4.12 (m, 2H), 3.88 (s, 3H), 3.84-3.79 (m, 3H), 3.62-3.46 (m, 5H), 3.41-3.34 (m, 3H), 2.77 (s, 3H), 2.37-2.26 (m, 2H), 2.15-2.06 (m, 2H), 1.90-1.86 (m, 1H), 1.78-1.74 (m, 1H), 1.57-1.42 (m, 2H).

LC-MS: m/z 632.45 $[M + H]^+$.

Example 31: Preparation of (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[piperidine-4,5'-thieno[2,3-d]pyridine]-1-carboxamide (31)

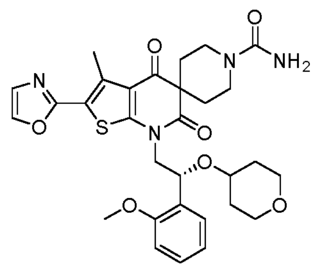

31

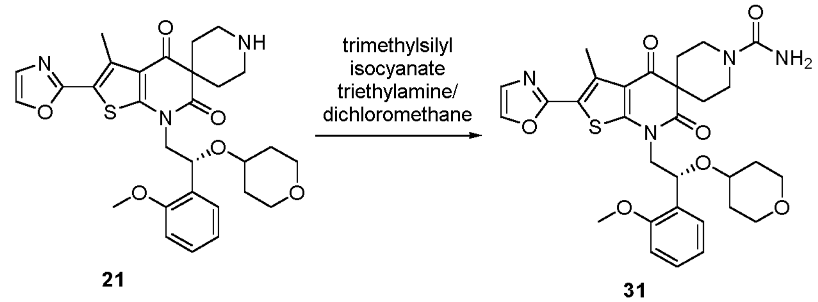


**21** **31**

Trimethylsilyl isocyanate (52.2 mg, 0.453 mmol) was added to a solution (3 mL) of compound **21** (25.0 mg, 0.045 mmol) and triethylamine (101.2 mg, 0.227 mmol) in dry dichloromethane at 0°C under nitrogen atmosphere. The reaction solution was stirred at room temperature overnight, and then quenched by adding water. The reaction system was extracted with ethyl acetate (3 × 10 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 60% to 80%) to obtain compound **31** (9.1 mg, 32.5 %) as a white solid.

$^1$H-NMR ($CD_3OD$) δ: 7.99 (d, *J* = 0.9 Hz, 1H), 7.54 (dd, *J* = 1.2, 5.7 Hz, 1H), 7.34-7.29 (m, 2H), 7.06-6.98 (m, 2H), 5.45 (t, *J* = 7.8 Hz, 1H), 4.18-4.14 (m, 2H), 3.88 (s, 3H), 3.84-3.76 (m, 3H), 3.72-3.63 (m, 2H), 3.60-3.56 (m, 1H), 3.52-3.44 (m, 2H), 3.39-3.34 (m, 2H), 2.77 (s, 3H), 2.12-2.00 (m, 3H), 1.93-1.84 (m, 2H), 1.77-1.74 (m, 1H), 1.5-1.40 (m, 2H).

LC-MS: m/z 595.20 $[M + H]^+$.

Example 32: Preparation of (R)-(7'-(2-(2-methoxyphenyl)-2-((tetrahydro-2*H*-pyran-4-yl)oxy)ethyl)-N,3'-dimethyl-2'-(oxazol-2-yl)-4',6'-dioxo-6',7'-dihydro-4'*H*-spiro[piperidine-4,5'-thieno[2,3-d]pyridine]-1-carboxamide (32)

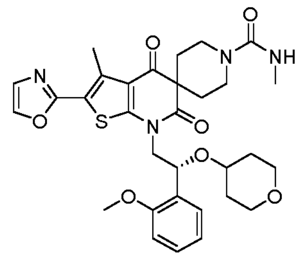

**32**

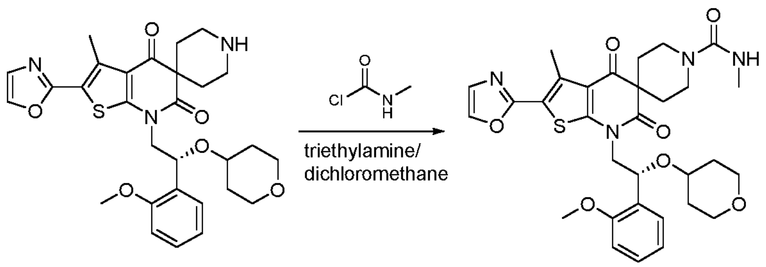


21 32

The preparation method was the same as in Example 31, except that methylcarbamic chloride was used instead of trimethylsilyl isocyanate to obtain compound 32.

$^1$H-NMR ($CD_3OD$) δ: 7.99 (d, *J* = 0.6 Hz, 1H), 7.54 (dd, *J* = 1.5, 7.5 Hz, 1H), 7.34-7.29 (m, 2H), 7.06-6.96 (m, 2H), 5.46-5.43 (m, 1H), 4.19-4.14 (m, 2H), 3.88 (s, 3H), 3.82-3.77 (m, 2H), 3.73-3.61 (m, 2H)3.58-3.44 (m, 3H), 2.77 (s, 3H), 2.76 (s, 3H), 2.10-1.99 (m, 3H), 1.91-1.84 (m, 2H), 1.77-1.73 (m, 1H), 1.55-1.40 (m, 2H).

LC-MS: m/z 609.20 $[M + H]^+$.

Example 33: Preparation of (R)-*N*-(2-hydroxyethyl)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4'*H*-spiro[piperidine-4,5'-thieno[2,3-d]pyridine]-1-carboxamide (33)

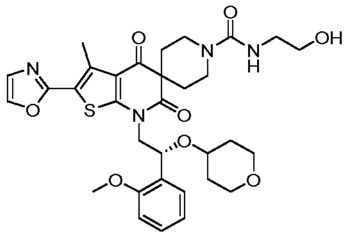

**33**

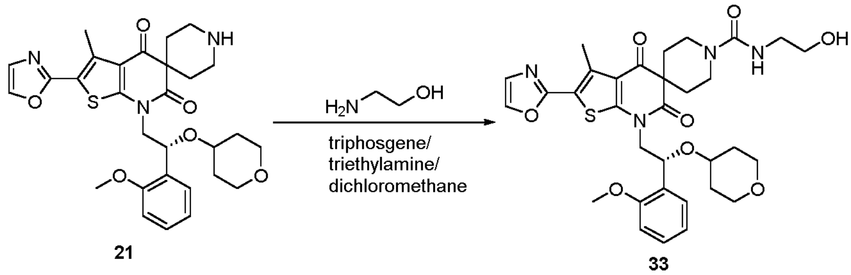


**21** **33**

Triphosgene (8.0 mg, 0.027 mmol) was added to a solution (3 mL) of aminoethanol (6.6 mg, 0.108 mmol) and triethylamine (27.3 mg, 0.27mmol) in dry dichloromethane at 0°C under nitrogen atmosphere. The reaction solution was stirred at 0°C for 3 hours, and then added with compound **21** (30.0 mg, 0.054 mmol). The reaction solution was warmed to room temperature and stirred overnight. The reaction was quenched by adding water and the system was extracted with ethyl acetate (3 × 10 mL). The combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 60% to 70%) to obtain compound **33** (7.2 mg, 22.5 %) as a white solid.

$^1$H-NMR ($CD_3OD$) δ: 7.99 (d, *J* = 0.9 Hz, 1H), 7.54 (dd, *J* = 1.8, 7.5 Hz, 1H), 7.35-7.29 (m, 2H), 7.06-6.98 (m, 2H), 5.45 (t, *J* = 7.8 Hz, 1H), 4.18-4.16 (m, 2H), 3.88 (s, 3H), 3.83-3.76 (m, 2H), 3.72-3.61 (m, 4H), 3.57-3.44 (m, 3H), 3.39-3.34 (m, 3H), 2.77 (s, 3H), 2.14-2.00 (m, 3H), 1.94-1.84 (m, 2H), 1.78-1.73 (m, 1H), 1.57-1.40 (m, 2H).

LC-MS: m/z 639.20 $[M + H]^+$.

Example 34: Preparation of diethyl (R)-(7'-(2-(2-methoxyphenyl)-2-((tetrahydro-2*H*-pyran-4-yl)oxy)ethyl)-3'-methyl-2'-(oxazol-2-yl)-4',6'-dioxo-6',7'-dihydro-4*H*-spiro[piperidine-4,5'-thieno[2,3-d]pyridine]-1-yl)phosphate (34)

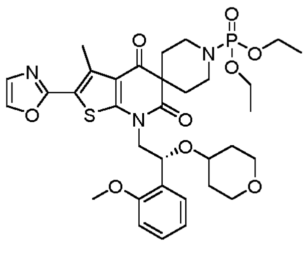

**34**

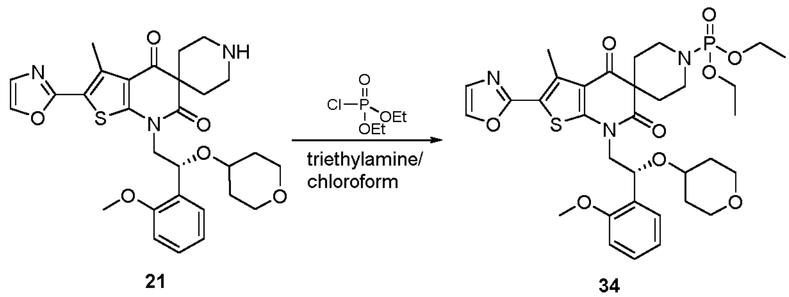


**21** **34**

Diethyl chlorophosphate (4.7 mg, 0.027 mmol) was added to a solution (3 mL) of compound 21 (10.0 mg, 0.018 mmol) and triethylamine (3.7 mg, 0.036 mmol) in dry chloroform at 0°C under nitrogen atmosphere. The reaction solution was stirred at room temperature for 3 hours, and was quenched by adding water. The reaction system was extracted with ethyl acetate (3 × 10 mL), and the combined organic phase was washed with saturated brine (20 mL), dried over anhydrous sodium sulfate and filtered. The organic phase was concentrated under reduced pressure. The residues were purified by silica gel column chromatography (mobile phase: ethyl acetate/petroleum ether = 40% to 80%) to obtain compound **34** (4.5 mg, 30.8 %) as a white solid.

$^1$H-NMR ($CD_3OD$) δ: 7.99 (d, *J* = 0.9 Hz, 1H), 7.54 (dd, *J* = 1.8, 7.5 Hz, 1H), 7.34-7.29 (m, 2H), 7.06-6.98 (m, 2H), 5.45 (t, *J* = 6.6 Hz, 1H), 4.17-4.14 (m, 1H), 4.12-4.02 (m, 4H), 3.89 (s, 3H), 3.83-3.78 (m, 3H), 3.50-3.34 (m, 4H), 2.77 (s, 3H), 2.06-1.97 (m, 3H), 1.88-1.84 (m, 2H), 1.78-1.74 (m, 1H), 1.56-1.42 (m, 2H), 1.39-1.31 (m, 6H).

LC-MS: m/z 688.25 $[M + H]^+$.

## Biological Tests

### Test Example 1: Analysis of the *in vitro* inhibition of the present compound on acetyl-CoA carboxylase (ACC) activity

ADP-Glo™ Kinase Analysis Kit (Promega) is used for the *in vitro* analysis of the inhibitory effect of the present compound on ACC1 or ACC2. ADP-Glo™ Kinase Analysis is a kinase detection analysis of luminescence method. ADP is produced in the process of catalyzing the substrate by ACC. ADP is converted into ATP which is then converted into light by Ultra-Glo™ luciferase. ADP is quantified by detecting the light signal, thus the enzyme activity can be measured by quantification of the ADP amount produced during the enzyme reaction. The analysis is carried out in two steps: in the first step, after the kinase reaction, a portion of ADP-Glo™ reagent equal in volume to the kinase reaction system is added to terminate the reaction and consume the remaining ATP; in the second step, a kinase detection reagent is added, which not only converts ADP into ATP, but also uses a coupled luciferase/luciferin reaction to detect newly synthesized ATP.

Test methods:

a. 10 mM compound stock solution (the present compound was dissolved in 100% DMSO to prepare the 10 mM stock solution) was diluted 50-fold to 200 μM with 100% DMSO, and then diluted in equal proportions (1:3) in a 96-well dilution plate (249944, Nunc). The gradient concentrations of the compound were 200 μM, 66.67 μM, 22.22 μM, 7.41 μM, 2.47 μM, 0.823 μM, 0.274 μM, 0.0914 μM, 0.0305 μM, 0.0102 μM, 0.00339 μM and 0.00113 μM.
b. 4.5 μL ACC1 working solution (BPS bioscience) (2.22 nM) was added to each well of a 384-well test plate (6007290, Perkin Elmer).
c. 0.5 μL of the serially diluted compound (prepared in step a) was added to each well of the 384-well test plate, which was centrifuged at 1000 rpm for 1 min and then incubated at room temperature for 15 min. The reaction system with compound GS-0976 at a final concentration of 0.1 μM was used as positive control; the reaction system with no compound but DMSO at a final concentration of 5% was used as negative control.
d. In the 384-well test plate (prepared in step c), 5 μL of the substrate working solution was added to each well to start the enzymatic reaction. The plate was centrifuged at 1000 rpm for 1 min and then incubated at room temperature for 30 min. The final concentration of each component in the ACC1 reaction system: 1 nM ACC1, 10 μM acetyl-CoA, 30 mM $NaHCO_3$, and 20 μM ATP. The final concentration of each component in the ACC2 reaction system: 1.1 nM ACC2, 20 μM acetyl-CoA, 20 mM $NaHCO_3$, and 15 μM ATP. Final concentrations of test compounds: 10 μM, 3.33 μM, 1.11 μM, 0.37 μM, 0.123 μM, 0.0411 μM, 0.0137 μM, 0.00457 μM, 0.00152 μM, 0.00051 μM, 0.00017 μM and 0.000056 μM. The final concentration of DMSO was 5% (v/v).
e. 10 μL of ADP-Glo reagent was added to each well of the 384-well test plate (prepared in step d), which was centrifuged at 1000 rpm for 1 min and then incubated at room temperature for 40 min.
f. 20 μL of kinase detection reagent was added to each well of the 384-well test plate (prepared in step e), which was centrifuged at 1000 rpm for 1 min and then incubated at room temperature for 40 min.
g. The chemiluminescence signal was detected with Perkin Elmer EnVision 2104.

ACC1 working solution and substrate working solution were both prepared with 1× kinase reaction buffer. The buffer was consisting of 50 mM HEPES buffer, 2 mM magnesium chloride, 0.01% BRIJ-35, 2 mM potassium citrate, 50 μg/mL BSA and 2 mM DTT.

The average value of the positive and negative control well and the standard deviation were calculated. The inhibition percentage was calculated according to the following formula: 100 × [1 - (compound - average value of positive control) / (average value of negative control-average value of positive control)].

$IC_{50}$ of the compound was obtained using GraphPad Prism 6 software according to the following nonlinear fitting formula:

$$Y = Bottom + (Top - Bottom) / (1 + 10 ^\wedge ((Log\ IC_{50} - X) \times Hillslope)),$$

wherein X is the logarithm of the compound concentration, Y is the inhibition rate; Top and Bottom are the Y values at the highest and lowest plateau of the curve; Hillslope is the Hill constant.

The inhibitory activities of the present compound on ACC1 and ACC2 are shown in Table 2 below.

In Table 2, A means the inhibitory activity of the compound with $IC_{50}$ < 10 nM; B means 10 nM < $IC_{50}$ < 100 nM; C means 100 nM < $IC_{50}$ < 500 nM; D means $IC_{50}$ > 500 nM.

Table 2 The inhibitory $IC_{50}$ values of the present compound on ACC1 and ACC2

| Compound No. | $IC_{50}$ (nM) | |
|---|---|---|
| | ACC1 | ACC2 |
| 1 | D | |
| 2 | D | |
| 3 | C | |
| 4 | C | |
| 5 | A | |
| 5x | A | |
| 5y | A | |
| 5a | B | |
| 5b | A | A |
| 5c | A | |
| 5d | A | |
| 5e | B | |
| 6 | C | |
| 7a | D | |
| 7b | C | |
| 8 | A | |
| 8a | B | |
| 8b | C | |
| 8c | A | A |
| 8d | A | A |
| 9 | A | |
| 10 | A | |
| 10-1 | A | |
| 11 | A | |
| 12 | A | |
| 13 | A | |
| 14 | A | |
| 14-1 | A | |
| 15 | A | |
| 15-1 | A | |
| 16 | A | |
| 17 | A | |
| 18 | A | |
| 20 | B | |
| 21 | A | |
| 22 | B | |
| 23 | A | |
| 24 | B | |
| 25-1 | B | |
| 25 | B | |

(continued)

| Compound No. | $IC_{50}$ (nM) | |
|---|---|---|
| | ACC1 | ACC2 |
| 26 | A | |
| 27 | A | |
| 28 | A | |
| 29 | A | |
| 30 | A | |
| 31 | A | |
| 32 | A | |
| 33 | A | |

The present compound can inhibit the enzyme activity of ACC1 and ACC2.

Test Example 2: *In vitro* inhibitory effect of the present compound on fatty acid synthesis in HepG2 cells

Human liver tumor cells (HepG2 cells) with strong fatty acid synthesis ability were selected. The sample to be tested was added and incubated for 1 hour. Isotope-labeled [$^{14}$C]-sodium acetate was added to the cell culture medium and incubated for 5 hours. Then the cells were lysed, and the fatty acid components in the cell were extracted and separated. The relative content of the isotope integrated into the fatty acid was analyzed, so as to detect the inhibitory effect of the compound on fatty acid synthesis in HepG2 cells at different concentrations, and the $IC_{50}$ value was calculated.

Test methods:

a. HepG2 cells were purchased from the American type culture collection (ATCC) resource bank. Cells were cultured in DMEM (GIBCO) containing 10% fetal bovine serum (GIBCO), penicillin (100 units/mL) and streptomycin (100 μg/mL) (GIBCO), and then incubated in a 37°C incubator containing 5% carbon dioxide, and passaged once every 2 to 3 days.
b. On the first day, HepG2 cells were inoculated in a 24-well cell culture plate at $2\times10^5$ per well, and were incubated in a 37°C incubator containing 5% carbon dioxide.
c. On the fourth day, the medium was changed to that containing the compound. The initial concentration of the compound was 3 μM or 0.3 μM, 3-fold dilution, 8 concentration gradients, and the final concentration of DMSO was 0.5% (v/v). The plate was incubated in a 37°C incubator containing 5% carbon dioxide for 1 hour. GS-0976 was positive compound control; the reaction system with no compound but DMSO at a final concentration of 0.5% was negative control.
d. 1 μCi [2-$^{14}$C]-acetic acid was added to each well, and incubated for 5 hours in a 37°C incubator containing 5% carbon dioxide.
e. The medium was first transferred to 15 mL centrifuge tubes. 0.5 mL of 0.1 M NaOH was added to each well of cells, and the lysed cell suspension was added to the corresponding 15 mL centrifuge tubes. 1 mL of ethanol and 0.17 mL of 50% KOH were added to each tube, and incubated in a water bath at 90°C for 1 hour.
f. The sample were taken out and cooled to room temperature, then 5 mL petroleum ether was added to each tube, inverted several times, and centrifuged at 1000 rpm for 5 min. The upper organic phase was discarded and the aqueous phase was kept for fatty acid extraction. 1 mL of concentrated hydrochloric acid (its pH was ensured to be below 1) was added to each tube.
g. 5 mL petroleum ether was added to each tube, invert several times and centrifuged at 1000 rpm for 5 min. 4 mL petroleum ether layer was transferred to a new glass tube (18 × 180 mm). This step was repeated once.
h. The extracts were collected and placed in a water bath at 64°C to evaporate for 30 min. Fatty acids were dissolved with 400 μL chloroform/n-hexane (1:1).
i. 50 μL sample were placed in iso-plate (Perkinelmer), 200 μL ULTIMA GOLD (Perkinelmer) was added to each well and incubated for 10 min at room temperature.
j. MicroBeta (Perkinelmer) was used to record the CPM scintillation signal.

The average value of the positive and negative control well and the standard deviation were calculated. $IC_{50}$ was fitted by using XLFit 5.3.1.3 (2006-2011 ID Business Solutions Limited) with 4-Parameter Logistic Model or Sigmoidal Dose-Response Model:

$$Y = (Bottom + ((Top - Bottom) / (1 + ((Log IC_{50} / X) ^ Hillslope)))),$$

wherein X is the logarithm of the compound concentration, Y is CPM (count per minute); Top and Bottom are the Y values at the highest and lowest plateau of the curve; Hillslope is the Hill constant.

The inhibitory activities of the present compound on fatty acid synthesis in HepG2 cells are shown in Table 3 below.

In Table 3, A means the inhibitory activity of the compound with $IC_{50}$ < 50 nM; B means 50 nM < $IC_{50}$ < 500 nM; C means $IC_{50}$ > 500 nM.

Table 3 The inhibitory $IC_{50}$ values of the present compound on fatty acid synthesis in HepG2 cells

| Example | $IC_{50}$ (nM) |
|---|---|
| 5x | B |
| 5y | A |
| 5b | A |
| 8 | A |
| 8c | A |
| 8d | A |

The compound of the invention can inhibit fatty acid synthesis in HepG2 cells.

Test Example 3: Analysis of the *in vitro* inhibition effect of the present compound on pyruvate carboxylase (PC) activity

The *in vitro* analysis of the inhibitory effect of the present compound on PC activity was carried out by a coupling method. The PC reaction product oxaloacetate and β-NADH undergo a redox reaction under the catalysis of malate dehydrogenase, generating malic acid and $NAD^+$. Accordingly, the inhibition ability of the compound on PC can be reflected by detecting the change of absorbance value at 340 nm.

Test methods:

a. 10 mM compound stock solution (the present compound was dissolved in 100% DMSO to prepare the 10 mM stock solution) was geometrically diluted with 100% DMSO in 1:3 in a 96-well dilution plate (249944, Nunc). The gradient concentrations of the compound were: 10 mM, 3.333 mM, 1.111 mM, 0.370 mM, 0.123 mM, 0.0412 mM, 0.0137 mM, 0.00457 mM, 0.00152 mM, 0.000508 mM, 0.000169 mM, and 0.0000565 mM.
b. 65.7 μL PC enzyme reaction buffer (201 mM triethanolamine, 7.5 mM magnesium sulfate, 0.18% bovine serum albumin) was added to each well of a 96-well dilution plate (249944, Nunc), and then 1 μL of the serially diluted compound to be tested in step a was added to each well.
c. 10 μL of compound (prepared in step b) was added to each well of a 384-well test plate (3702, Corning). The reaction system with $ZnCl_2$ at a final concentration of 500 μM was used as positive control; the reaction system with no compound but DMSO at a final concentration of 0.5% was used as negative control.
d. 10 μL of PC enzyme (Sigma, 0.15 U/ml stock solution of PC enzyme dissolved in 0.15 mM Tris-HCl pH 7.4, 0.006 mM $Mg(AcO)_2$, 0.15% glycerol, and 0.003 mM EDTA) was added to each well of the 384-well test plate (prepared in step c), centrifuged at 1000 rpm for 1 min, and incubated at room temperature for 15 min.
e. 10 μL of substrate mixture (1.5 mM sodium pyruvate, 1.5 mM ATP, 0.15 mM acetyl-CoA, 45 mM sodium bicarbonate, 0.69 mMNADH, 3 U/ml malate dehydrogenase, 201 mM triethanolamine, 7.5 mM magnesium sulfate, and 0.18% bovine serum albumin) was added to each well of the 384-well test plate (prepared in step d), and centrifuged at 1000 rpm for 1 min. The final concentrations of each component in the PC enzymatic reaction system: 0.05 U/ml pyruvate carboxylase, 0.5 mM sodium pyruvate, 0.5 mM ATP, 15 mM sodium bicarbonate, 0.23 mM β-NADH, 1 U/ml malic acid dehydrogenase, and 0.05 mM acetyl-CoA. Test compound concentrations: 50 μM, 16.667 μM, 5.556 μM, 1.852 μM, 0.617 μM, 0.206 μM, 0.0686 μM, 0.0229 μM, 0.00763 μM, 0.00254 μM, 0.000847 μM and 0.000282 μM. The final concentration of DMSO was 0.5% (v/v).
f. The absorbance at 340 nm was read by using EnVision 2104 plate reader (Perkin Elmer) with reading every 1 min for 40 min.

GraphPad Prism 6 software was used to perform a linear fit, and the concentration of each compound and the slope of the positive and negative controls were calculated. The inhibition percentage was calculated according to the following formula: 100 × [1 - (compound - average value of positive control) / (average value of negative control - average

value of positive control)].

$IC_{50}$ of the compound was obtained using GraphPad Prism 6 software according to the following nonlinear fitting formula:

$$Y = Bottom + (Top - Bottom) / (1 + 10 \wedge ((Log\ IC50 - X) \times HillSlope)),$$

wherein X is the logarithm of the compound concentration, Y is the inhibition rate; Top and Bottom are the Y values at the highest and lowest plateau of the curve; Hillslope is the Hill constant.

The inhibitory activities of the present compound on PC are shown in Table 4 below.

Table 4 The inhibitory $IC_{50}$ values of the present compound on PC

| Example | $IC_{50}$ (μM) |
|---|---|
| 5b | > 50 |
| 8 | > 50 |
| 8c | > 50 |
| 8d | > 50 |

The compound of the invention has no significant inhibitory effect on PC, suggesting a relatively high selectivity.

Test Example 4: *In vitro* inhibition effect of the present compound on the proliferation of HepG2 cells

CellTiter-Glo Analysis Kit (Promega) was used for the detection of the inhibitory effect of the present compound on the proliferation of HepG2 cells. The CellTiter-Glo detection method uses luciferase, which requires ATP for its reaction and luminescence. The intensity of the luminescent signal depends on the amount of ATP, and the amount of ATP depends on the number of living cells. Said analysis only requires that ATP in living cells is converted to luminescence by Ultra-Glo™ luciferase after treatment with the tested drug, so that the inhibitory effect of the compound is detected based on the luminescence intensity, and its $IC_{50}$ value is calculated.

Test methods:

a. HepG2 cells were purchased from the American type culture collection (ATCC) resource bank. Cells were cultured in MEM (GIBCO) containing 10% fetal bovine serum, penicillin (100 units/mL) and streptomycin (100 μg/mL), and then incubated in a 37°C incubator containing 5% carbon dioxide, and passaged once every 2 to 3 days.

b. On the first day, 800 HepG2 cells were inoculated in each well of a 384-well cell culture plate, and then incubated in a 37°C incubator containing 5% carbon dioxide.

c. On the second day, the medium was changed to that containing the compound. The concentrations of the compound were 30 μM, 10 μM, 3.33 μM, 1.11 μM, 0.37 μM, 0.123 μM, 0.0411 μM, 0.0137 μM, 0.00457 μM and 0.00152 μM. The final concentration of DMSO was 0.3% (v/v). The plate was incubated for 3 days in a incubator containing 5% carbon dioxide at 37°C. The reaction system with paclitaxel at a final concentration of 1 μM was used as positive control; the reaction system with no compound but DMSO at a final concentration of 0.3% was used as negative control.

d. The cell plate to be tested was placed at room temperature to equilibrate for 30 min.

e. 30 μL of CTG reagent (CelTiter Glo reagent) was added to each well, placed on a fast shaker for 2 min, and placed in the dark at room temperature for 10 min.

f. The chemiluminescence signal value was read with Envision 2104 instrument.

The average value of the positive and negative control well and the standard deviation were calculated. The percentage inhibition rate was calculated according to the following formula: 100 × [1 - (compound - average value of positive control) / (average value of negative control - average value of positive control)].

$IC_{50}$ of the compound was obtained using GraphPad Prism 6 software according to the following nonlinear fitting formula:

$$Y = Bottom + (Top - Bottom) / (1 + 10 \wedge ((Log\ IC50 - X) \times HillSlope)),$$

wherein X is the logarithm of the compound concentration, Y is the inhibition rate; Top and Bottom are the Y values at the highest and lowest plateau of the curve; Hillslope is the Hill constant.

The inhibitory activities of the present compound on proliferation of HepG2 cells are shown in Table 5 below.

Table 5 The inhibitory $IC_{50}$ values of the present compound on proliferation of HepG2 cells

| Example | $IC_{50}$ (μM) |
|---|---|
| 5x | > 30 |
| 5y | > 30 |
| 8 | > 30 |

The compound of the invention has no significant inhibitory effect on the proliferation of HepG2 cells, suggesting a relatively high liver safety.

## Claims

1. A compound of formula (I):

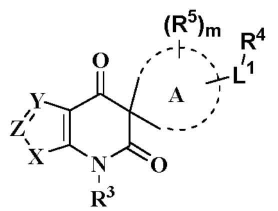

(I)

or a mesomer, racemate, enantiomer, diastereomer, or mixture thereof, or a pharmaceutically acceptable salt thereof,
wherein,
X is selected from -O-, -S- and -NR-;
Y is selected from N and $CR^1$;
Z is selected from N and $CR^2$;
ring A is cycloalkyl or heterocyclyl;
$L^1$ is selected from a single bond and a $C_1$-$C_3$ alkylene, wherein said alkylene is optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
$R^4$ is selected from the group consisting of hydrogen, halogen, cyano, $-R^a$, $-OR^a$, $-S(O)_nR^a$, $-N(R^a)C(O)R^b$, $-C(O)NR^aR^b$, $-C(O)N(R^a)S(O)_nR^b$, $-N(R^a)C(O)NR^aR^b$, $-N(R^a)C(O)OR^b$, $-OC(O)NR^aR^b$, $-N(R^a)S(O)_nR^b$, $-S(O)_nNR^aR^b$, $-C(O)R^a$, $-C(O)OR^a$, $-OC(O)R^a$, $-P(O)(OR^a)(OR^b)$ and $-B(OH)_2$;
$R^5$ is each independently selected from the group consisting of hydrogen, halogen, amino, nitro, cyano, hydroxyl, thiol, carboxyl, ester, oxo, alkyl, alkoxyl, cycloalkyl, heterocyclyl, aryl and heteroaryl, wherein said alkyl, alkoxyl, cycloalkyl, heterocyclyl, aryl and heteroaryl are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
R is selected from the group consisting of hydrogen, halogen, oxo, alkyl, alkoxyl, cycloalkyl, heterocyclyl, aryl and heteroaryl, wherein said alkyl, alkoxyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
$R^1$ is selected from the group consisting of hydrogen, halogen, alkyl, cycloalkyl and heterocyclyl, wherein said alkyl, cycloalkyl, and heterocyclyl are optionally further substituted by one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
$R^2$ is selected from the group consisting of hydrogen, halogen, alkyl, alkoxyl, cycloalkyl, heterocyclyl, aryl and heteroaryl, wherein said alkyl, alkoxyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;
$R^3$ is selected from alkyl, said alkyl is further substituted with one or more Q groups; each Q is independently

selected from the group consisting of halogen, alkyl, cycloalkyl, heterocyclyl, aryl, heteroaryl, $OR^a$ and $SR^a$; wherein said alkyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxy, haloalkyl, haloalkoxyl, cycloalkyl, heterocyclyl, aryl and heteroaryl;

$R^a$ and $R^b$ are each independently selected from the group consisting of hydrogen, halogen, hydroxyl, nitro, cyano, oxo, carboxyl, ester, alkyl, cycloalkyl, heterocyclyl, aryl and heteroaryl, wherein said alkyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl; or

$R^a$ and $R^b$ together with the nitrogen atom attached to them form a N-containing heterocyclic group, said N-containing heterocyclic group is optionally further substituted with one or more groups selected from the group consisting of halogen, amino, nitro, cyano, oxo, hydroxyl, thiol, carboxyl, ester, alkyl, alkoxyl, cycloalkyl, heterocyclyl, aryl and heteroaryl;

m is an integer from 0 to 5;

n is an integer from 0 to 2.

**2.** The compound of formula (I) according to claim 1, wherein X is -S-.

**3.** The compound of formula (I) according to claim 1 or 2, which is a compound of formula (II),

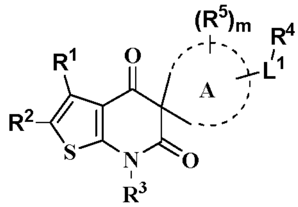

(II)

wherein, $R^1$, $R^2$, $R^3$, ring A, $L^1$, $R^4$, $R^5$ and m are as defined in claim 1.

**4.** The compound of formula (I) according to any one of claims 1 to 3, wherein,

$R^3$ is selected from alkyl, preferably $C_1$-$C_6$ alkyl, said alkyl is further substituted by one or more Q groups;

each Q is independently selected from the group consisiting of aryl, heteroaryl, $OR^a$ and $SR^a$; wherein said aryl and heteroaryl are optionally further substituted with one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;

$R^a$ is selected from the group consisiting of alkyl, cycloalkyl and heterocyclyl, wherein said alkyl, cycloalkyl and heterocyclic are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl.

**5.** The compound of formula (I) according to any one of claims 1 to 4, which is a compound of formula (III),

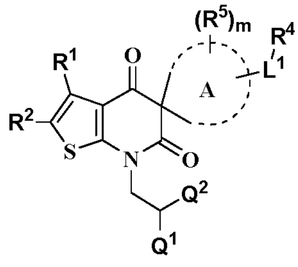

(III)

wherein,

$Q_1$ is selected from aryl and heteroaryl, preferably $C_5$-$C_{10}$ aryl or 5- to 10-membered heteroaryl, said aryl and heteroaryl are optionally further substituted with one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;
$Q_2$ is selected from $OR^a$ and $SR^a$, preferably $OR^a$;
$R^a$ is selected from cycloalkyl and heterocyclyl, preferably $C_3$-$C_7$ cycloalkyl or 5- to 7-membered heterocyclyl; wherein said cycloalkyl and heterocyclyl are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
wherein, $R^1$, $R^2$, ring A, $L^1$, $R^4$, $R^5$ and m are as defined in claim 1.

**6.** The compound of formula (I) according to any one of claims 1 to 5, which is a compound of formula (IV),

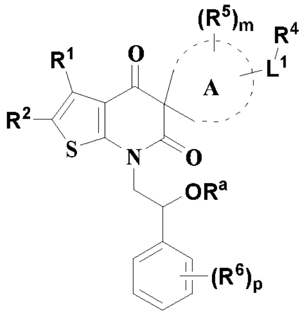

(IV)

wherein,

$R^6$ is selected from the group consisting of hydrogen, halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl;
$R^a$ is selected from cycloalkyl and heterocyclyl, preferably $C_3$-$C_7$ cycloalkyl or 5- to 7-membered heterocyclyl; wherein said cycloalkyl and heterocyclyl are optionally further substituted with one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl;
p is an integer from 1 to 4;
wherein, $R^1$, $R^2$, ring A, $L^1$, $R^4$, $R^5$ and m are as defined in claim 1.

**7.** The compound of formula (I) according to any one of claims 1 to 6,

wherein,
ring A is a $C_3$-$C_7$ cycloalkyl group or a 5- to 7-membered heterocyclic group;
$L^1$ is selected from a single bond and a $C_1$-$C_3$ alkylene, preferably a single bond;
$R^4$ is selected from the group consisting of hydrogen, halogen, cyano, $-R^a$, $-OR^a$, - $S(O)_nR^a$, $-N(R^a)C(O)R^b$, $-C(O)NR^aR^b$, $-C(O)N(R^a)S(O)nR^b$, $-N(R^a)C(O)NR^aR^b$, $-N(R^a)C(O)OR^b$, $-OC(O)NR^aR^b$, $-N(R^a)S(O)_nR^b$, $-S(O)_nNR^aR^b$, $-C(O)R^a$, $-C(O)OR^a$, - $OC(O)R^a$, $-P(O)(OR^a)(OR^b)$ and $-B(OH)_2$;
$R^5$ is each independently selected from the group consisting of hydrogen, halogen, amino, hydroxyl, thiol, carboxyl, ester, oxo and alkyl, wherein said alkyl is optionally further substituted with halogen;
$R^a$ and $R^b$ are each independently selected from the group consisting of hydrogen, halogen, hydroxyl, nitro, cyano, oxo, carboxyl, ester, alkyl, cycloalkyl, heterocyclyl, aryl and heteroaryl, wherein said alkyl, cycloalkyl, heterocyclyl, aryl and heteroaryl are optionally further substituted by one or more groups selected from the group consisting of halogen, amino, nitro, cyano, hydroxyl, thiol, carboxyl, ester, oxo, alkyl, alkoxyl, cycloalkyl, heterocyclyl, aryl and heteroaryl; or
$R^a$ and $R^b$ together with the nitrogen atom attached to them form a N-containing heterocyclic group, wherein said N-containing heterocyclic group is optionally further substituted with one or more groups selected from the group consisting of halogen, amino, nitro, cyano, oxo, hydroxyl, thiol, carboxyl, ester, alkyl, alkoxyl, cycloalkyl,

heterocyclyl, aryl and heteroaryl;
m is 0, 1 or 2;
n is an integer from 0 to 2; preferably 1 or 2.

**8.** The compound of formula (I) according to any one of claims 1 to 7,

wherein,
ring A is selected from the group consisting of cyclopropyl, cyclobutyl, cyclopentyl, cyclohexyl, pyrrolidinyl, piperidinyl and piperazinyl;
$L^1$ is selected from a single bond;
$R^4$ is selected from the group consisting of hydrogen, -Ra, -C(O)$R^a$, -C(O)O$R^a$, - C(O)N$R^aR^b$, -$S(O)_nR^a$ and -P(O)(O$R^a$)(O$R^b$);
$R^5$ is each independently selected from the group consisting of hydrogen, halogen, amino, hydroxyl, thiol, carboxyl, ester, oxo and alkyl, wherein said alkyl is optionally further substituted with halogen;
$R^a$ and $R^b$ are each independently selected from the group consisting of hydrogen, hydroxyl, carboxyl, ester and alkyl, wherein said alkyl is optionally further substituted with one or more groups selected from the group consisting of hydroxyl, thiol, carboxyl and ester;
m is 0, 1 or 2;
n is 1 or 2.

**9.** The compound of formula (I) according to any one of claims 1 to 8,

wherein,
$R^1$ is selected from the group consisting of hydrogen, halogen and alkyl, wherein said alkyl is optionally further substituted with halogen.

**10.** The compound of formula (I) according to any one of claims 1 to 9,

wherein,
$R^2$ is selected from aryl and heteroaryl, preferably $C_5$-$C_{10}$ aryl or 5- to 10-membered heteroaryl, more preferably oxazolyl, imidazolyl, pyrazolyl, thiazolyl, said aryl and heteroaryl are optionally further substituted by one or more groups selected from the group consisting of halogen, amino, nitro, cyano, hydroxyl, thiol, carboxyl, ester, oxo, alkyl, alkoxyl, haloalkyl, haloalkoxyl, cycloalkyl, heterocyclyl, aryl and heteroaryl.

**11.** The compound of formula (I) according to any one of claims 1 to 10 or a mesomer, racemate, enantiomer, diastereomer, or mixture thereof, or a pharmaceutically acceptable salt thereof, wherein said compound is selected from the group consisting of:

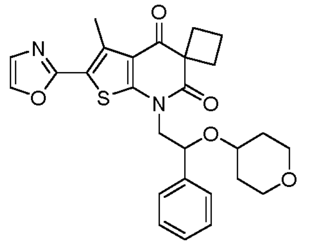 , 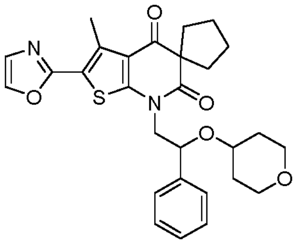 , 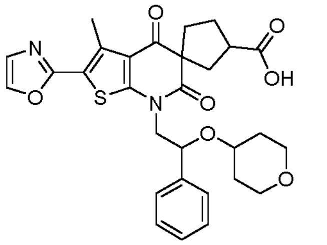 ,

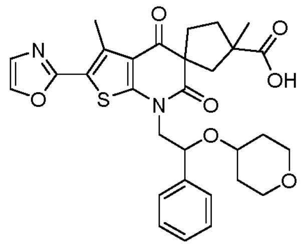 , 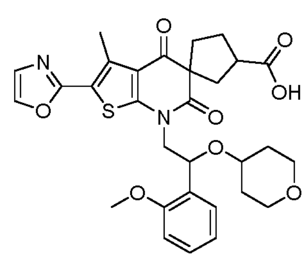 , 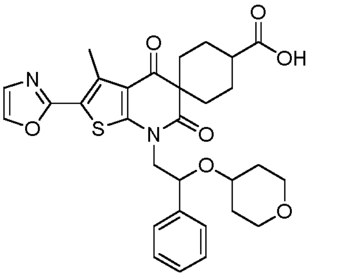 ,

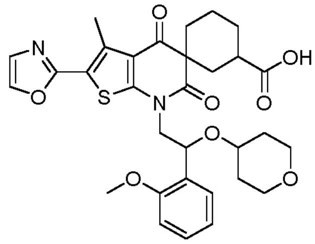 , 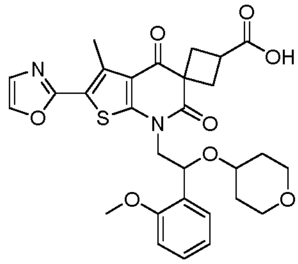 , 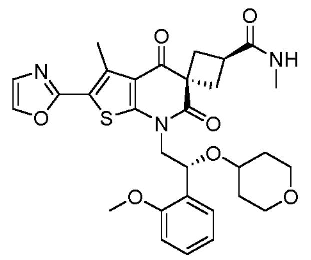 ,

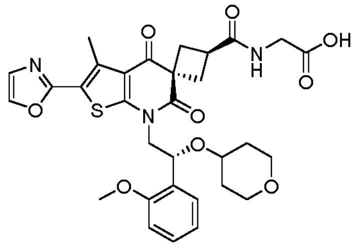 , 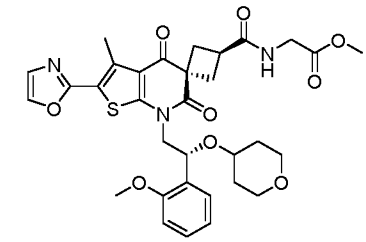 , 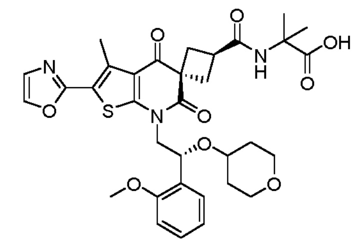 ,

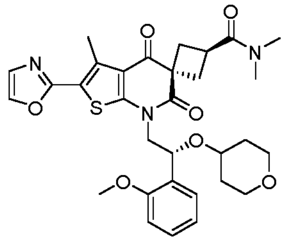 , 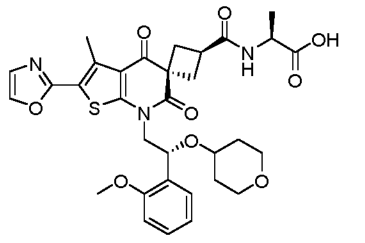 , 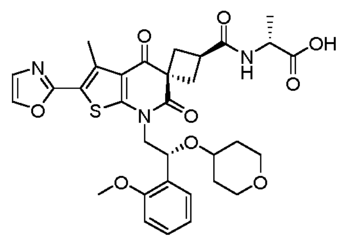 ,

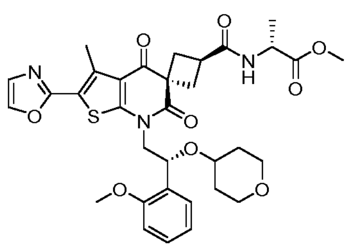 , 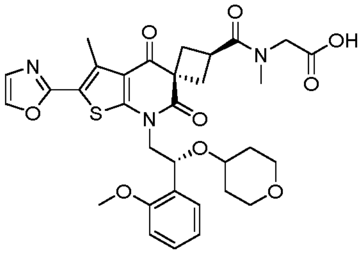 , 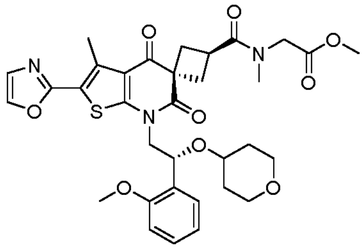 ,

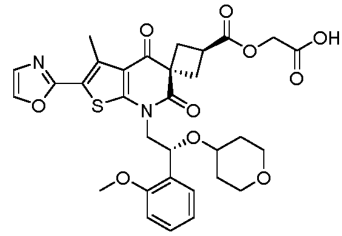,
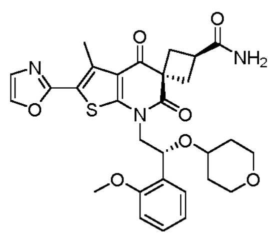,
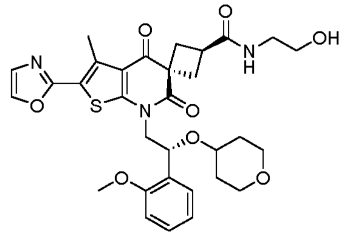,

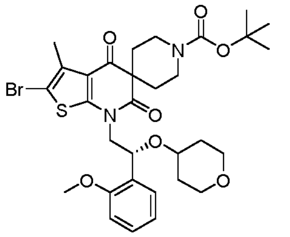,
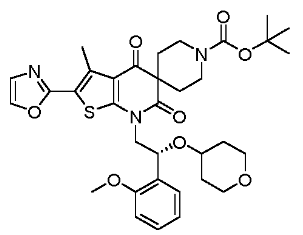,
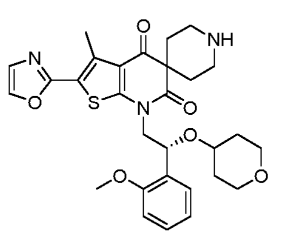,

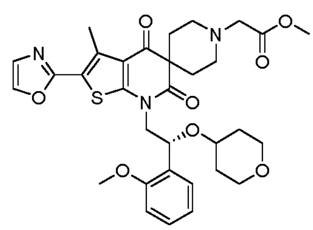,
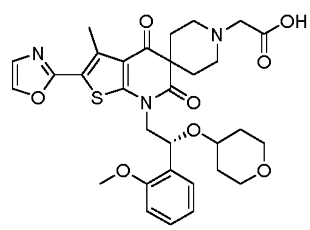,
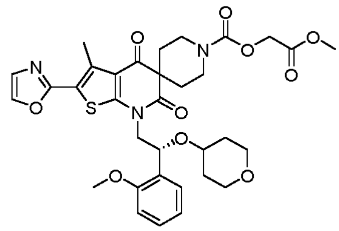,

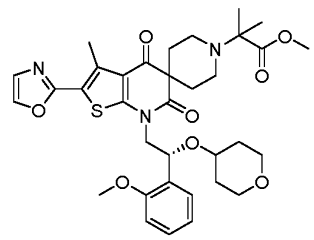,
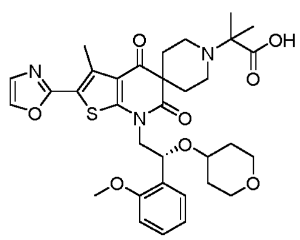,
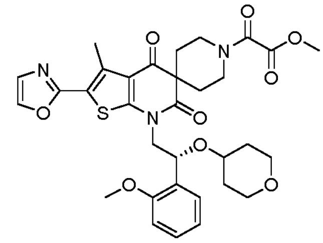,

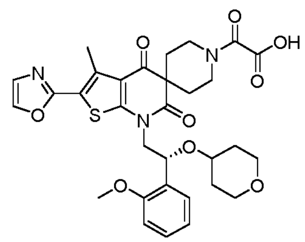,
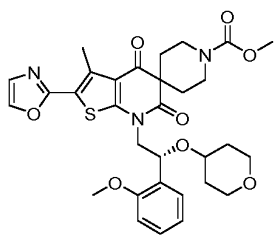,
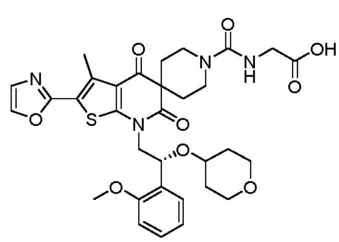,

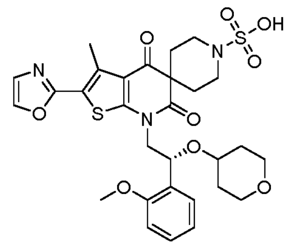,
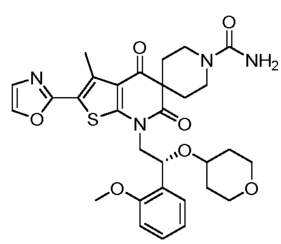,
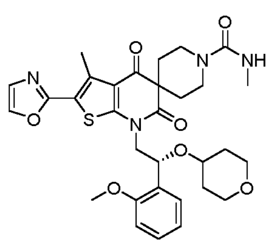,

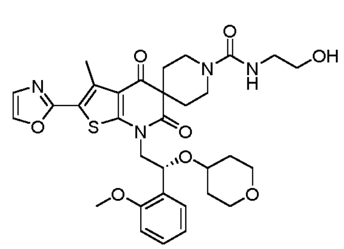
and
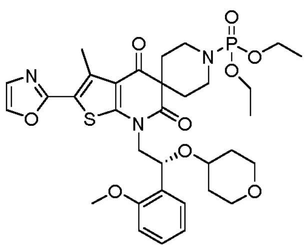.

**12.** A method for preparing the compound of formula (I) according to any one of claims 1 to 11, which comprises the following steps:

when Z is $CR^2$,

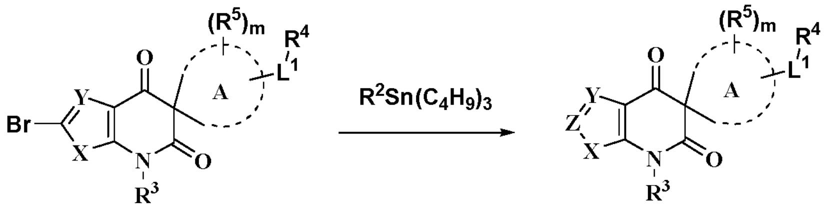


IF

(I)

the compound IF is reacted with $R^2Sn(C_4H_9)_3$ in the presence of a catalyst to obtain a compound of formula (I), wherein said catalyst is preferably bis(triphenylphosphorus)palladium dichloride;
X, Y, ring A, $L^1$, $R^2$, $R^3$, $R^4$, $R^5$ and m are as defined in claim 1.

**13.** A pharmaceutical composition comprising the compound of formula (I) according to any one of claims 1 to 11 and a pharmaceutically acceptable carrier or excipient.

**14.** Use of the compound of formula (I) according to any one of claims 1 to 11 or a pharmaceutical composition according to claim 13 in the preparation of acetyl-Coenzyme A carboxylase inhibitor.

**15.** Use of the compound of formula (I) according to any one of claims 1 to 11 or a pharmaceutical composition according to claim 13 in the preparation of medicaments for the prevention or treatment of diseases associated with acetyl-Coenzyme A carboxylase activity; said disease is preferably metabolic disease, cardiovascular disease or cancer; said metabolic disease is for example dyslipidemia, obesity, diabetes, insulin resistance, metabolic syndrome, fatty liver disease or steatohepatitis, preferably fatty liver disease or steatohepatitis; said cardiovascular disease is for example atherosclerosis, angina pectoris, acute coronary syndrome or heart failure; said cancer is for example breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma or non-small cell lung cancer, preferably liver cancer.

FIG. 1

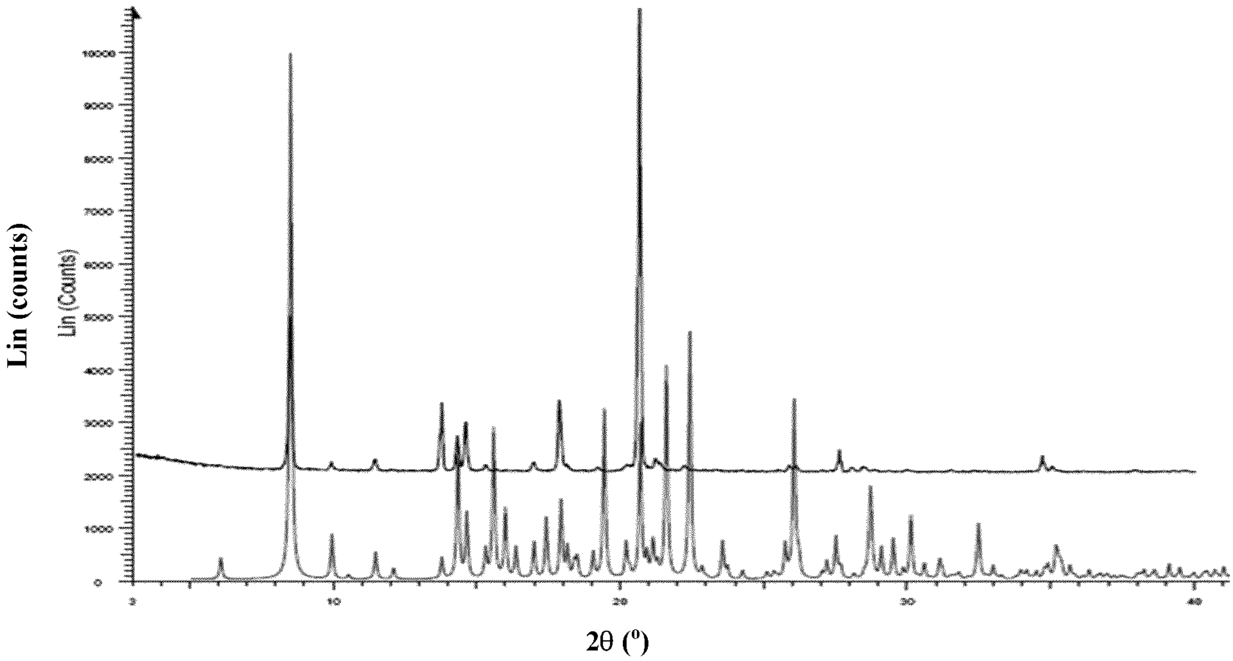
Lin (counts)
Lin (Counts)
0
1000
2000
3000
4000
5000
6000
7000
8000
9000
10000
3
10
20
30
40
2θ (°)

FIG. 2

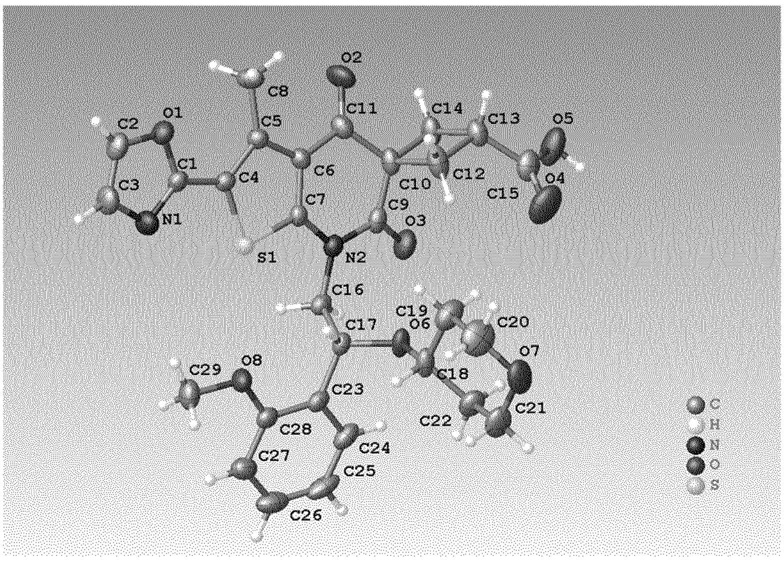

FIG. 3

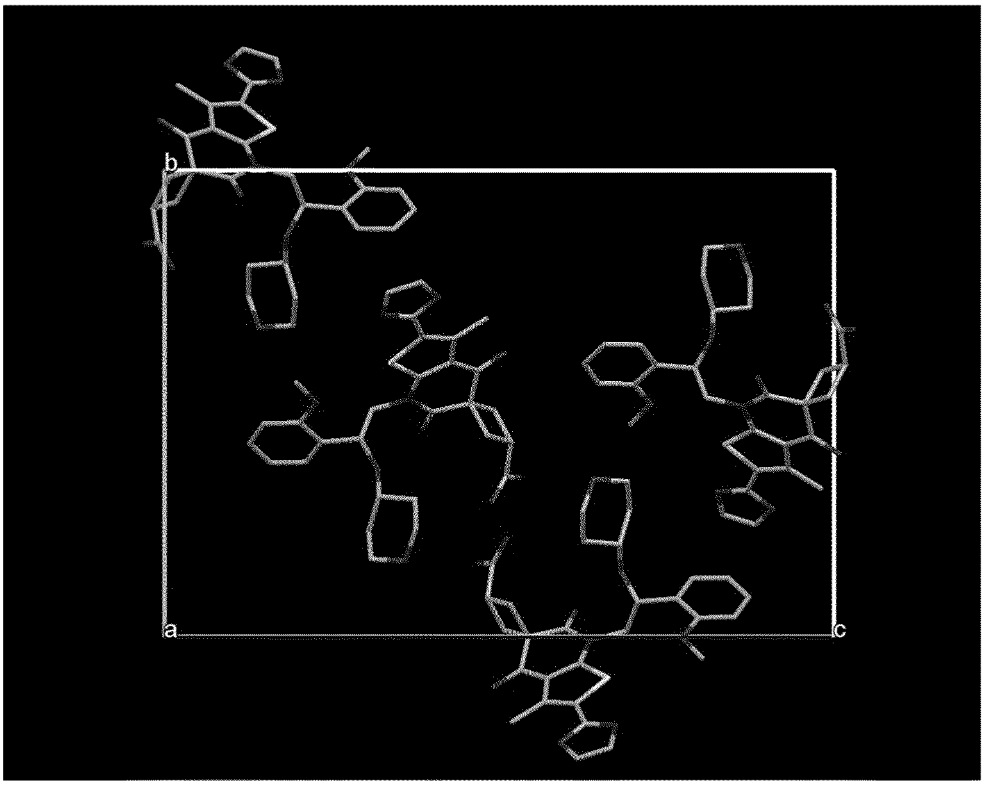

## INTERNATIONAL SEARCH REPORT

International application No.
**PCT/CN2019/119631**

**A. CLASSIFICATION OF SUBJECT MATTER**

C07D 495/04(2006.01)i; A61K 31/497(2006.01)i; A61P 1/16(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT; CNKI; WPI; EPODOC; CA; STN REGISTRY; STN CAPLUS; 中国医药研究开发中心有限公司; 螺环; 乙酰辅酶A羧化酶; ACC; 抑制剂; 代谢性疾病; 心血管疾病; 癌症; 肿瘤; CHINA MEDICINE RES & DEV CENT; Spirocyclic; Acetyl-CoA carboxylase; Inhibitor; Metabolic disease; Cardiovascular disease; Cancer; Tumor.

**C. DOCUMENTS CONSIDERED TO BE RELEVANT**

| Category* | Citation of document, with indication, where appropriate, of the relevant passages | Relevant to claim No. |
|---|---|---|
| X | CN 105358152 A (NIMBUS APOLLO, INC.) 24 February 2016 (2016-02-24)<br>claims 1-2 and 4-14, and description, table 1 and paragraph 0389 | 1-15 |
| A | CN 107106873 A (GILEAD APOLLO, LLC) 29 August 2017 (2017-08-29)<br>claims 1-37, and description, paragraphs 0022-0141 | 1-15 |
| A | WO 2015007451 A1 (SYNGENTA PARTICIPATIONS AG) 22 January 2015 (2015-01-22)<br>claims 1-15, and table 1 | 1-15 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 February 2020** | **14 February 2020** |
| Name and mailing address of the ISA/CN<br>**China National Intellectual Property Administration**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/119631**

| Patent document cited in search report | Publication date (day/month/year) | Patent family member(s) | Publication date (day/month/year) |
|---|---|---|---|
| CN 105358152 A | 24 February 2016 | AU 2014262636 A1 | 26 November 2015 |
| | | EP 2994138 A4 | 28 December 2016 |
| | | BR 112015028171 A2 | 25 July 2017 |
| | | HK 1221410 A1 | 02 June 2017 |
| | | JP 6417401 B2 | 07 November 2018 |
| | | AR 096241 A1 | 16 December 2015 |
| | | EP 2994138 A1 | 16 March 2016 |
| | | CA 2911926 A1 | 13 November 2014 |
| | | WO 2014182943 A1 | 13 November 2014 |
| | | MX 2015015417 A | 21 June 2016 |
| | | US 9988399 B2 | 05 June 2018 |
| | | EA 201591959 A1 | 31 March 2016 |
| | | JP 2016526024 A | 01 September 2016 |
| | | HK 1221659 A1 | 09 June 2017 |
| | | US 2016108060 A1 | 21 April 2016 |
| | | CL 2015003314 A1 | 15 July 2016 |
| | | KR 20160007598 A | 20 January 2016 |
| CN 107106873 A | 29 August 2017 | US 2019381045 A1 | 19 December 2019 |
| | | BR 112017014341 A2 | 27 March 2018 |
| | | EP 3242722 A4 | 11 July 2018 |
| | | EA 201892625 A1 | 31 July 2019 |
| | | EA 201791258 A1 | 29 December 2017 |
| | | WO 2016112305 A1 | 14 July 2016 |
| | | SG 11201705361P A | 30 August 2017 |
| | | KR 20170102299 A | 08 September 2017 |
| | | US 2018021341 A1 | 25 January 2018 |
| | | JP 2018501276 A | 18 January 2018 |
| | | MX 2017008844 A | 14 March 2018 |
| | | AU 2016205138 A1 | 13 July 2017 |
| | | EP 3242722 A1 | 15 November 2017 |
| | | CA 2972919 A1 | 14 July 2016 |
| WO 2015007451 A1 | 22 January 2015 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Molecules,* 2013, vol. 18, 1704-1719
- *Elsevier Science & Technology,* 2010, vol. 45 (10), 95-108
- *J. Med. Chem.,* 2015, vol. 58 (2), 525-36
- *Proc. Natl. Acad. Sci.,* 2016, vol. 113 (13), E1796-805
- **T. W. GREENE ; G. M. WUTS.** Protective Groups in Organic Synthesis. Wiley, 1999